# EUROPEAN PATENT APPLICATION

(11) **EP 3 872 096 A1**
(43) Date of publication of application: **01.09.2021**
(21) Application number: 19877152.9
(22) Date of filing: 25.10.2019
(51) Int. Cl.: C07K 19/00, C12P 21/02, C12N 15/62, C12N 15/77

(54) **METHOD FOR SECRETORY PRODUCTION OF PROTEIN**

(30) Priority: 25.10.2018 JP 2018200673
(71) Applicant: AJINOMOTO CO., INC., Chuo-ku Tokyo 104-8315 (JP)
(72) Inventor: MATSUDA, Yoshihiko, Kawasaki-shi, Kanagawa 210-8681 (JP)
(74) Representative: Hoffmann Eitle
(86) International application number: PCT/JP2019/042069
(87) International publication number: WO 2020/085511

(57) **Abstract**

A novel technique for reducing the mis-cleavage of the TorA signal peptide, and thereby a method for efficient secretory production of a heterologous protein by a coryneform bacterium using a TorA signal peptide is provided. A coryneform bacterium having an ability of secretory production of a heterologous protein using a TorA signal peptide and has been modified so that the activity of a LepB protein is increased is cultured to produce the heterologous protein by secretory production.

## Description

### Technical Field

The present invention relates to a method for secretory production of a heterologous protein.

### Background Art

As secretory production methods of heterologous proteins, for example, methods using microorganisms such as coryneform bacteria as expression hosts have been known. For the purpose of efficient secretory production of heterologous proteins, for example, signal peptides (also referred to as "signal sequences") can be used. That is, by expressing a heterologous protein fused at the N-terminus with a signal peptide corresponding to a protein secretion system possessed by a host, the heterologous protein can be efficiently produced by secretory production.

A general protein secretion pathway is a pathway called "Sec system", which is widely present from prokaryotes to eukaryotes. In secretory production of heterologous proteins by coryneform bacteria using the Sec system, secretory production of a heterologous protein can be improved by, for example, inserting an amino acid sequence comprising Gln-Glu-Thr or Ala-Glu-Thr between a signal peptide and the heterologous protein.

Meanwhile, a protein secretion pathway completely different from the Sec system has recently been found in thylakoid membranes of chloroplasts of plant cells (Non-patent document 1). This novel secretory pathway has been named "Tat system" (Twin-Arginine Translocation system) because an arginine-arginine sequence is commonly present in the signal peptide of a protein secreted thereby (Non-patent document 1). It is known that proteins are secreted by the Sec system in a state before forming a higher-order structure, while proteins are secreted by the Tat system through a cell membrane after forming a higher-order structure in the cell (Non-patent document 2). Also for coryneform bacteria, secretory production of proteins utilizing a Tat-dependent signal peptide such as a TorA signal peptide has been reported (Patent documents 1, 2, *etc.*)*.*

Signal peptides are generally cleaved at the C-terminus by a signal peptidase and removed from proteins at a time when the proteins are secreted out of cells. However, in secretory production of proteins, there can be sometimes a problem that signal peptides are not cleaved and thereby proteins accumulate in cells. In such a case, it has been known that secretion efficiency of proteins can be improved by overexpressing a signal peptidase such as LepB protein (Patent documents 3-8 and Non-patent documents 3-7.

However, there has been not known a phenomenon that signal peptides are cleaved inside thereof instead of at the C-terminus and thereby proteins are secreted out of cells while holding a C-terminal-side partial sequence of the signal peptides.

### Prior art references

### Patent documents

Patent document 1: WO2013/118544
Patent document 2: Japanese Patent No. 4730302
Patent document 3: EP444759
Patent document 4: EP974654
Patent document 5: WO2004/007740
Patent document 6: DE102004035543
Patent document 7: KR101077783
Patent document 8: CN104611317

### Non-patent documents

Non-patent document 1: EMBO J., 14, 2715-2722(1995)
Non-patent document 2: J. Biol. Chem., 25;273(52), 34868-74(1998)
Non-patent document 3: Biochem Biophys Res Commun. 1999 255(2):444-50.
Non-patent document 4: Biotechnol Bioeng. 2005 91(5):616-21.
Non-patent document 5: J Microbiol Biotechnol. 2007 Aug;17(8):1316-23.
Non-patent document 6: J Biol Chem. 2011 Dec 23;286(51):43601-10.
Non-patent document 7: J Appl Microbiol. 2016 Sep;121(3):704-12.

### Summary of the Invention

### Object to be Achieved by the Invention

The inventors of the present invention found that, in secretory production of a heterologous protein by a coryneform bacterium using a TorA signal peptide, the TorA signal peptide is not cleaved at a correct position and thereby mis-cleavage of C-terminal side 15 residues occurs. An object of the present invention is to develop a novel technique for reducing the mis-cleavage of the TorA signal peptide, and thereby to provide a method for secretory production of a heterologous protein by a coryneform bacterium using the TorA signal peptide.

### Means for Achieving the Object

The inventors of the present invention conducted various researches in order to achieve the aforementioned object. As a result, they found that mis-cleavage of a TorA signal peptide in secretory production of a heterologous protein by a coryneform bacterium using the TorA signal peptide can be reduced by modifying the coryneform bacterium so that the activity of a LepB protein is increased, and accomplished the present invention.

The present invention can be thus embodied as follows.
[1] A method for producing a heterologous protein comprising:
   culturing a coryneform bacterium having a genetic construct for secretory expression of the heterologous protein; and
   collecting the heterologous protein produced by secretory production,
   wherein the coryneform bacterium has been modified so that the activity of a LepB protein is increased,
   wherein the genetic construct comprises, in the direction from 5' to 3', a promoter sequence that functions in the coryneform bacterium, a nucleic acid sequence encoding a TorA signal peptide, and a nucleic acid sequence encoding the heterologous protein, and
   wherein the heterologous protein is expressed as a fusion protein with the TorA signal peptide.
[2] The method mentioned above, wherein the LepB protein is a protein defined in (a), (b), or (c) mentioned below:
   (a) a protein comprising the amino acid sequence of SEQ ID NO: 2;
   (b) a protein comprising the amino acid sequence of SEQ ID NO: 2, but which includes substitution, deletion, insertion, and/or addition of 1 to 10 amino acid residues, wherein said protein has a signal peptidase activity for the TorA signal peptide;
   (c) a protein comprising an amino acid sequence showing an identity of 90% or higher to the amino acid sequence of SEQ ID NO: 2, wherein said protein has a signal peptidase activity for the TorA signal peptide.
[3] The method mentioned above, wherein the activity of the LepB protein is increased by increasing the expression of a *lepB* gene.
[4] The method mentioned above, wherein the expression of the *lepB* gene is increased by increasing the copy number of *lepB* gene and/or modifying an expression control sequence of the *lepB* gene.
[5] The method mentioned above, wherein the TorA signal peptide is a peptide defined in (a), (b), or (c) mentioned below:
   (a) a peptide comprising the amino acid sequence of SEQ ID NO: 46;
   (b) a peptide comprising the amino acid sequence of SEQ ID NO: 46, but which includes substitution, deletion, insertion, and/or addition of 1 to 3 amino acid residues, wherein said protein has a function as a Tat-dependent signal peptide;
   (c) a peptide comprising an amino acid sequence showing an identity of 90% or higher to the amino acid sequence of SEQ ID NO: 46, wherein said protein has a function as a Tat-dependent signal peptide.
[6] The method mentioned above, wherein the TorA signal peptide consists of the amino acid sequence of SEQ ID NO: 46.
[7] The method mentioned above, wherein the heterologous protein produced by secretory production is a heterologous protein from which the TorA signal peptide has been completely removed.
[8] The method mentioned above, wherein the coryneform bacterium has been further modified so as to harbor *a phoS* gene encoding a mutant PhoS protein.
[9] The method mentioned above, wherein the mutation is a mutation of replacing an amino acid residue corresponding to the tryptophan residue at position 302 in SEQ ID NO: 29 with an amino acid residue other than aromatic amino acid and histidine residues in a wild-type PhoS protein.
[10] The method mentioned above, wherein the amino acid residue other than aromatic amino acid and histidine residues is a lysine residue, alanine residue, valine residue, serine residue, cysteine residue, methionine residue, aspartic acid residue, or asparagine residue.
[11] The method mentioned above, wherein the wild-type PhoS protein is a protein defined in (a), (b), or (c) mentioned below:
   (a) a protein comprising any of the amino acid sequences of SEQ ID NOS: 29 to 34;
   (b) a protein comprising any of the amino acid sequences of SEQ ID NOS: 29 to 34, but which includes substitution, deletion, insertion, and/or addition of 1 to 10 amino acid residues, wherein said protein has a function as a sensor kinase of a PhoRS system;
   (c) a protein comprising an amino acid sequence showing an identity of 90% or higher to any of the amino acid sequences of SEQ ID NOS: 29 to 34, wherein said protein has a function as a sensor kinase of a PhoRS system.
[12] The method mentioned above, wherein the coryneform bacterium has been further modified so that the expression of one or more genes selected from genes encoding a Tat secretion system is increased as compared with a non-modified strain.
[13] The method mentioned above, wherein the genes encoding a Tat secretion system consists of a *tatA* gene, *tatB* gene, *tatC* gene, and *tatE* gene.
[14] The method mentioned above, wherein the coryneform bacterium is a bacterium belonging to the genus *Corynebacterium.*
[15] The method mentioned above, wherein the coryneform bacterium is *Corynebacterium glutamicum.*
[16] The method mentioned above, wherein the coryneform bacterium is a modified strain derived from *Corynebacterium glutamicum* AJ12036 (FERM BP-734) or a modified strain derived from *Corynebacterium glutamicum* ATCC 13869.
[17] The method mentioned above, wherein the coryneform bacterium is a coryneform bacterium in which the number of molecules of a cell surface layer protein per cell is reduced as compared with a non-modified strain.

### Brief Description of the Drawings

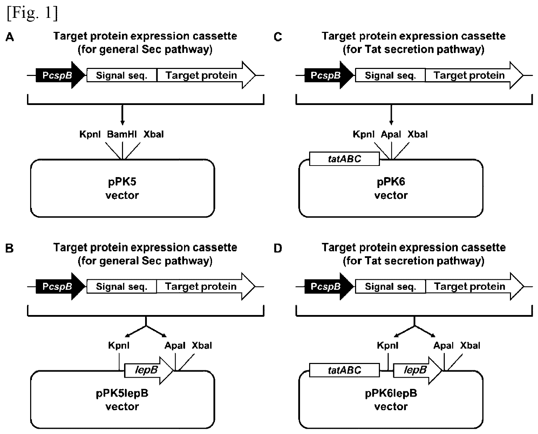

Fig. 1 is a diagram showing the structures of vectors.

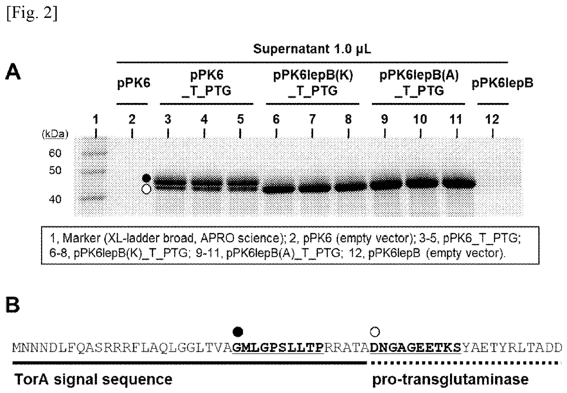

Fig. 2 is a photograph showing the result of SDS-PAGE observed upon expressing PTG (transglutaminase comprising a pro-structure moiety) fused with TorA signal peptide in the C. *glutamicum* YDK010::phoS(W302C) strain and a *lepB* gene expression-enhanced strain thereof. The amino acid sequence in this Fig. is shown in SEQ ID NO: 54.

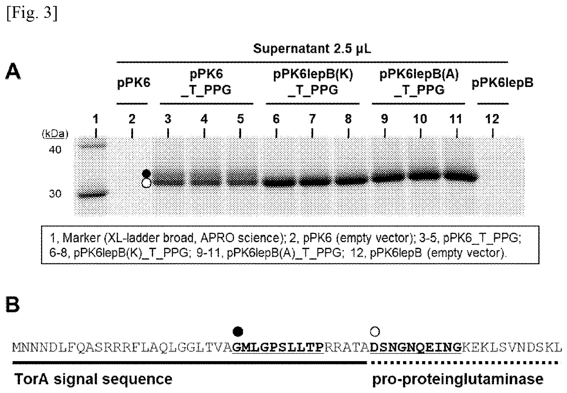

Fig. 3 is a photograph showing the result of SDS-PAGE observed upon expressing PPG (protein glutaminase comprising a pro-structure moiety) fused with TorA signal peptide in the *C*. *glutamicum* YDK010::phoS(W302C) strain and a *lepB* gene expression-enhanced strain thereof. The amino acid sequence in this Fig. is shown in SEQ ID NO: 55.

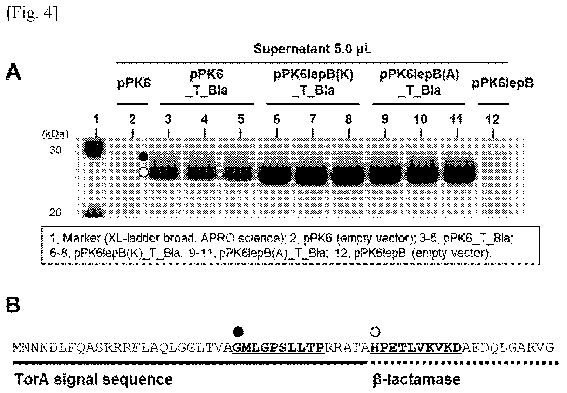

Fig. 4 is a photograph showing the result of SDS-PAGE observed upon expressing Bla (beta-lactamase) fused with TorA signal peptide in the *C*. *glutamicum* YDK010::phoS(W302C) strain and a *lepB* gene expression-enhanced strain thereof. The amino acid sequence in this Fig. is shown in SEQ ID NO: 56.

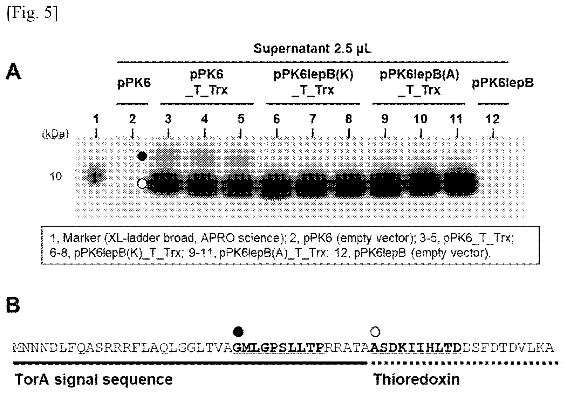

Fig. 5 is a photograph showing the result of SDS-PAGE observed upon expressing Trx (thioredoxin) fused with TorA signal peptide in the *C*. *glutamicum* YDK010::phoS(W302C) strain and a *lepB* gene expression-enhanced strain thereof. The amino acid sequence in this Fig. is shown in SEQ ID NO: 57.

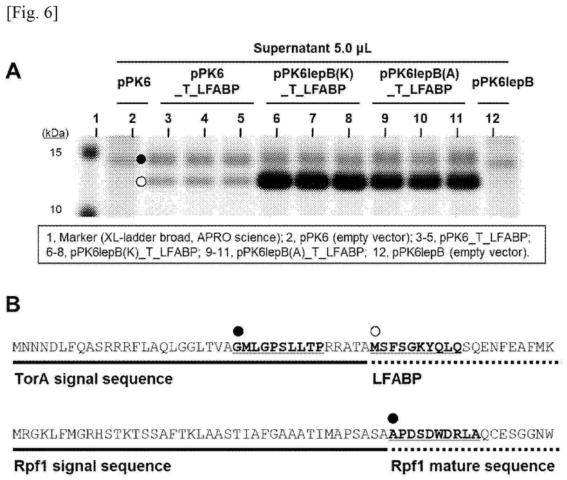

Fig. 6 is a photograph showing the result of SDS-PAGE observed upon expressing LFABP (Liver-type fatty acid-binding protein) fused with TorA signal peptide in the *C*. *glutamicum* YDK010::phoS(W302C) strain and a *lepB* gene expression-enhanced strain thereof. The amino acid sequences in this Fig. are shown in SEQ ID NOS: 58 and 59.

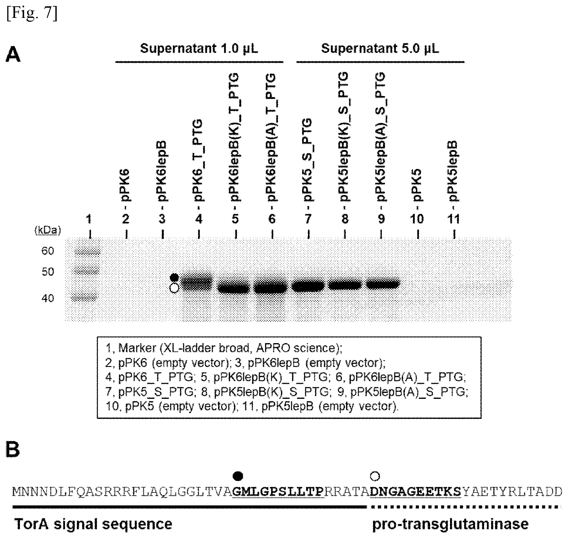

Fig. 7 is a photograph showing the result of SDS-PAGE observed upon expressing PTG (transglutaminase comprising a pro-structure moiety) fused with SlpA signal peptide in the *C. glutamicum* YDK010::phoS(W302C) strain and a *lepB* gene expression-enhanced strain thereof. The amino acid sequence in this Fig. is shown in SEQ ID NO: 54.

### Modes for Carrying out the Invention

Hereinafter, the present invention will be explained in detail.

The method of the present invention is a method for producing a heterologous protein, the method comprising culturing a coryneform bacterium having a genetic construct for secretory expression of the heterologous protein, and collecting the heterologous protein produced by secretory production, wherein the coryneform bacterium has been modified so that the activity of a LepB protein is increased, and wherein a TorA signal peptide is used in secretory production of the heterologous protein.

In method of the present invention, mis-cleavage of a TorA signal peptide can be reduced, and thereby a heterologous protein in a completely-cleaved form can be produced by secretory production. That is, the phrase "heterologous protein" to be produced by secretory production in the method of the present invention refers to a heterologous protein in a completely-cleaved form, unless otherwise stated. The phrase "heterologous protein in a completely-cleaved form" refers to a heterologous protein from which the TorA signal peptide has been completely removed, i.e. a heterologous protein not holding the TorA signal peptide at all.

### <1> Coryneform bacterium used for the method of the present invention

The coryneform bacterium used for the method of the present invention is a coryneform bacterium having a genetic construct for secretory expression of a heterologous protein, which has been modified so that the activity of a LepB protein is increased. The coryneform bacterium used for the method of the present invention is also referred to as "bacterium of the present invention" or "coryneform bacterium of the present invention". Furthermore, the genetic construct for secretory expression of a heterologous protein harbored by the bacterium of the present invention is also referred to as "genetic construct used for the present invention". Furthermore, the bacterium of the present invention or a strain used for constructing the same is also referred to as "host".

### <1-1> Coryneform bacterium having ability of secretory production of heterologous protein

The coryneform bacterium of the present invention has an ability of secretory production of a heterologous protein, specifically, a heterologous protein in a completely-cleaved form. The coryneform bacterium of the present invention has an ability of secretory production of a heterologous protein, relying at least on having the genetic construct for secretory expression of the heterologous protein (genetic construct used for the method of the present invention). Specifically, the coryneform bacterium of the present invention may have an ability of secretory production of a heterologous protein, relying on having the genetic construct for secretory expression of the heterologous protein, or relying on a combination of having the genetic construct for secretory expression of the heterologous protein and other characteristic(s).

In the present invention, the expression that a protein is "secreted" means that the protein is transported out of a bacterial cell (extracellularly transported). Examples of a position outside of a bacterial cell (outside of a cell) include a medium and a cell surface layer. That is, molecules of the secreted protein may be present, for example, in the medium, in the cell surface layer, or in both of the medium and the cell surface layer. That is, the expression that a protein is "secreted" is not limited to cases where all the molecules of the protein eventually exist in the medium in completely free forms, and also include, for example, cases where all the molecules of the protein exist in the cell surface layer, and cases where a part of the molecules of the protein exists in the medium and the remaining part of the molecules of the protein exists in the cell surface layer.

That is, in the present invention, the term "ability to produce a heterologous protein by secretory production" refers to an ability of the bacterium of the present invention to secrete the heterologous protein into a medium and/or a cell surface layer, and accumulate it there to such an extent that the heterologous protein can be collected from the medium and/or the cell surface layer, when the bacterium is cultured in the medium. The accumulation amount may be, for example, in terms of the accumulation amount in the medium, preferably 10 µg/L or more, more preferably 1 mg/L or more, particularly preferably 100 mg/L or more, still more preferably 1 g/L or more. Also, the accumulation amount may be, for example, in terms of the accumulation amount in the cell surface layer, such an amount that if the heterologous protein in the cell surface layer is collected and suspended in a liquid of the same volume as the medium, the concentration of the heterologous protein in the suspension is preferably 10 µg/L or more, more preferably 1 mg/L or more, particularly preferably 100 mg/L or more. In addition, in the present invention, the term "protein" to be produced by secretory production refers to a concept also including those called peptide, such as oligopeptides and polypeptides.

In the present invention, the term "heterologous protein" refers to an exogenous protein relative to a coryneform bacterium that expresses and secretes that protein. The heterologous protein may be, for example, a protein derived from a microorganism, a protein derived from a plant, a protein derived from an animal, a protein derived from a virus, or even a protein of which the amino acid sequence is artificially designed. The heterologous protein may particularly be a derived from human. The heterologous protein may be a monomeric protein or a multimeric protein. The term "multimeric protein" refers to a protein that may exist as a multimer consisting of two or more subunits. In the multimer, the subunits may be linked by covalent bonds such as disulfide bonds, linked by non-covalent bonds such as hydrogen bonds and hydrophobic interaction, or linked by a combination thereof. The multimer preferably comprises one or more intermolecular disulfide bonds. The multimer may be a homo-multimer consisting of a single kind of subunit, or may be a hetero-multimer consisting of two or more kinds of subunits. In the case where the multimeric protein is a hetero-multimer, it is sufficient that at least one subunit selected from the subunits constituting the hetero-multimer is a heterologous protein. That is, all the subunits may be heterologous, or only a part of subunits may be heterologous. Although the heterologous protein may be a secretory protein in nature, or may be a non-secretory protein in nature, it is preferably a secretory protein in nature. Furthermore, the heterologous protein may be a Tat-dependent secretory protein in nature, or may be a Sec-dependent secretory protein in nature. Specific examples of the "heterologous protein" will be mentioned later.

The heterologous protein to be produced may consist of a single kind of protein, or two or more kinds of proteins. Moreover, when the heterologous protein is a hetero-multimer, only one kind of subunit may be produced, or two or more kinds of subunits may be produced. That is, the term "secretory production of a heterologous protein" includes secretory production of all the subunits constituting an objective heterologous protein, as well as secretory production of only a part of the subunits constituting an objective heterologous protein.

Coryneform bacteria are aerobic gram-positive bacilli. Examples of the coryneform bacteria include *Corynebacterium* bacteria, *Brevibacterium* bacteria, *Microbacterium* bacteria, and so forth. Advantages of use of the coryneform bacteria include that they inherently secrete an extremely small amount of proteins out of cells compared with fungi, yeasts, *Bacillus* bacteria, *etc.,* which are conventionally used for secretory production of proteins, and therefore the purification process of a heterologous protein produced by secretory production is expected to be simplified or eliminated, that they can grow well in a simple medium containing a saccharide, ammonia, mineral salts, etc., and therefore they are excellent in view of cost of medium, culture method, and culture productivity, and so forth.

Specific examples of coryneform bacteria include the following species:
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes* (*Corynebacterium efficiens*)
*Corynebacterium herculis*
*Brevibacterium divaricatum* (*Corynebacterium glutamicum*)
*Brevibacterium flavum* (*Corynebacterium glutamicum*)
*Brevibacterium immariophilum*
*Brevibacterium lactofermentum* (*Corynebacterium glutamicum*)
*Brevibacterium roseum*
*Brevibacterium saccharolyticum*
*Brevibacterium thiogenitalis*
*Corynebacterium ammoniagenes* (*Corynebacterium stationis*)
*Brevibacterium album*
*Brevibacterium cerinum*
*Microbacterium ammoniaphilum*

Specific examples of coryneform bacteria include the following strains:
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS 1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium thermoaminogenes* (*Corynebacterium efficiens*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The *Corynebacterium* bacteria include bacteria that had previously been classified into the genus *Brevibacterium,* but are presently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but are presently re-classified into *Corynebacterium stationis* on the basis of nucleotide sequence analysis of 16S rRNA etc. (Int. J. Syst. Evol. Microbiol., 60, 874-879 (2010)).

These strains are available from, for example, the American Type Culture Collection (Address: 12301 Parklawn Drive, Rockville, Maryland 20852, P.O. Box 1549, Manassas, VA 20108, United States of America). That is, registration numbers are assigned to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

In particular, the *Corynebacterium glutamicum* (*C*. *glutamicum*) AJ12036 strain (FERM BP-734), which was isolated as a streptomycin (Sm) resistant mutant strain from a wild-type strain *C. glutamicum* ATCC 13869 is predicted to have a mutation in a gene responsible for a function involved in secretion of proteins, and shows an extremely high secretory production ability for proteins as high as about 2 to 3 times in terms of accumulation amount of proteins under optimum culture conditions, compared with the parent strain (wild-type strain), and therefore it is preferred as a host bacterium (WO2002/081694). The AJ12036 strain was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on March 26, 1984 as an international deposit, and assigned an accession number of FERM BP-734.

*Corynebacterium thermoaminogenes* AJ12340 (FERM BP-1539) was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on March 13, 1987 as an international deposit, and assigned an accession number of FERM BP-1539. *Brevibacterium flavum* AJ12418 (FERM BP-2205) was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on December 24, 1988 as an international deposit, and assigned an accession number of FERM BP-2205.

Moreover, a strain having an enhanced ability to produce a protein by secretory production may be selected from such a coryneform bacterium as mentioned above as a parent strain by using a mutagenesis method or a genetic recombination method, and used as a host. For example, after a parent strain is treated with ultraviolet irradiation or a chemical mutation agent such as N-methyl-N'-nitrosoguanidine, a strain having an enhanced ability to produce a protein by secretory production can be selected.

Furthermore, if a strain obtained by modifying such a strain as mentioned above so that it does not produce a cell surface layer protein is used as a host, purification of the heterologous protein secreted in the medium or on the cell surface layer becomes easy, and therefore it is particularly preferred. Such modification can be carried out by introducing a mutation into the coding region of the cell surface layer protein or an expression control region thereof, on the chromosome by mutagenesis or genetic recombination. Examples of coryneform bacterium modified so that it does not produce a cell surface layer protein include the *C. glutamicum* YDK010 strain (WO2002/081694), which is a cell surface layer protein PS2 deficient strain of the *C. glutamicum* AJ12036 strain (FERM BP-734).

A coryneform bacterium having an ability of secretory production of a heterologous protein can be obtained by introducing the genetic construct used for the method of the present invention into such a coryneform bacterium as described above so as to make the bacterium harbor the genetic construct. That is, the bacterium of the present invention may be, for example, a modified strain derived from such a coryneform bacterium as described above. The bacterium of the present invention may be, specifically, for example, a modified strain derived from *C. glutamicum* AJ12036 (FERM BP-734) or a modified strain derived from *C. glutamicum* ATCC 13869. A modified strain derived from *C. glutamicum* AJ12036 (FERM BP-734) falls also within a modified strain derived from *C. glutamicum* ATCC 13869. The genetic construct used for the method of the present invention and methods for introduction of the same will be described later.

### <1-2> Increase in activity of LepB protein

The bacterium of the present invention has been modified so that the activity of a LepB protein is increased. Specifically, the bacterium of the present invention has been modified so that the activity of a LepB protein is increased as compared with a non-modified strain. More specifically, the bacterium of the present invention may have been modified so that the expression of a *lepB* gene is increased. By modifying a coryneform bacterium so that the activity of a LepB protein is increased, mis-cleavage of a TorA signal peptide in secretory production of a heterologous protein by the bacterium using the TorA signal peptide can be reduced. Examples of reduction in mis-cleavage of a TorA signal peptide include reduction in mis-cleavage rate of a TorA signal peptide. The phrase "mis-cleavage rate of a TorA signal peptide" refers to weight ratio of the secretory production amount of a heterologous protein in a mis-cleaved form to the total secretory production amount of the heterologous protein. The phrase "total secretory production amount of a heterologous protein" refers to the sum of the secretory production amount of a heterologous protein in a completely-cleaved form and the secretory production amount of the heterologous protein in a mis-cleaved form. The phrase "heterologous protein in a mis-cleaved form" refers to a heterologous protein from which the TorA signal peptide has been partially removed, i.e. a heterologous protein holding a C-terminal side partial sequence of the TorA signal peptide. Examples of the C-terminal side partial sequence of the TorA signal peptide include the amino acid sequence of C-terminal side 15 residues of the TorA signal peptide. Specific examples of the C-terminal side partial sequence of the TorA signal peptide include the amino acid sequence of positions 25-39 of SEQ ID NO: 46. The mis-cleavage rate of the TorA signal peptide in the method of the present invention may be, for example, 3% or lower, 1% or lower, 0.5% or lower, 0.3% or lower, 0.1% or lower, or 0%. The mis-cleavage rate of the TorA signal peptide in the method of the present invention may also be, for example, 50% or lower, 30% or lower, 10% or lower, 5% or lower, 3% or lower, 1% or lower, or 0% of the mis-cleavage rate of the TorA signal peptide observed when using a non-modified strain, which herein refers to a strain not modified so that the activity of a LepB protein is increased. Also, by modifying a coryneform bacterium so that the activity of a LepB protein is increased, it is expected that an ability of the bacterium to produce a heterologous protein, specifically a heterologous protein in a completely-cleaved form, by secretory production when using the TorA signal peptide can be improved, that is, secretory production of a heterologous protein, specifically a heterologous protein in a completely-cleaved form, by the bacterium using the TorA signal peptide can be increased.

The bacterium of the present invention can be obtained by modifying a coryneform bacterium having an ability of secretory production of a heterologous protein so that the activity of a LepB protein is increased. The bacterium of the present invention can also be obtained by modifying a coryneform bacterium so that the activity of a LepB protein is increased, and then imparting an ability of secretory production of a heterologous protein thereto. In the present invention, modifications for constructing the bacterium of the present invention can be performed in any order. A strain to be used for constructing the bacterium of the present invention and before being modified so that the activity of a LepB protein is increased may or may not be able to produce a heterologous protein, on the assumption that the strain has the genetic construct for secretory expression of the heterologous protein. That is, the bacterium of the present invention may also be, for example, a bacterium that has acquired an ability of secretory production of a heterologous protein due to being modified so that the activity of a LepB protein is increased. Specifically, for example, the bacterium of the present invention may also be a bacterium obtained from a strain that is not able to produce a heterologous protein by secretory production even when it has the genetic construct for secretory expression of the heterologous protein before it is modified so that the activity of a LepB protein is increased, which came to be able to produce the heterologous protein by secretory production due to being modified so that the activity of a LepB protein is increased.

Hereinafter, the LepB protein and the *lepB* gene encoding the same will be explained. The LepB protein is a signal peptidase. The phrase "signal peptidase" refers to a protein (enzyme) having an activity of catalyzing processing of a signal peptide. This activity is also referred to as "signal peptidase activity". The phrase "processing of a signal peptide" refers to a reaction of removing the whole of a signal peptide from a protein having the signal peptide. The LepB protein at least has the signal peptidase activity for the TorA signal peptide, i.e. an activity of catalyzing processing of the TorA signal peptide.

Examples of the *lepB* gene and the LepB protein include those of various organisms such as coryneform bacteria and bacteria of the family *Enterobacteriaceae.* The nucleotide sequences of *lepB* genes possessed by various organisms and the amino acid sequences of LepB proteins encoded by them can be obtained from, for example, public databases such as NCBI (National Center for Biotechnology Information). The nucleotide sequence of the *lepB* gene of *C. glutamicum* ATCC 13869 is shown as SEQ ID NO: 1, and the amino acid sequence of the LepB protein encoded by this gene is shown as SEQ ID NO: 2. That is, the *lepB* gene may be, for example, a gene having the nucleotide sequence shown as SEQ ID NO: 1. Also, the LepB protein may be, for example, a protein having the amino acid sequence shown as SEQ ID NO: 2. The expression "having a (nucleotide or amino acid) sequence" means "comprising the (nucleotide or amino acid) sequence" unless otherwise stated, and also includes cases of "consisting of the (nucleotide or amino acid) sequence".

The *lepB* gene may be a variant of any of the *lepB* genes exemplified above (such as a gene having the nucleotide sequence shown as SEQ ID NO: 1), so long as the original function thereof is maintained. Similarly, the LepB protein may be a variant of any of the LepB proteins exemplified above (such as a protein having the amino acid sequence shown as SEQ ID NO: 2), so long as the original function thereof is maintained. Such a variant is also referred to as "conservative variant". In the present invention, the term *"lepB* gene" includes not only the *lepB* genes exemplified above, but also includes conservative variants thereof. Similarly, the term "LepB protein" includes not only the LepB proteins exemplified above, but also includes conservative variants thereof. Examples of the conservative variants include, for example, homologues and artificially modified versions of the *lepB* genes and LepB proteins exemplified above.

The expression "the original function is maintained" means that a variant of a gene or protein has a function (such as activity or property) corresponding to the function (such as activity or property) of the original gene or protein. That is, the expression "the original function is maintained" used for the *lepB* gene means that a variant of the gene encodes a protein that maintains the original function. Furthermore, the expression "the original function is maintained" used for the LepB protein means that a variant of the protein has the signal peptidase activity for the TorA signal peptide.

The signal peptidase activity can be measured by, for example, incubating the enzyme with a substrate, i.e. a protein having a signal peptide, and measuring the enzyme-dependent processing of the signal peptide. Processing of the signal peptide can be measured by, for example, using the molecular weight or the N-terminal amino acid sequence of the substrate after the incubation as an indicator.

Hereinafter, examples of the conservative variants will be explained.

Homologues of the *lepB* genes and homologues of the LepB proteins can be easily obtained from public databases by, for example, BLAST search or FASTA search using any of the nucleotide sequences of the *lepB* genes exemplified above or any of the amino acid sequences of the LepB proteins exemplified above as a query sequence. Furthermore, homologues of the *lepB* genes can be obtained by, for example, PCR using a chromosome of coryneform bacteria as the template, and oligonucleotides prepared on the basis of any of the nucleotide sequences of these known *lepB* genes as primers.

The *lepB* gene may be a gene encoding a protein having any of the amino acid sequences of the LepB proteins exemplified above (such as the amino acid sequence shown as SEQ ID NO: 2), but which includes substitution, deletion, insertion, and/or addition of one or several amino acid residues at one or several positions, so long as the original function thereof is maintained. Although the number meant by the term "one or several" mentioned above may differ depending on the positions of amino acid residues in the three-dimensional structure of the protein or the types of amino acid residues, specifically, it is, for example, 1 to 50, 1 to 40, 1 to 30, preferably 1 to 20, more preferably 1 to 10, still more preferably 1 to 5, particularly preferably 1 to 3.

The aforementioned substitution, deletion, insertion, and/or addition of one or several amino acid residues is a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the bacterium from which the gene is derived (mutant or variant).

The *lepB* gene may also be a gene encoding a protein having an amino acid sequence showing an identity of, for example, 80% or more, preferably 90% or more, more preferably 95% or more, still more preferably 97% or more, particularly preferably 99% or more, to the total amino acid sequence of any of the amino acid sequences of the LepB proteins exemplified above (such as the amino acid sequence shown as SEQ ID NO: 2), so long as the original function thereof is maintained.

The *lepB* gene may also be DNA that is able to hybridize under stringent conditions with a complementary sequence of any of the nucleotide sequences of the *lepB* genes exemplified above (such as the nucleotide sequence shown as SEQ ID NO: 1), or with a probe that can be prepared from the complementary sequence, so long as the original function thereof is maintained. The term "stringent conditions" refers to conditions under which a so-called specific hybrid is formed, and a non-specific hybrid is not formed. Examples of the stringent conditions include those under which highly identical DNAs hybridize to each other, for example, DNAs not less than 80% identical, preferably not less than 90% identical, more preferably not less than 95% identical, still more preferably not less than 97% identical, particularly preferably not less than 99% identical, hybridize to each other, and DNAs less identical than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe may be, for example, a part of a sequence that is complementary to the gene as described above. Such a probe can be prepared by PCR using oligonucleotides prepared on the basis of the nucleotide sequences of known genes as primers and a DNA fragment containing any of these nucleotide sequences as a template. As the probe, for example, a DNA fragment having a length of about 300 bp can be used. In such a case, the washing conditions of the hybridization may be, for example, 50°C, 2 x SSC and 0.1% SDS.

Furthermore, the *lepB* gene may be a gene having a nucleotide sequence corresponding to any of the nucleotide sequences of the *lepB* genes exemplified above or conservative variants thereof in which any codon(s) is/are replaced with respective equivalent codon(s). For example, the *lepB* gene may have been modified so as to have optimal codons according to the codon usage frequency of the host to be used.

The term "identity" between amino acid sequences means an identity between the amino acid sequences calculated by blastp with default scoring parameters (i.e. Matrix, BLOSUM62; Gap Costs, Existence = 11, Extension = 1; Compositional Adjustments, Conditional compositional score matrix adjustment), unless otherwise stated. The term "identity" between nucleotide sequences means an identity between the nucleotide sequences calculated by blastn with default scoring parameters (i.e. Match/Mismatch Scores = 1, -2; Gap Costs = Linear), unless otherwise stated.

The aforementioned descriptions concerning variants of the genes and proteins can also be applied *mutatis mutandis* to any proteins such as PhoRS proteins, cell surface layer protein, Tat secretion system, and heterologous proteins to be produced by secretory production in the present invention, and genes encoding them.

### <1-3> Other characteristics

The bacterium of the present invention may have desired characteristics, so long as it can produce a heterologous protein by secretory production. For example, the activity of a cell surface layer protein may have been reduced in the bacterium of the present invention (WO2013/065869, WO2013/065772, WO2013/118544, and WO2013/062029). Also, the bacterium of the present invention may have been modified so that the activity of a penicillin-binding protein is reduced (WO2013/065869). Also, the bacterium of the present invention may have been modified so that the expression of a gene encoding a metallopeptidase is increased (WO2013/065772). Also, the bacterium of the present invention may have been modified so as to have a mutant ribosomal protein S1 gene (mutant rpsA gene) (WO2013/118544). Also, the bacterium of the present invention may have been modified so as to have a mutant phoS gene (WO2016/171224). Also, the bacterium of the present invention may have been modified so that the activity of a RegX3 protein is reduced (WO2018/074578). Also, the bacterium of the present invention may have been modified so that the activity of a HrrSA system is reduced (WO2018/074579). Also, the bacterium of the present invention may have been modified so that the activity of the Tat secretion system is increased. These characteristics or modifications can be used solely or in any appropriate combination.

### <1-3-1> Introduction of mutant phoS gene

The bacterium of the present invention may have been modified so as to harbor a mutant *phoS* gene. The expression "to harbor a mutant *phoS* gene" is also referred to as "to have a mutant *phoS* gene" or "to have a mutation in a *phoS* gene". In addition, the expression "to harbor a mutant *phoS* gene" is also referred to as "to have a mutant PhoS protein" or "to have a mutation in a PhoS protein".

Hereinafter, the *phoS* gene and the PhoS protein will be explained. The *phoS* gene is a gene encoding a PhoS protein, which is a sensor kinase of the PhoRS system. The PhoRS system is one of two-component regulatory systems, and induces a response against phosphate depletion. The PhoRS system consists of a sensor kinase PhoS encoded by a *phoS* gene and a response regulator PhoR encoded by a *phoR* gene.

In the present invention, a PhoS protein having the "specific mutation" is also referred to as "mutant PhoS protein", and a gene encoding it is also referred to as "mutant *phoS* gene". The mutant *phoS* gene is, in other words, a *phoS* gene having the "specific mutation". Furthermore, in the present invention, a PhoS protein not having the "specific mutation" is also referred to as "wild-type PhoS protein", and a gene encoding it is also referred to as "wild-type *phoS* gene". The wild-type *phoS* gene is, in other words, a *phoS* gene not having the "specific mutation". The term "wild-type" referred to herein is used for convenience to distinguish "wild-type" ones from "mutant" ones, and "wild-type" ones are not limited to those obtained as natural substances, so long as those do not have the "specific mutation". The "specific mutation" will be described later.

Examples of the wild-type *phoS* gene include, for example, *phoS* genes of coryneform bacteria. Specific examples of the *phoS* genes of coryneform bacteria include, for example, the *phoS* genes of *C*. *glutamicum* YDK010, *C. glutamicum* ATCC 13032, *C. glutamicum* ATCC 14067, *C*. *callunae, C. crenatum,* and *C*. *efficiens.* The nucleotide sequence of the *phoS* gene of *C. glutamicum* YDK010 is shown as SEQ ID NO: 28. The amino acid sequences of the wild-type PhoS proteins encoded by these *phoS* genes are shown as SEQ ID NOS: 29 to 34, respectively.

The wild-type *phoS* gene may be a variant of any of the wild-type *phoS* genes exemplified above, so long as it does not have the "specific mutation" and the original function thereof is maintained. Similarly, the wild-type PhoS protein may be a variant of any of the wild-type PhoS proteins exemplified above, so long as it does not have the "specific mutation" and the original function thereof is maintained. That is, the term "wild-type *phoS* gene" includes not only the wild-type *phoS* genes exemplified above, but also includes conservative variants thereof that do not have the "specific mutation". Similarly, the term "wild-type PhoS protein" includes not only the wild-type PhoS proteins exemplified above, but also includes conservative variants thereof that do not have the "specific mutation". The aforementioned descriptions concerning conservative variants of the LepB protein and the *lepB* gene can be applied *mutatis mutandis* to variants of the wild-type PhoS protein and the wild-type *phoS* gene. For example, the wild-type *phoS* gene may also be a gene encoding a protein having any of the aforementioned amino acid sequences, but which includes substitution, deletion, insertion, and/or addition of one or several amino acid residues at one or several positions, so long as it does not have the "specific mutation" and the original function thereof is maintained. Also, for example, the wild-type *phoS* gene may also be a gene encoding a protein having an amino acid sequence showing an identity of 80% or more, preferably 90% or more, more preferably 95% or more, still more preferably 97% or more, particularly preferably 99% or more, to the total amino acid sequence of any of the aforementioned amino acid sequences, so long as it does not have the "specific mutation" and the original function thereof is maintained.

Incidentally, the expression "the original function is maintained" used for the wild-type PhoS may mean that a variant of the protein has a function as a PhoS protein (such as a function of a protein consisting of any of the amino acid sequences shown as SEQ ID NOS: 29 to 34). Furthermore, the expression "the original function is maintained" used for the wild-type PhoS protein may also mean that a variant of the protein has a function as a sensor kinase of the PhoRS system. That is, the term "function as a PhoS protein" may specifically refer to a function as a sensor kinase of the PhoRS system. The term "function as a sensor kinase of the PhoRS system" may specifically refer to a function of inducing a response against phosphate depletion in the environment in combination with a response regulator PhoR protein. The term "function as a sensor kinase of the PhoRS system" may more specifically refer to a function of sensing phosphate depletion in the environment to be autophosphorylated, and activating the PhoR protein via transfer of phosphate group.

Whether or not a variant of the PhoS protein has a function as a sensor kinase of the PhoRS system can be confirmed by, for example, introducing a gene encoding the variant into a *phoS*-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the *phoS*-gene-deletion strain of a coryneform bacterium, for example, a *phoS*-gene-deletion strain of *C*. *glutamicum* YDK010 or a *phoS*-gene-deletion strain of *C. glutamicum* ATCC 13032 can be used.

It is preferred that a histidine residue that is autophosphorylated is conserved in the wild-type PhoS protein. That is, it is preferred that a conservative mutation occurs at an amino acid residue other than the histidine residue that is autophosphorylated. The term "histidine residue that is autophosphorylated" refers to a histidine residue at position 276 of the wild-type PhoS protein. Furthermore, it is preferred that, for example, the wild-type PhoS protein has a conservative sequence of the wild-type PhoS proteins exemplified above. That is, it is preferred that a conservative mutation occurs at, for example, an amino acid residue not conserved in the wild-type PhoS proteins exemplified above.

The mutant PhoS protein has the "specific mutation" in the amino acid sequence of such a wild-type PhoS protein as described above.

That is, in other words, the mutant PhoS protein may be identical to any of the wild-type PhoS proteins exemplified above or conservative variants thereof except that the mutant PhoS protein has the "specific mutation". Specifically, the mutant PhoS protein may be, for example, a protein having any of the amino acid sequences shown in SEQ ID NOS: 29 to 34 except that the mutant PhoS protein has the "specific mutation". Specifically, the mutant PhoS protein may also be, for example, a protein having any of the amino acid sequences shown in SEQ ID NOS: 29 to 34 but including substitution, deletion, insertion, and/or addition of one or several amino acid residues, except that the mutant PhoS protein has the "specific mutation". Specifically, the mutant PhoS protein may also be, for example, a protein showing an identity of 80% or more, preferably 90% or more, more preferably 95% or more, still more preferably 97% or more, particularly preferably 99% or more, to any of the amino acid sequences shown in SEQ ID NOS: 29 to 34 except that the mutant PhoS protein has the "specific mutation".

Furthermore, in other words, the mutant PhoS protein may be a variant of any of the wild-type PhoS proteins exemplified above having the "specific mutation", and further including a conservative mutation at a site other than that of the "specific mutation". Specifically, the mutant PhoS protein may be, for example, a protein having any of the amino acid sequences shown in SEQ ID NOS: 29 to 34 but having the "specific mutation", and further including substitution, deletion, insertion, and/or addition of one or several amino acid residues at a site other than that of the "specific mutation".

The mutant *phoS* gene is not particularly limited so long as it encodes such a mutant PhoS protein as described above.

Hereinafter, the "specific mutation" of the mutant PhoS protein will be explained.

The "specific mutation" is not particularly limited, so long as it is a mutation that changes the amino acid sequence of such a wild-type PhoS protein described above, and that is effective for secretory production a heterologous protein.

It is preferred that the "specific mutation" is a mutation that improves the secretory production amount of a heterologous protein. The expression "to improve the secretory production amount of a heterologous protein" means that a coryneform bacterium modified so as to have a mutant *phoS* gene (modified strain) is able to produce the heterologous protein by secretory production in an amount larger than that obtainable with a non-modified strain. The "non-modified strain" refers to a control strain not having the "specific mutation" in the *phoS* gene, i.e. a control strain not having any mutant *phoS* gene, and it may be, for example, a wild-type strain or a parent strain. Although the degree of increase meant by the expression "to produce a heterologous protein by secretory production in an amount larger than that obtainable with a non-modified strain" is not particularly limited so long as the secretory production amount of the heterologous protein is increased compared with that obtainable with a non-modified strain, the expression may mean that the heterologous protein is produced by secretory production in an amount of, for example, preferably 1.1 times or more, more preferably 1.2 times or more, still more preferably 1.3 times or more, still more preferably 2 times or more, particularly preferably 5 times or more, of that obtainable with a non-modified strain, in terms of the accumulation amount in the medium and/or on the cell surface layer. In addition, the expression "to produce a heterologous protein by secretory production in an amount larger than that obtainable with a non-modified strain" may also mean that whereas the heterologous protein cannot be detected when a non-concentrated culture supernatant of a non-modified strain is applied to SDS-PAGE and stained with CBB, the heterologous protein can be detected when a non-concentrated culture supernatant of a modified strain is applied to SDS-PAGE and stained with CBB. Incidentally, the expression "to improve the secretory production amount of a heterologous protein" does not necessarily mean that the secretory production amount of every heterologous protein is improved, and it is sufficient that the secretory production amount of a heterologous protein chosen as the target of secretory production is improved. The expression "to improve the secretory production amount of a heterologous protein" may specifically mean, for example, that the secretory production amount of a heterologous protein described in the Example section is improved.

Whether a certain mutation is a mutation that improves the secretory production amount of a heterologous protein can be confirmed by, for example, preparing a strain modified so as to have a gene encoding the PhoS protein having the certain mutation from a strain belonging to a coryneform bacterium, quantifying the amount of the heterologous protein produced by secretory production when the strain is cultured in a medium, and comparing it with the amount of the heterologous protein produced by secretory production when the strain before the modification (non-modified strain) is cultured in the medium.

Preferred examples of the change of the amino acid sequence include substitution of an amino acid residue. That is, it is preferred that the "specific mutation" is a mutation of replacing an amino acid residue of the wild-type PhoS protein with another amino acid residue. The amino acid residue substituted by the "specific mutation" may be one residue, or may be a combination of two or more residues. The amino acid residue substituted by the "specific mutation" may preferably be an amino acid residue other than the histidine residue that is autophosphorylated. The amino acid residue substituted by the "specific mutation" may more preferably be an amino acid residue in the HisKA domain other than the histidine residue that is autophosphorylated. The term "histidine residue that is autophosphorylated" refers to a histidine residue at position 276 of the wild-type PhoS protein. The term "HisKA domain" refers to a region consisting of amino acid residues at positions 266-330 of the wild-type PhoS protein. The amino acid residue substituted by the "specific mutation" may particularly preferably be a tryptophan residue at position 302 of the wild-type PhoS protein (W302).

In the aforementioned mutation, examples of the amino acid residue after substitution include K(Lys), R(Arg), H(His), A(Ala), V(Val), L(Leu), I(Ile), G(Gly), S(Ser), T(Thr), P(Pro), F(Phe), W(Trp), Y(Tyr), C(Cys), M(Met), D(Asp), E(Glu), N(Asn), and Q(Gln), provided that the amino acid residue after substitution is other than the original one. As the amino acid residue after substitution, for example, one resulting in improvement in the secretory production amount of a heterologous protein can be chosen.

When substitution occurs at W302, examples of the amino acid residue after substitution include amino acid residues other than aromatic amino acid and histidine residues. Specific examples of the "amino acid residues other than aromatic amino acid and histidine residues" include K(Lys), R(Arg), A(Ala), V(Val), L(Leu), I(Ile), G(Gly), S(Ser), T(Thr), P(Pro), C(Cys), M(Met), D(Asp), E(Glu), N(Asn), and Q(Gln). More specific examples of the "amino acid residues other than aromatic amino acid and histidine residues" include K(Lys), A(Ala), V(Val), S(Ser), C(Cys), M(Met), D(Asp), and N(Asn).

Incidentally, the term "specific mutation" used for the *phoS* gene refers to a mutation on the nucleotide sequence thereof that results in such a "specific mutation" as described above into the encoded PhoS protein.

In the present invention, the "amino acid residue at position X of the wild-type PhoS protein" refers to an amino acid residue corresponding to the amino acid residue at position X in SEQ ID NO: 29. For example, "W302" refers to an amino acid residue corresponding to the tryptophan residue at position 302 in SEQ ID NO: 29. The aforementioned positions of amino acid residues indicate relative positions, and the absolute positions thereof may shift due to deletion, insertion, addition, or the like of an amino acid residue or residues. For example, if one amino acid residue is deleted or inserted at a position on the N-terminal side of position X in the wild-type PhoS protein consisting of the amino acid sequence shown as SEQ ID NO: 29, the amino acid residue originally at position X is relocated at position X-1 or X+1 counted from the N-terminus, however, it is still regarded as the "amino acid residue at position X of the wild-type PhoS protein". Specifically, for example, "W302" refers to the tryptophan residue at positions 302, 302, 302, 321, 275, and 286, respectively, in the amino acid sequences of wild-type PhoS proteins shown in SEQ ID NOS: 29 to 34. Furthermore, the "histidine residue at position 276 of the wild-type PhoS protein (histidine residue that is autophosphorylated)" refers to the histidine residue at positions 276, 276, 276, 295, 249, and 260, respectively, in the amino acid sequences of wild-type PhoS proteins shown in SEQ ID NOS: 29 to 34. Furthermore, the "region consisting of amino acid residues at positions 266-330 of the wild-type PhoS protein (HisKA domain)" refers to the region consisting of amino acid residues at positions 266-330, 266-330, 266-330, 285-349, 239-303, and 250-314, respectively, in the amino acid sequences of wild-type PhoS proteins shown in SEQ ID NOS: 29 to 34.

Incidentally, while "W302" referred to herein is typically a tryptophan residue, it may also be other than a tryptophan residue. That is, when the wild-type PhoS protein has an amino acid sequence other than the amino acid sequences shown in SEQ ID NOS: 29 to 34, "W302" can be other than a tryptophan residue. Hence, for example, the "mutation replacing W302 with a cysteine residue" includes not only a mutation, when "W302" is a tryptophan residue, for replacing this tryptophan residue with a cysteine residue, but also includes a mutation, when "W302" is K(Lys), R(Arg), H(His), A(Ala), V(Val), L(Leu), I(Ile), G(Gly), S(Ser), T(Thr), P(Pro), F(Phe), Y(Tyr), M(Met), D(Asp), E(Glu), N(Asn), or Q(Gln), for replacing this residue with a cysteine residue. The same can be applied *mutatis mutandis* to the other mutations.

Which amino acid residue is the "amino acid residue corresponding to the amino acid residue at position X in SEQ ID NO: 29" in the amino acid sequence of an arbitrary PhoS protein can be determined by alignment between the amino acid sequence of the arbitrary PhoS protein and the amino acid sequence of SEQ ID NO: 29. The alignment can be performed by, for example, using known gene analysis software. Specific examples of such software include DNASIS produced by Hitachi Solutions, GENETYX produced by Genetyx, and so forth (Elizabeth C. Tyler et al., Computers and Biomedical Research, 24 (1) 72-96, 1991; Barton GJ et al., Journal of Molecular Biology, 198 (2), 327-37, 1987).

The mutant *phoS* gene can be obtained by, for example, modifying a wild-type *phoS* gene so that the encoded PhoS protein has the aforementioned "specific mutation". The wild-type *phoS* gene to be modified can be obtained by, for example, cloning from an organism having the wild-type *phoS* gene, or chemical synthesis. Furthermore, the mutant *phoS* gene can also be obtained without using a wild-type *phoS* gene. For example, the mutant *phoS* gene may be directly obtained by chemical synthesis. The obtained mutant *phoS* gene may be further modified before use.

Genes can be modified by known methods. For example, an objective mutation can be introduced into a target site of DNA by the site-specific mutagenesis method. Examples of the site-specific mutagenesis method include a method of using PCR (Higuchi, R., 61, in PCR Technology, Erlich, H.A. Eds., Stockton Press (1989); Carter P., Meth. In Enzymol., 154, 382 (1987)), and a method of using a phage (Kramer, W. and Frits, H.J., Meth. in Enzymol., 154, 350 (1987); Kunkel, T.A. et al., Meth. in Enzymol., 154, 367 (1987)).

Hereinafter, methods for modifying a coryneform bacterium so as to have a mutant *phoS* gene will be explained.

A coryneform bacterium can be modified so as to have a mutant *phoS* gene by introducing the mutant *phoS* gene into the coryneform bacterium. A coryneform bacterium can be modified so as to have a mutant *phoS* gene also by introducing a mutation into the *phoS* gene on the chromosome of the coryneform bacterium. A mutation can be introduced into a gene on a chromosome by natural mutation, mutagenesis treatment, or genetic engineering means.

Methods for introducing a mutant *phoS* gene into a coryneform bacterium are not particularly limited. It is sufficient that the mutant *phoS* gene is harbored by the bacterium of the present invention so that it can be expressed under control of a promoter that functions in a coryneform bacterium. The promoter may be a promoter derived from the host, or a heterogenous promoter. The promoter may be the native promoter of the *phoS* gene, or a promoter of another gene. In the bacterium of the present invention, the mutant *phoS* gene may be present on a vector that autonomously replicates out of the chromosome, such as plasmid, or may be incorporated into the chromosome. The bacterium of the present invention may have only one copy of the mutant *phoS* gene, or two or more copies of the mutant *phoS* gene. The bacterium of the present invention may have only one kind of mutant *phoS* gene, or two or more kinds of mutant *phoS* genes. The mutant *phoS* gene can be introduced, for example, in the same manner as that for introduction of a gene in methods for increasing the expression of a gene described below, or for introduction of the genetic construct used for the present invention described below.

The bacterium of the present invention may or may not have the wild-type *phoS* gene. It is preferred that the bacterium of the present invention does not have the wild-type *phoS* gene.

A coryneform bacterium not having the wild-type *phoS* gene can be obtained by disrupting the wild-type *phoS* gene on the chromosome. The wild-type *phoS* gene can be disrupted by known methods. Specifically, the wild-type *phoS* gene can be disrupted by, for example, deleting a part or the whole of the promoter region and/or the coding region of the wild-type *phoS* gene.

Furthermore, by replacing the wild-type *phoS* gene on the chromosome with a mutant *phoS* gene, a coryneform bacterium modified so that it does not have the wild-type *phoS* gene and has the mutant *phoS* gene can be obtained. Examples of methods for performing such gene substitution include, for example, a method of using a linear DNA such as a method called "Red-driven integration" (Datsenko, K.A, and Wanner, B.L., Proc. Natl. Acad. Sci. USA, 97:6640-6645 (2000)), a method of utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method of using a plasmid including a temperature sensitive replication origin, a method of using a plasmid capable of conjugative transfer, a method of utilizing a suicide vector not including a replication origin that functions in a host (U.S. Patent No. 6,303,383, Japanese Patent Laid-open (Kokai) No. 05-007491), and so forth.

The PhoS protein functions, i.e. induces a response against phosphate depletion in the environment, in combination with a response regulator PhoR protein. Hence, the bacterium of the present invention has a *phoR* gene so that the mutant PhoS protein functions. The *phoR* gene is a gene encoding a PhoR protein, which is a response regulator of the PhoRS system. The expression "to have a *phoR* gene" is also referred to as "to have a PhoR protein". Typically, it is sufficient that the PhoR protein inherently possessed by the bacterium of the present invention functions in combination with the mutant PhoS protein. Alternatively, the bacterium of the present invention may be introduced with an appropriate *phoR* gene, in addition to or instead of the *phoR* gene inherently possessed by the bacterium of the present invention. The *phoR* gene to be introduced is not particularly limited, as long as it encodes a PhoR protein that functions in combination with the mutant PhoS protein.

Examples of the *phoR* gene include, for example, *phoR* genes of coryneform bacteria. Specific examples of the *phoR* genes of coryneform bacteria include, for example, the *phoR* genes of *C*. *glutamicum* YDK010, *C. glutamicum* ATCC 13032, *C. glutamicum* ATCC 14067, *C. callunae, C. crenatum,* and *C*. *efficiens.* The nucleotide sequence of the *phoR* gene of *C. glutamicum* ATCC 13032 and the amino acid sequence of the PhoR protein of the same are shown as SEQ ID NO: 35 and 36, respectively.

The *phoR* gene may be a variant of any of the *phoR* genes exemplified above, so long as the original function thereof is maintained. Similarly, the PhoR protein may be a variant of any of the PhoR proteins exemplified above, so long as the original function thereof is maintained. That is, the term *"phoR* gene" includes not only the *phoR* genes exemplified above, but also includes conservative variants thereof. Similarly, the term "PhoR protein" includes not only the PhoR proteins exemplified above, but also includes conservative variants thereof. The aforementioned descriptions concerning conservative variants of the LepB protein and the *lepB* gene can be applied *mutatis mutandis* to variants of the *phoR* gene and PhoR protein. For example, the *phoR* gene may be a gene encoding a protein having the aforementioned amino acid sequence, but including substitution, deletion, insertion, and/or addition of one or several amino acid residues at one or several positions, so long as the original function is maintained. Also, for example, the *phoR* gene may also be a gene encoding a protein having an amino acid sequence showing an identity of 80% or more, preferably 90% or more, more preferably 95% or more, still more preferably 97% or more, particularly preferably 99% or more, to the total amino acid sequence of any of the aforementioned amino acid sequences, so long as the original function is maintained. Incidentally, the expression "the original function is maintained" used for the PhoR protein may mean that a variant of the protein has a function as a PhoR protein (such as a function of a protein consisting of the amino acid sequence shown as SEQ ID NO: 36). Furthermore, the expression "the original function is maintained" used for the PhoR protein may also mean that a variant of the protein has a function as a response regulator of the PhoRS system. That is, the term "function as a PhoR protein" may specifically refer to a function as a response regulator of the PhoRS system. The term "function as a response regulator of the PhoRS system" may specifically refer to a function of inducing a response against phosphate depletion in the environment in combination with a sensor kinase PhoS protein. The term "function as a response regulator of the PhoRS system" may more specifically refer to a function of being activated via transfer of phosphate group from the PhoS protein that sensed phosphate depletion in the environment to be autophosphorylated, and regulating the expression of genes that respond to phosphate depletion in the environment.

Whether or not a variant of the PhoR protein has a function as a response regulator of the PhoRS system can be confirmed by, for example, introducing a gene encoding the variant into a *phoR*-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the *phoR*-gene-deletion strain of a coryneform bacterium, for example, a *phoR*-gene-deletion strain of *C. glutamicum* YDK010 or a *phoR*-gene-deletion strain of *C. glutamicum* ATCC 13032 can be used.

### <1-3-2> Reduction in activity of cell surface layer protein

The bacterium of the present invention may be a bacterium of which the activity(s) of cell surface layer protein(s) is/are reduced. Specifically, the bacterium of the present invention may be a bacterium of which the activity(s) of cell surface layer protein(s) is/are reduced as compared with a non-modified strain. The phrase "the activity of a cell surface layer protein is reduced" may particularly mean that the number of molecules of the cell surface layer protein per cell is reduced. Hereinafter, the cell surface layer proteins and genes encoding them will be explained.

The cell surface layer protein is a protein constituting the surface layer (S layer) of bacteria or archaea. Examples of cell surface layer proteins of coryneform bacteria include PS1 and PS2 (CspB) of *C. glutamicum* (Japanese Patent Laid-open (Kohyo) No. 6-502548), and SlpA (CspA) of *C. stationis* (Japanese Patent Laid-open (Kokai) No. 10-108675). It is preferable to reduce the activity of the PS2 protein among these.

The nucleotide sequence of the *cspB* gene of *C. glutamicum* ATCC 13869 and the amino acid sequence of the PS2 protein (CspB protein) encoded by the gene are shown in SEQ ID NOS: 37 and 38, respectively.

Furthermore, for example, amino acid sequences of CspB homologues were reported for 28 strains of *C .glutamicum* (J. Biotechnol., 112, 177-193 (2004)). These 28 strains of *C*. *glutamicum* and the GenBank accession numbers of the *cspB* gene homologues in NCBI database are exemplified below (the GenBank accession numbers are shown in the parentheses).
*C. glutamicum* ATCC 13058 (AY524990)
*C. glutamicum* ATCC 13744 (AY524991)
*C. glutamicum* ATCC 13745 (AY524992)
*C. glutamicum* ATCC 14017 (AY524993)
*C. glutamicum* ATCC 14020 (AY525009)
*C. glutamicum* ATCC 14067 (AY524994)
*C. glutamicum* ATCC 14068 (AY525010)
*C*. *glutamicum* ATCC 14747 (AY525011)
*C*. *glutamicum* ATCC 14751 (AY524995)
*C*. *glutamicum* ATCC 14752 (AY524996)
*C*. *glutamicum* ATCC 14915 (AY524997)
*C*. *glutamicum* ATCC 15243 (AY524998)
*C*. *glutamicum* ATCC 15354 (AY524999)
*C*. *glutamicum* ATCC 17965 (AY525000)
*C*. *glutamicum* ATCC 17966 (AY525001)
*C*. *glutamicum* ATCC 19223 (AY525002)
*C*. *glutamicum* ATCC 19240 (AY525012)
*C*. *glutamicum* ATCC 21341 (AY525003)
*C*. *glutamicum* ATCC 21645 (AY525004)
*C. glutamicum* ATCC 31808 (AY525013)
*C*. *glutamicum* ATCC 31830 (AY525007)
*C*. *glutamicum* ATCC 31832 (AY525008)
*C*. *glutamicum* LP-6 (AY525014)
*C*. *glutamicum* DSM20137 (AY525015)
*C*. *glutamicum* DSM20598 (AY525016)
*C. glutamicum* DSM46307 (AY525017)
*C. glutamicum* 22220 (AY525005)
*C. glutamicum* 22243 (AY525006)

Since the nucleotide sequence of a gene encoding a cell surface layer protein may differ depending on species or strain to which the coryneform bacterium belongs, the gene encoding a cell surface layer protein may be a variant of any of genes encoding the cell surface layer proteins exemplified above, so long as the original function thereof is maintained. Similarly, the cell surface layer protein may be a variant of any of the cell surface layer proteins exemplified above, so long as the original function thereof is maintained. That is, the term "*cspB* gene" includes not only the *cspB* genes exemplified above, but also includes conservative variants thereof. Similarly, the term "CspB protein" includes not only the CspB proteins exemplified above, but also includes conservative variants thereof. The aforementioned descriptions concerning conservative variants of the LepB protein and the *lepB* gene can be applied *mutatis mutandis* to variants of the cell surface layer protein and the gene encoding it. For example, the gene encoding the cell surface layer protein may be a gene encoding a protein having the aforementioned amino acid sequence, but including substitution, deletion, insertion, and/or addition of one or several amino acid residues at one or several positions, so long as the original function is maintained. Also, for example, the gene encoding the cell surface layer protein may also be a gene encoding a protein having an amino acid sequence showing an identity of 80% or more, preferably 90% or more, more preferably 95% or more, still more preferably 97% or more, particularly preferably 99% or more, to the total amino acid sequence of any of the aforementioned amino acid sequences, so long as the original function is maintained. Incidentally, the expression "original function is maintained" used for the cell surface layer protein may mean that the protein has a property that if the activity of the protein is reduced in a coryneform bacterium, the secretory production amount of a heterologous protein is increased compared with that obtainable with a non-modified strain.

The "property that if the activity of the protein is reduced in a coryneform bacterium, the secretory production amount of a heterologous protein is increased compared with that obtainable with a non-modified strain" refers to a property imparting an ability to produce a heterologous protein by secretory production in an amount larger than that obtainable with a non-modified strain to a coryneform bacterium when the activity thereof is reduced in the coryneform bacterium. The "non-modified strain" refers to a control strain of which the activity(s) of cell surface layer protein(s) is/are not reduced, and it may be, for example, a wild-type strain or a parent strain. Although the degree of increase meant by the expression "to produce a heterologous protein by secretory production in an amount larger than that obtainable with a non-modified strain" is not particularly limited so long as the secretory production amount of the heterologous protein is increased compared with that obtainable with a non-modified strain, the expression may mean that the heterologous protein is produced by secretory production in an amount of, for example, preferably 1.1 times or more, more preferably 1.2 times or more, still more preferably 1.3 times or more, particularly preferably 2 times or more, of that obtainable with a non-modified strain, in terms of the accumulation amount in the medium and/or on the cell surface layer. In addition, the expression "to produce a heterologous protein by secretory production in an amount larger than that obtainable with a non-modified strain" may also mean that whereas the heterologous protein cannot be detected when a non-concentrated culture supernatant of a non-modified strain is applied to SDS-PAGE and stained with CBB, the heterologous protein can be detected when a non-concentrated culture supernatant of a modified strain is applied to SDS-PAGE and stained with CBB.

Whether a protein has a property that if the activity of the protein is reduced in a coryneform bacterium, the secretory production amount of a heterologous protein is increased compared with that obtainable with a non-modified strain can be confirmed by preparing a strain modified so that the activity of the protein is reduced from a strain belonging to the coryneform bacteria, quantifying the secretory production amount of the heterologous protein observed when the modified strain is cultured in a medium, and comparing the quantified amount with the secretory production amount of the heterologous protein observed when the strain before being modified (un-modified strain) is cultured in the medium.

In the present invention, the expression "activity of a cell surface layer protein is reduced" includes a case where a coryneform bacterium has been modified so that the activity of a cell surface layer protein is reduced and a case where the activity of a cell surface layer protein is inherently reduced in a coryneform bacterium. The "case where activity of a cell surface layer protein is inherently reduced in a coryneform bacterium" includes a case where a coryneform bacterium is inherently deficient in a cell surface layer protein. That is, examples of a coryneform bacterium in which the activity of a cell surface layer protein is reduced include a coryneform bacterium that is inherently deficient in a cell surface layer protein. Examples of the "case where a coryneform bacterium is inherently deficient in a cell surface layer protein" include a case where a coryneform bacterium is inherently deficient in the gene encoding a cell surface layer protein. The expression "a coryneform bacterium is inherently deficient in a cell surface layer protein" may mean that a coryneform bacterium is inherently deficient in one or more proteins selected from cell surface layer protein(s) found in other strain(s) of the species to which the coryneform bacterium belongs. For example, *"C. glutamicum* is inherently deficient in a cell surface layer protein" may mean that a C. *glutamicum* strain is inherently deficient in one or more proteins selected from cell surface layer protein(s) found in other *C. glutamicum* strain(s), i.e. for example, deficient in PS1 and/or PS2 (CspB). Examples of the coryneform bacterium that is inherently deficient in a cell surface layer protein include *C. glutamicum* ATCC 13032, which is inherently deficient in the *cspB* gene.

### <1-3-3> Protein secretion system

The bacterium of the present invention has the Tat system (Tat secretion system), which is a protein secretion system. The bacterium of the present invention may inherently the Tat secretion system. Also, the bacterium of the present invention may have been modified so as to have the Tat secretion system. The bacterium of the present invention may have been modified so that the activity of the Tat secretion system is increased. Specifically, the bacterium of the present invention may have been modified so that the activity of the Tat secretion system is increased as compared with a non-modified strain. The activity of the Tat secretion system can be increased by, for example, increasing the expression of one or more genes selected from genes encoding the Tat secretion system. That is, more specifically, the bacterium of the present invention may have been modified so that the expression of one or more genes selected from genes encoding the Tat secretion system is increased. Methods for increasing the expression of genes encoding the Tat secretion system are described in Japanese Patent No. 4730302. By modifying a coryneform bacterium so that the activity of the Tat secretion system is increased, it is expected that an ability of the bacterium to produce a heterologous protein, specifically a heterologous protein in a completely-cleaved form, by secretory production when using the TorA signal peptide can be improved, that is, secretory production of a heterologous protein, specifically a heterologous protein in a completely-cleaved form, by the bacterium using the TorA signal peptide can be increased.

Examples of the genes encoding the Tat secretion system include *tatA, tatB, tatC,* and *tatE* genes.

Specific examples of the genes encoding the Tat secretion system include *tatA, tatB,* and *tatC* genes of *C. glutamicum.* The *tatA, tatB,* and *tatC* genes of *C. glutamicum* ATCC 13032 correspond to the complementary sequence of positions 1571065-1571382, the sequence of positions 1167110-1167580, and the complementary sequence of positions 1569929-1570873 in the genome sequence registered as GenBank accession NC_003450 (VERSION NC_003450.3 GI:58036263) in NCBI database, respectively. The TatA, TatB, and TatC proteins of *C. glutamicum* ATCC 13032 have been registered as GenBank accession NP_600707 (version NP_600707.1 GI:19552705, locus_tag="NCgl1434"), GenBank accession NP_600350 (version NP_600350.1 GI:19552348, locus_tag="NCgl1077"), and GenBank accession NP_600706 (version NP_600706.1 GI:19552704, locus_tag="NCgl1433"), respectively. The nucleotide sequences of the *tatA, tatB,* and *tatC* genes of C. *glutamicum* ATCC 13032 and the amino acid sequences of the TatA, TatB, and TatC proteins of the same are shown as SEQ ID NOS: 39 to 44.

Specific examples of the genes encoding the Tat secretion system also include *tatA, tatB, tatC,* and *tatE* genes of *E. coli.* The *tatA, tatB, tatC,* and *tatE* genes of *E. coli* K-12 MG1655 correspond to the sequence of positions 4019968-4020237, the sequence of positions 4020241-4020756, the sequence of positions 4020759-4021535, and the sequence of positions 658170-658373 in the genome sequence registered as GenBank accession NC_000913(VERSION NC_000913.2 GI:49175990) in NCBI database, respectively. The TatA, TatB, TatC, and TatE proteins of *E. coli* K-12 MG1655 have been registered as GenBank accession NP_418280 (version NP_418280.4 GI:90111653, locus_tag="b3836"), GenBank accession YP_026270 (version YP_026270.1 GI:49176428, locus_tag="b3838"), GenBank accession NP_418282 (version NP418282.1 GI:16131687, locus_tag="b3839"), and GenBank accession NP_415160 (version NP_415160.1 GI:16128610, locus_tag="b0627"), respectively.

The gene encoding the Tat secretion system may be a variant of any of the genes encoding the Tat-secretion-system exemplified above, so long as the original function thereof is maintained. Similarly, the Tat-secretion-system may be a variant of any of the Tat-secretion-systems exemplified above, so long as the original function thereof is maintained. That is, the terms "*tatA* gene", "*tatB* gene", *"tatC* gene", and *"tatE* gene" include not only the *tatA, tatB, tatC,* and *tatE* genes exemplified above, respectively, but also includes conservative variants thereof. Similarly, the terms "TatA protein", "TatB protein", "TatC protein", and "TatE protein" include not only the TatA, TatB, TatC, and TatE proteins exemplified above, respectively, but also includes conservative variants thereof. The aforementioned descriptions concerning conservative variants of the LepB protein and the *lepB* gene can be applied *mutatis mutandis* to variants of the Tat-secretion-system and the gene encoding it. For example, the gene encoding the Tat-secretion-system may be a gene encoding a protein having any of the aforementioned amino acid sequences, but including substitution, deletion, insertion, and/or addition of one or several amino acid residues at one or several positions, so long as the original function is maintained. Also, for example, the gene encoding the Tat-secretion-system may also be a gene encoding a protein having an amino acid sequence showing an identity of 80% or more, preferably 90% or more, more preferably 95% or more, still more preferably 97% or more, particularly preferably 99% or more, to the total amino acid sequence of any of the aforementioned amino acid sequences, so long as the original function is maintained. Incidentally, the expression "original function is maintained" used for the Tat-secretion-system may mean that the system has a function of secreting a protein fused with a Tat-dependent signal peptide, such as the TorA signal peptide, at the N-terminus out of the cell.

An increase in the activity of the Tat secretion system can be confirmed by, for example, confirming an increase in the secretory production amount of a protein fused with a Tat-dependent signal peptide, such as the TorA signal peptide, at the N-terminus. The secretory production amount of the protein fused with a Tat-dependent signal peptide at the N-terminus may be increased to, for example, 1.5 times or more, 2 times or more, or 3 times or more, of that of a non-modified strain.

### <1-4> Methods for increasing activity of protein

Hereinafter, the methods for increasing the activity of a protein such as the LepB protein, including the methods for increasing the expression of a gene, will be explained.

The expression "the activity of a protein is increased" means that the activity of the protein is increased as compared with a non-modified strain. Specifically, the expression "the activity of a protein is increased" means that the activity of the protein per cell is increased as compared with that of a non-modified strain. The term "non-modified strain" used herein refers to a control strain that has not been modified so that the activity of an objective protein is reduced. Examples of the non-modified strain include a wild-type strain and parent strain. Specific examples of the non-modified strain include the respective type strains of the species of bacteria. Specific examples of the non-modified strain also include strains exemplified above in relation to the description of coryneform bacteria. That is, in an embodiment, the activity of a protein may be increased as compared with a type strain, i.e. the type strain of the species to which the bacterium of the present invention belongs. In another embodiment, the activity of a protein may also be increased as compared with *C. glutamicum* ATCC 13869. In another embodiment, the activity of a protein may also be increased as compared with *C. glutamicum* ATCC 13032. In another embodiment, the activity of a protein may also be increased as compared with *C. glutamicum* AJ12036 (FERM BP-734). In another embodiment, the activity of a protein may also be increased as compared with *C. glutamicum* YDK010. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e. the amount of mRNA) encoding the protein, or the translation amount of the gene (i.e. the amount of the protein). The term "the number of molecules of a protein per cell" may mean an average value of the number of molecules of the protein per cell. Furthermore, the state that "the activity of a protein is increased" includes not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" means that the expression of the gene is increased as compared with a non-modified strain such as a wild-type strain and parent strain. Specifically, the expression "the expression of a gene is increased" means that the expression amount of the gene per cell is increased as compared with that of a non-modified strain. The term "the expression amount of a gene per cell" may mean an average value of the expression amount of the gene per cell. More specifically, the expression "the expression of a gene is increased" may mean that the transcription amount of the gene (i.e. the amount of mRNA) is increased, and/or the translation amount of the gene (i.e. the amount of the protein expressed from the gene) is increased. The state that "the expression of a gene is increased" may also be referred to as "the expression of a gene is enhanced". The expression of a gene may be increased to, for example, 1.5 times or more, 2 times or more, or 3 times or more of that of a non-modified strain. Furthermore, the state that "the expression of a gene is increased" includes not only a state that the expression amount of an objective gene is increased in a strain that inherently expresses the objective gene, but also a state that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J.H., Experiments in Molecular Genetics, 1972, Cold Spring Harbor Laboratory). Examples of the gene transfer method utilizing homologous recombination include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Specifically, a host can be transformed with a recombinant DNA containing an objective gene, so that homologous recombination occurs at a target region on the chromosome of the host, to thereby introduce the objective gene into the chromosome of the host. The structure of the recombinant DNA to be used for homologous recombination is not particularly limited as long as it causes homologous recombination in a desired manner. For example, a host can be transformed with a linear DNA containing an objective gene and further containing nucleotide sequences homologous to upstream and downstream of a target region on the chromosome at the respective ends of the objective gene, so that homologous recombination occurs at each of upstream and downstream of the target region, to thereby replace the target region with the objective gene. The recombinant DNA to be used for homologous recombination may contain a marker gene for selection of transformants. Only one copy, or two or more copies of a gene may be introduced. For example, by performing homologous recombination using a nucleotide sequence which is present in multiple copies on a chromosome as a target, multiple copies of a gene can be introduced into the chromosome. Examples of such a sequence which is present in multiple copies on a chromosome include repetitive DNAs, and inverted repeats located at the both ends of a transposon. Alternatively, homologous recombination may be performed by using an appropriate sequence on a chromosome such as a gene unnecessary for production of an objective substance as a target. Furthermore, a gene can also be randomly introduced into a chromosome by using a transposon or Mini-Mu (Japanese Patent Laid-open (Kokai) No. 2-109985, U.S. Patent No. 5,882,888, EP805867B1). Such methods for modifying a chromosome using homologous recombination can be used for any modification on a chromosome, such as a modification of an expression control sequence, as well as for introduction of an objective gene.

Introduction of an objective gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of a target gene can be increased by ligating a DNA fragment containing the target gene with a vector that functions in a host to construct an expression vector of the gene, and transforming the host with the expression vector. The DNA fragment containing the target gene can be obtained by, for example, PCR using the genomic DNA of a microorganism having the target gene as the template. As the vector, a vector autonomously replicable in the cell of the host can be used. The vector is preferably a multi-copy vector. Furthermore, the vector preferably has a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in coryneform bacteria include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 (Japanese Patent Laid-open (Kokai) No. 3-210184); plasmids pCRY21, pCRY2KE, pCRY2KX, pCRY31, pCRY3KE, and pCRY3KX (Japanese Patent Laid-open (Kokai) No. 2-72876 and U.S. Patent No. 5,185,262); plasmids pCRY2 and pCRY3 (Japanese Patent Laid-open (Kokai) No. 1-191686); pAJ655, pAJ611, and pAJ1844 (Japanese Patent Laid-open (Kokai) No. 58-192900); pCG1 (Japanese Patent Laid-open (Kokai) No. 57-134500); pCG2 (Japanese Patent Laid-open (Kokai) No. 58-35197); pCG4 and pCG11 (Japanese Patent Laid-open (Kokai) No. 57-183799); pVK7 (Japanese Patent Laid-open (Kokai) No. 10-215883); pVK9 (US2006-0141588); pVC7 (Japanese Patent Laid-open (Kokai) No. 9-070291); pVS7 (WO2013/069634).

When a gene is introduced, it is sufficient that the gene is expressibly harbored by a host. Specifically, it is sufficient that the gene is harbored by a host so that it is expressed under control by a promoter that functions in the host. The promoter is not particularly limited so long as it functions in the host. The term "promoter that functions in a host" refers to a promoter that shows a promoter activity in the host. The promoter may be a promoter derived from the host, or a heterogenous promoter. The promoter may be the native promoter of the gene to be introduced, or a promoter of another gene. As the promoter, for example, such a stronger promoter as mentioned later may also be used.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are expressibly harbored by the host. For example, all the genes may be carried by a single expression vector or a chromosome. Furthermore, the genes may be separately carried by two or more expression vectors, or separately carried by a single or two or more expression vectors and a chromosome. An operon constituted by two or more genes may also be introduced.

The gene to be introduced is not particularly limited so long as it encodes a protein that functions in the host. The gene to be introduced may be a gene derived from the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein include substitution, deletion, insertion, and/or addition of amino acid residues. Examples of the site-specific mutation method include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Incidentally, when a protein functions as a complex consisting of a plurality of subunits, a part or all of the plurality of subunits may be modified, so long as the activity of the protein is eventually increased. That is, for example, when the activity of a protein is increased by increasing the expression of a gene, the expression of a part or all of the plurality of genes that encode the subunits may be enhanced. It is usually preferable to enhance the expression of all of the plurality of genes encoding the subunits. Furthermore, the subunits constituting the complex may be derived from a single kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The term "expression control sequence" collectively refers to sites that affect the expression of a gene. Examples of the expression control sequence include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" refers to a promoter providing an improved transcription of a gene compared with an inherently existing wild-type promoter of the gene. Examples of stronger promoters usable in coryneform bacteria include, for example, the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, *cspB, SOD,* and *tuf*(EF-Tu) promoters, which are strong promoters capable of providing a large expression amount in coryneform bacteria (Journal of Biotechnology, 104 (2003) 311-323; Appl. Environ. Microbiol., 2005 Dec; 71 (12):8587-96), as well as *lac* promoter, *tac* promoter, and *trc* promoter. Furthermore, as the stronger promoter, a highly-active type of an existing promoter may also be obtained by using various reporter genes. For example, by making the -35 and -10 regions in a promoter region closer to the consensus sequence, the activity of the promoter can be enhanced (WO00/18935). Examples of highly active-type promoter include various tac-like promoters (Katashkina JI et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" means a SD sequence that provides an improved translation of mRNA compared with the inherently existing wild-type SD sequence of the gene. Examples of stronger SD sequences include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, so as to have optimal codons according to the codon usage frequency of a host to be used. Codons can be replaced by, for example, the site-specific mutation method. Alternatively, a gene fragment in which objective codons are replaced may be totally synthesized. Frequencies of codons in various organisms are disclosed in the "Codon Usage Database" (http://www.kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any appropriate combination.

Furthermore, the modification that increases the activity of a protein can also be attained by, for example, enhancing the specific activity of the protein. A protein showing an enhanced specific activity can be obtained by, for example, searching various organisms. Furthermore, a highly-active type of an existing protein may also be obtained by introducing a mutation into the existing protein. The mutation to be introduced may be, for example, substitution, deletion, insertion, and/or addition of one or several amino acid residues at one or several position of the protein. The mutation can be introduced by, for example, such a site-specific mutation method as mentioned above. The mutation may also be introduced by, for example, a mutagenesis treatment. Examples of the mutagenesis treatment include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any appropriate combination with such methods for enhancing gene expression as mentioned above.

The method for the transformation is not particularly limited, and conventionally known methods can be used. There can be used, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C.H., Wilson, G.A. and Young, F.E., Gene, 1977, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.5 times or more, 2 times or more, or 3 times or more of that of a non-modified strain.

An increase in the amount of a protein can be confirmed by Western blotting using antibodies (Sambrook, J., et al., Molecular Cloning A Laboratory Manual/Third Edition, Cold Spring Harbor Laboratory Press, Cold Spring Harbor (USA), 2001). The amount of the protein (such as the number of molecules of the protein per cell) may be increased to, for example, 1.5 times or more, 2 times or more, or 3 times or more of that of a non-modified strain.

The aforementioned methods for increasing the activity of a protein can be used for enhancement of the activities of any proteins and enhancement of the expression of any genes.

### <1-5> Method for reducing activity of protein

Hereinafter, methods for reducing the activity of a protein will be explained. The methods for reducing the activity of a protein described below can also be utilized for disruption of the wild-type PhoS protein.

The expression "the activity of a protein is reduced" means that the activity of the protein is reduced as compared with a non-modified strain. Specifically, the expression "the activity of a protein is reduced" means that the activity of the protein per cell is reduced as compared with that of a non-modified strain. The term "non-modified strain" used herein refers to a control strain that has not been modified so that the activity of an objective protein is reduced. Examples of the non-modified strain include a wild-type strain and parent strain. Specific examples of the non-modified strain include the respective type strains of the species of bacteria. Specific examples of the non-modified strain also include strains exemplified above in relation to the description of coryneform bacteria. That is, in an embodiment, the activity of a protein may be reduced as compared with a type strain, i.e. the type strain of the species to which the bacterium of the present invention belongs. In another embodiment, the activity of a protein may also be reduced as compared with *C. glutamicum* ATCC 13032. In another embodiment, the activity of a protein may also be reduced as compared with *C. glutamicum* ATCC 13869. In another embodiment, the activity of a protein may also be reduced as compared with *C. glutamicum* AJ12036 (FERM BP-734). In another embodiment, the activity of a protein may also be reduced as compared with *C. glutamicum* YDK010. The state that "the activity of a protein is reduced" also includes a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein, but may also mean the transcription amount of a gene (i.e. the amount of mRNA) encoding the protein or the translation amount of the gene (i.e. the amount of the protein). The phrase "the number of molecules of a protein per cell" may mean an average value per cell of the number of molecules of the protein. The state that "the number of molecules of the protein per cell is reduced" also includes a state that the protein does not exist at all. The state that "the function of each molecule of the protein is reduced" also includes a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" means that the expression of the gene is reduced as compared with a non-modified strain. Specifically, the expression "the expression of a gene is reduced" means that the expression amount of the gene per cell is reduced as compared with that of a non-modified strain. The phrase "the expression amount of a gene per cell" may mean an average value per cell of the expression amount of the gene. More specifically, the expression "the expression of a gene is reduced" may mean that the transcription amount of the gene (i.e. the amount of mRNA) is reduced, and/or the translation amount of the gene (i.e. the amount of the protein expressed from the gene) is reduced. The state that "the expression of a gene is reduced" also includes a state that the gene is not expressed at all. The state that "the expression of a gene is reduced" is also referred to as "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by modifying an expression control sequence of the gene. The term "expression control sequence" collectively refers to sites that affect the expression of a gene, such as a promoter, Shine-Dalgarno (SD) sequence (also referred to as ribosome-binding site (RBS)), and spacer region between RBS and the start codon. Expression control sequences can be identified by, for example, using a promoter search vector or gene analysis software such as GENETYX. When an expression control sequence is modified, preferably one or more nucleotides, more preferably two or more nucleotides, particularly preferably three or more nucleotides, of the expression control sequence are modified. The transcription efficiency of a gene can be reduced by, for example, replacing the promoter of the gene on a chromosome with a weaker promoter. The term "weaker promoter" means a promoter providing an attenuated transcription of a gene compared with an inherently existing wild-type promoter of the gene. Examples of weaker promoters include, for example, inducible promoters. That is, an inducible promoter may function as a weaker promoter under a non-induced condition, such as in the absence of the corresponding inducer. Furthermore, a part or the whole region of an expression control sequence may be deleted. The expression of a gene can also be reduced by, for example, manipulating a factor responsible for expression control. Examples of the factor responsible for expression control include low molecules responsible for transcription or translation control (inducers, inhibitors, etc.), proteins responsible for transcription or translation control (transcription factors etc.), nucleic acids responsible for transcription or translation control (siRNA etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described later.

The modification for reducing the activity of a protein can also be attained by, for example, disrupting a gene encoding the protein. The expression "a gene is disrupted" means that a gene is modified so that a protein that can normally function is not produced. The state that "a protein that normally functions is not produced" includes a state that the protein is not produced at all from the gene, and a state that the protein of which the function (such as activity or property) per molecule is reduced or eliminated is produced from the gene.

Disruption of a gene can be attained by, for example, deleting the gene on a chromosome. The term "deletion of a gene" refers to deletion of a partial or entire region of the coding region of the gene. Furthermore, the whole of a gene including sequences upstream and downstream from the coding region of the gene on a chromosome may be deleted. The sequences upstream and downstream from the coding region of the gene may include, for example, an expression control sequence of the gene. The region to be deleted may be any region such as an N-terminal region (region encoding an N-terminal region of a protein), an internal region, or a C-terminal region (region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing a mutation for an amino acid substitution (missense mutation), a stop codon (nonsense mutation), addition or deletion of one or two nucleotide residues (frame shift mutation), or the like into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the region to be deleted. The other nucleotide sequence is not particularly limited so long as a sequence that reduces or eliminates the activity of the encoded protein is chosen, and examples thereof include, for example, a marker gene such as antibiotic resistance genes, and a gene useful for production of an objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" refers to deletion of a partial or entire region of the amino acid sequence of the protein. In addition, the term "deletion of the amino acid sequence of a protein" means that the original amino acid sequence disappears in the protein, and also includes cases where the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a partial or entire region of the coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be applied *mutatis mutandis* to the position and length of the region to be deleted in deletion of the amino acid sequence of a protein.

Such modification of a gene on a chromosome as described above can be attained by, for example, preparing a disruption-type gene modified so that it is unable to produce a protein that normally functions, and transforming a host with a recombinant DNA containing the disruption-type gene to cause homologous recombination between the disruption-type gene and the wild-type gene on a chromosome and thereby substitute the disruption-type gene for the wild-type gene on the chromosome. In this procedure, if a marker gene selected according to the characteristics of the host such as auxotrophy is included in the recombinant DNA, the operation becomes easier. Examples of the disruption-type gene include a gene of which a partial or entire region of the coding region is deleted, gene including a missense mutation, gene including a nonsense mutation, gene including a frame shift mutation, and gene inserted with an insertion sequence such as a transposon or marker gene. The protein encoded by the disruption-type gene has a conformation different from that of the wild-type protein, even if it is produced, and thus the function thereof is reduced or eliminated. The structure of the recombinant DNA to be used for homologous recombination is not particularly limited as long as it causes homologous recombination in a desired manner. For example, a host can be transformed with a linear DNA containing the disruption-type gene and further containing upstream and downstream sequences of the wild-type gene on the chromosome at the respective ends, so that homologous recombination occurs at each of upstream and downstream sides of the wild-type gene, to thereby replace the wild-type gene with the disruption-type gene. Such gene disruption based on gene substitution utilizing homologous recombination has already been established, and there are methods of using a linear DNA such as a method called "Red driven integration" (Datsenko, K.A, and Wanner, B.L., Proc. Natl. Acad. Sci. USA, 97:6640-6645 (2000)), and a method utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Patent No. 6,303,383, Japanese Patent Laid-open (Kokai) No. 05-007491), and so forth. Such methods for modifying a chromosome using homologous recombination can be used for any modification on a chromosome, such as a modification of an expression control sequence, as well as for disruption of an objective gene.

Modification for reducing activity of a protein can also be attained by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

Such methods for reducing the activity of a protein as mentioned above may be used independently or in an arbitrary combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that of a non-modified strain. Examples of the method for evaluating the amount of mRNA include Northern hybridization, RT-PCR, microarray, RNA-Seq, and so forth (Sambrook, J., et al., Molecular Cloning: A Laboratory Manual/Third Edition, Cold Spring Harbor Laboratory Press, Cold Spring Harbor (USA), 2001). The amount of mRNA may be reduced to, for example, 50% or less, 20% or less, 10% or less, 5% or less, or 0% of that of a non-modified strain.

A reduction in the amount of a protein can be confirmed by performing SDS-PAGE and confirming the intensity of the separated protein band. A reduction in the amount of a protein can be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of the protein (such as the number of molecules of the protein per cell) may be reduced to, for example, 50% or less, 20% or less, 10% or less, 5% or less, or 0% of that of a non-modified strain.

Disruption of a gene can be confirmed by determining nucleotide sequence of a part or the whole of the gene, restriction enzyme map, full length, or the like of the gene depending on the means used for the disruption.

The aforementioned methods for reducing the activity of a protein as mentioned above can be applied to reduction in the activities of any proteins and reduction in the expression of any genes.

### <1-6> Genetic construct for secretory expression of heterologous protein and introduction of the same

It is known that a secretory protein is generally translated as a preprotein (also referred to as prepeptide) or a preproprotein (also referred to as prepropeptide), and then becomes a mature protein through processing. Specifically, a secretory protein is generally translated as a preprotein or preproprotein, then a signal peptide as the pre-moiety is cleaved with a protease (generally called signal peptidase), and the secretory protein is thereby converted into a mature protein or proprotein. As for the proprotein, the pro-moiety thereof is further cleaved by a protease, and the proprotein thereby becomes a mature protein. Therefore, a signal peptide is used for the secretory production of a heterologous protein in the method of the present invention. In the present invention, a preprotein and a preproprotein of a secretory protein may be collectively referred to as "secretory protein precursor".

The genetic construct used for the present invention comprises, in the direction from 5' to 3', a promoter sequence that functions in a coryneform bacterium, a nucleic acid sequence encoding a TorA signal peptide, and a nucleic acid sequence encoding a heterologous protein. The nucleic acid sequence encoding the TorA signal peptide may be ligated downstream from the promoter sequence so that the TorA signal peptide is expressed under the control of the promoter. The nucleic acid sequence encoding the heterologous protein may be ligated downstream from the nucleic acid sequence encoding the TorA signal peptide so that the heterologous protein is expressed as a fusion protein with the signal peptide. This fusion protein is also referred to as "fusion protein of the present invention". As described above, in the method of the present invention, a heterologous protein from which the TorA signal peptide has been completely removed, i.e. a heterologous protein in a completely-cleaved form, is produced by secretory production. That is, the heterologous protein eventually obtained does not has the TorA signal peptide. Hence, the expression "a heterologous protein is expressed as a fusion protein with a TorA signal peptide" means that the heterologous protein constitutes a fusion protein with the TorA signal peptide at the time of expression, and it does not mean that the eventually-obtained heterologous protein constitutes a fusion protein with the TorA signal peptide. A nucleic acid sequence may also be read as "gene". For example, a nucleic acid sequence encoding a heterologous protein is also referred to as "gene encoding a heterologous protein" or "heterologous protein gene". Examples of the nucleic acid sequence include DNA. The genetic construct used for the present invention may also comprise a control sequence (operator, SD sequence, terminator, etc.) effective for expression of the fusion protein of the present invention in a coryneform bacterium at such an appropriate position that it can function.

The promoter used in the present invention is not particularly limited so long as a promoter that functions in a coryneform bacterium is chosen. The promoter may be a promoter derived from a coryneform bacterium, such as one derived from the host, or it may be a heterologous promoter. The promoter may be the native promoter of the heterologous protein, or a promoter of another gene. The "promoter that functions in a coryneform bacterium" refers to a promoter that possesses promoter activity in a coryneform bacterium.

Specific examples of the heterologous promoter include, for example, promoters derived from *E. coli* such as *tac* promoter, *lac* promoter, *trp* promoter, and *araBAD* promoter. Among these, strong promoters such as *tac* promoter and inducible promoters such as *araBAD* promoter are preferred.

Examples of the promoter derived from a coryneform bacterium include, for example, promoters of the genes of the cell surface layer proteins PS1, PS2 (also referred to as CspB), and SlpA (also referred to as CspA), and promoters of various amino acid biosynthesis system genes. Specific examples of the promoters of various amino acid biosynthesis system genes include, for example, promoters of the glutamate dehydrogenase gene of the glutamic acid biosynthesis system, the glutamine synthetase gene of the glutamine synthesis system, the aspartokinase gene of the lysine biosynthesis system, the homoserine dehydrogenase gene of the threonine biosynthesis system, the acetohydroxy acid synthetase gene of the isoleucine and valine biosynthesis system, 2-isopropylmalate synthetase gene of the leucine biosynthesis system, the glutamate kinase gene of the proline and arginine biosynthesis system, the phosphoribosyl-ATP pyrophosphorylase gene of the histidine biosynthesis system, the deoxyarabinoheptulonate phosphate (DAHP) synthetase gene of the aromatic amino acid biosynthesis systems such as those for tryptophan, tyrosine, and phenylalanine, the phosphoribosyl pyrophosphate (PRPP) amidotransferase gene of the nucleic acid biosynthesis systems such as those for inosinic acid and guanylic acid, the inosinic acid dehydrogenase gene, and the guanylic acid synthetase gene.

Examples of the promoter that functions in a coryneform bacterium include such strong promoters as described above usable in coryneform bacteria. As the promoter, a high activity type of an existing promoter may be obtained by using various reporter genes, and used. For example, by making the -35 and -10 regions in a promoter region closer to a consensus sequence, activity of the promoter can be enhanced (International Patent Publication WO00/18935). Examples of the method for evaluating strength of a promoter and strong promoters are described in the paper of Goldstein et al. (Prokaryotic promoters in biotechnology, Biotechnol. Annu. Rev., 1, 105-128 (1995)) and so forth. Furthermore, it is known that substitution, insertion, or deletion of several nucleotides in a spacer region between the ribosome-binding site (RBS) and the start codon, especially in a sequence immediately upstream of the start codon (5'-UTR), significantly affects stability and translation efficiency of mRNA, and these sequences can also be modified.

The signal peptide used in the present invention is a TorA signal peptide. The TorA signal peptide is a Tat-dependent signal peptide. The term "Tat-dependent signal peptide" refers to a signal peptide recognized by the Tat system. The term "Tat-dependent signal peptide" may specifically refer to a signal peptide that, upon being linked at the N-terminus of an objective protein, results in secretion of the protein by the Tat secretion system. The Tat-dependent signal peptide has a twin-arginine motif. Examples of the twin-arginine motif include R-R-X-#-# (#: hydrophobic residue) (SEQ ID NO: 45). Examples of the TorA signal peptide include signal peptides of TorA proteins (trimethylamine-N-oxidoreductase) of bacteria of the family *Enterobacteriaceae* such as *E. coli.* The amino acid sequence of the TorA signal peptide *of E. coli* is "MNNNDLFQASRRRFLAQLGGLTVAGMLGPSLLTPRRATA" (SEQ ID NO: 46).

The TorA signal peptide may be a variant of any of the TorA signal peptides exemplified above, so long as it contains a twin-arginine motif and the original function thereof is maintained. The above descriptions concerning conservative variants of the LepB protein and the *lepB* gene can be applied *mutatis mutandis* to variants of the TorA signal peptide and the gene encoding it. For example, the TorA signal peptide may be a peptide having any of the amino acid sequences of the TorA signal peptides exemplified above, but including substitution, deletion, insertion, and/or addition of one or several amino acid residues at one or several positions. The number meant by the term "one or several" used for a variant of the TorA signal peptide is specifically, preferably 1 to 7, more preferably 1 to 5, still more preferably 1 to 3, particularly preferably 1 to 2. Also, for example, the TorA signal peptide may be a peptide having an amino acid sequence showing an identity of 80% or more, preferably 90% or more, more preferably 95% or more, still more preferably 97% or more, particularly preferably 99% or more, to the total amino acid sequence of any of the amino acid sequences of the TorA signal peptides exemplified above. In the present invention, the term "TorA signal peptide" includes not only the peptide of SEQ ID NO: 46, respectively, but also includes conservative variants thereof.

The expression "original function is maintained" used for the TorA signal peptide means that the peptide has a function as a Tat-dependent signal peptide. The expression "a peptide has a function as a Tat-dependent signal peptide" means that the peptide is recognized by the Tat system, and specifically, may mean that the peptide has a function of, upon being linked at the N-terminus of an objective protein, resulting in secretion of the protein by the Tat secretion system. Whether a peptide function as the Tat-dependent signal peptide can be confirmed by, for example, confirming an increase in the secretory production amount of a protein linked with the peptide at the N-terminus due to enhancement of the Tat secretion system, or confirming a reduction in the secretory production amount of a protein linked with the peptide at the N-terminus due to deletion of the Tat secretion system.

The nucleic acid sequence encoding a TorA signal peptide is not particularly limited so long as it encodes any of such TorA signal peptides as mentioned above.

Examples of the heterologous protein to be produced by secretory production according to the method of the present invention include, for example, physiologically active proteins, receptor proteins, antigenic proteins to be used as vaccines, enzymes, and any other proteins.

Examples of the enzymes include, for example, transglutaminase, protein glutaminase, isomaltodextranase, protease, endopeptidase, exopeptidase, aminopeptidase, carboxypeptidase, collagenase, chitinase, and so forth. Examples of transglutaminase include, for example, secretory-type transglutaminases of Actinomycetes such as *Streptoverticillium mobaraense* IFO 13819 (WO01/23591), *Streptoverticillium cinnamoneum* IFO 12852, *Streptoverticillium griseocarneum* IFO 12776, and *Streptomyces lydicus* (WO96/06931), and of filamentous fungi such as Oomycetes (WO96/22366). Examples of protein glutaminase include, for example, protein glutaminase of *Chryseobacterium proteolyticum* (WO2005/103278). Examples of isomaltodextranase include, for example, isomaltodextranase of *Arthrobacter globiformis* (WO2005/103278).

Examples of the physiologically active proteins include, for example, growth factors, hormones, cytokines, and antibody-related molecules.

Specific examples of the growth factors include, for example, epidermal growth factor (EGF), insulin-like growth factor-1 (IGF-1), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), acidic fibroblast growth factor (aFGF or FGF1), basic fibroblast growth factor (bFGF or FGF2), keratinocyte growth factor (KGF-1 or FGF7, and, KGF-2 or FGF10), and hepatocyte growth factor (HGF).

Specific examples of the hormones include, for example, insulin, glucagon, somatostatin, human growth hormone (hGH), parathyroid hormone (PTH), calcitonin, and exenatide.

Specific examples of the cytokines include, for example, interleukins, interferons, and tumor necrosis factors (TNFs).

The growth factors, hormones, and cytokines may not be strictly distinguished from one another. For example, a physiologically active protein may be a protein belonging to a single group selected from growth factors, hormones, and cytokines, or may be a protein belonging to a plurality of groups selected from those.

Furthermore, a physiologically active protein may be an intact protein, or may be a part of a protein. Examples of a part of a protein include, for example, a part having physiological activity. Specific examples of a part having physiological activity include, for example, Teriparatide, a physiologically active peptide consisting of the N-terminal 34 amino acid residues of parathyroid hormone (PTH).

The term "antibody-related molecule" refers to a protein containing a molecular species consisting of a single domain or a combination of two or more domains selected from the domains constituting a complete antibody. Examples of the domains constituting a complete antibody include heavy chain domains VH, CH1, CH2, and CH3, and light chain domains VL and CL. The antibody-related molecule may be a monomeric protein, or may be a multimeric protein, so long as it contains the above-mentioned molecular species. When the antibody-related molecule is a multimeric protein, it may be a homo-multimer consisting of a single kind of subunit, or may be a hetero-multimer consisting of two or more kinds of subunits. Specific examples of the antibody-related molecules include, for example, complete antibody, Fab, F(ab'), F(ab')₂, Fc, dimer consisting of a heavy chain (H chain) and a light chain (L chain), Fc-fusion protein, heavy chain (H chain), light chain (L chain), light chain Fv (scFv), sc(Fv)₂, disulfide-bonded Fv (sdFv), diabody, and VHH fragment (Nanobody (registered trademark)). More specific examples of the antibody-related molecules include, for example, Trastuzumab, Adalimumab, and Nivolumab.

The receptor proteins are not particularly limited. A receptor protein may be, for example, a receptor protein for any of physiologically active proteins and other physiologically active substances. Examples of the other physiologically active substances include, for example, neurotransmitters such as dopamine. Furthermore, a receptor protein may be an orphan receptor of which the corresponding ligand is not known.

The antigen proteins to be used as vaccines are not particularly limited, so long as they are proteins that can induce an immune response. An antigen protein can be appropriately selected depending on the intended object of the immune response.

In addition, examples of other proteins include Liver-type fatty acid-binding protein (LFABP), fluorescent protein, immunoglobulin-binding protein, albumin, and extracellular protein. Examples of the fluorescent protein include Green Fluorescent Protein (GFP). Examples of the immunoglobulin-binding protein include Protein A, Protein G, and Protein L. Examples of albumin include human serum albumin.

Examples of the extracellular protein include fibronectin, vitronectin, collagen, osteopontin, laminin, and partial sequences thereof. Laminin is a protein having a heterotrimeric structure consisting of an α chain, a β chain, and a γ chain. Examples of laminin include laminin of mammals. Examples of the mammals include primates such as human, monkey, and chimpanzee; rodents such as mouse, rat, hamster, and guinea pig; and other various mammals such as rabbit, horse, cattle, sheep, goat, pig, dog, and cat. Particular examples of the mammals include human. Examples of the subunit chains of laminin (i.e. α, β, and γ chains) include 5 kinds of α chains (α1 to α5), 3 kinds of β chains (β1 to β3), and 3 kinds of γ chains (γ1 to γ3). Laminin constitutes various isoforms depending on combinations of these subunits. Specific examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. Examples of the partial sequence of laminin include laminin E8, which is an E8 fragment of laminin. Laminin E8 is a protein having a heterotrimeric structure consisting of an E8 fragment of α chain (α chain E8), an E8 fragment of β chain (β chain E8), and an E8 fragment of γ chain (γ chain E8). The subunit chains of laminin E8 (i.e. α chain E8, β chain E8, and γ chain E8) are also collectively referred to as "E8 subunit chains". Examples of the E8 subunit chains includes E8 fragments of the laminin subunit chains exemplified above. Laminin E8 constitutes various isoforms depending on combinations of these E8 subunit chains. Specific examples of laminin E8 include, for example, laminin 111E8, laminin 121E8, laminin 211E8, laminin 221E8, laminin 332E8, laminin 421E8, laminin 411E8, laminin 511E8, and laminin 521E8.

A gene encoding the heterologous protein such as these proteins can be used as it is, or after being modified as required. A gene encoding the heterologous protein can be modified, for example, depending on a host to be used and/or for obtaining a desired activity. For example, a gene encoding the heterologous protein may be modified so that the amino acid sequence of the encoded heterologous protein include substitution, deletion, insertion, and/or addition of one or several amino acid residues. The above descriptions concerning variants of the LepB protein and the *lepB* gene can be applied *mutatis mutandis* to the heterologous protein to be produced by secretory production by the method of the present invention and the gene encoding it. A protein specified with the type of organism from which the protein is derived is not limited to proteins *per se* found in that organism, and shall also include proteins having any of the amino acid sequences of proteins found in that organism and variants thereof. That is, for example, the term "protein derived from human" is not limited to proteins *per se* found in human, and shall also include proteins having any of the amino acid sequences of proteins found in human and variants thereof. Furthermore, in the gene encoding the heterologous protein, any codon(s) may be replaced with respective equivalent codon(s) thereof. For example, the gene encoding the heterologous protein may be modified so as to have optimal codons according to the codon usage frequency of the host to be used.

The N-terminal region of the heterologous protein eventually obtained by the method of the present invention may be the same as that of the natural protein, or may not be the same as that of the natural protein, so long as the TorA signal peptide has been completely removed. For example, the N-terminal region of the eventually obtained heterologous protein may be that of the natural protein including addition or deletion of one or several amino acid residues. Although the number of the "one or several" amino acid residues may differ depending on the full length or structure of the objective heterologous protein, specifically, it is preferably 1 to 20, more preferably 1 to 10, still more preferably 1 to 5, particularly preferably 1 to 3.

Furthermore, the heterologous protein to be produced by secretory production may be a protein comprising a pro-structure moiety (proprotein). When the heterologous protein to be produced by secretory production is a proprotein, the heterologous protein to be eventually obtained may be the proprotein or may not be the proprotein. That is, the proprotein may be processed into the mature protein by cleavage of the pro-structure moiety. The cleavage can be attained with, for example, a protease. When a protease is used, generally, the proprotein is preferably cleaved at a position substantially the same as that of the natural protein, or more preferably at exactly the same position as that of the natural protein so that the same mature protein as the natural mature protein is obtained, in view of the activity of the eventually obtained protein. Therefore, generally, a specific protease that cleaves the proprotein at such a position that the same protein as the naturally occurring mature protein is generated is most preferred. However, the N-terminal region of the heterologous protein to be eventually obtained may not be the same as that of the natural protein as described above. For example, depending on type, purpose of use etc. of the heterologous protein to be produced, a protein having an N-terminus longer or shorter by one to several amino acid residues compared with the natural protein may have more appropriate activity. Proteases usable in the present invention include, for example, commercially available proteases such as Dispase (produced by Boehringer Mannheim) as well as those obtainable from culture broth of a microorganism such as culture broth of actinomycetes. Such proteases may be used in an un-purified state, or may be used after purification to an appropriate purity as required.

The method for introducing the genetic construct used for the present invention into the coryneform bacterium is not particularly limited. The term "introduction of the genetic construct used for the present invention" refers to making a host harbor the genetic construct. The term "introduction of the genetic construct used for the present invention" includes not only cases where the genetic construct that has been preliminarily constructed is collectively introduced into a host, but also includes cases where at least the heterologous protein gene is introduced into a host and the genetic construct is constructed in the host. In the bacterium of the present invention, the genetic construct used for the present invention may be present on a vector that autonomously replicates out of the chromosome such as a plasmid, or may be incorporated into the chromosome. The genetic construct used for the present invention can be introduced, for example, in the same manner as that for introduction of a gene in methods for increasing the expression of a gene described above. In addition, for constructing the bacterium of the present invention, introduction of the genetic structure used for the present invention, increase in the activity of the LepB protein, and other modifications can be performed in any order.

The genetic construct used for the present invention can be introduced into a host by using, for example, a vector comprising the genetic construct. For example, the genetic construct used for the present invention can be introduced into a host by ligating the genetic construct with a vector to construct an expression vector of the genetic construct, and transforming the host with the expression vector. Also, when the vector contains a promoter that functions in a coryneform bacterium, an expression vector of the genetic construct used for the present invention can be constructed by ligating the nucleic acid sequence encoding the fusion protein of the present invention downstream from the promoter. The vector is not particularly limited so long as a vector autonomously replicable in a coryneform bacterium is chosen. The vector usable in a coryneform bacterium is as described above.

Furthermore, the genetic construct used for the present invention can be introduced into the chromosome of a host by using, for example, a transposon such as an artificial transposon. When a transposon is used, the genetic construct used for the present invention is introduced into the chromosome by homologous recombination or translocation ability of the transposon itself. Furthermore, the genetic construct used for the present invention can also be introduced into the chromosome of a host by other introduction methods utilizing homologous recombination. Examples of the introduction methods utilizing homologous recombination include, for example, methods utilizing a linear DNA, a plasmid having a temperature sensitive replication origin, a plasmid capable of conjugative transfer, a suicide vector not having a replication origin that functions in a host, and so forth. In addition, at least the heterologous protein gene may be introduced into the chromosome so that the genetic construct used for the present invention is constituted on the chromosome. In this case, a part or all of the constituents contained in the genetic construct, other than the heterologous protein gene, may be inherently present on the chromosome of the host. Specifically, for example, by using a promoter sequence inherently present on the chromosome of the host as it is, and replacing only the gene ligated downstream from the promoter sequence with a nucleic acid sequence encoding the TorA signal peptide and an objective heterologous protein gene, the genetic construct used for the present invention can be constituted on the chromosome, and the bacterium of the present invention can be thereby constructed. A part of the genetic construct used for the present invention, such as the heterologous protein gene, can be introduced into the chromosome in the same manner as that for introduction of the genetic construct used for the present invention into the chromosome.

The genetic construct used for the present invention or a constituent thereof, such as promoter sequence, nucleic acid sequence encoding the TorA signal peptide, or nucleic acid sequence encoding a heterologous protein, can be obtained by, for example, cloning. Specifically, for example, the genetic construct used for the present invention can be obtained by obtaining an objective heterologous protein gene by cloning from an organism having the objective heterologous protein, and then subjecting the gene to modification such as introduction of the nucleic acid sequence encoding the TorA signal peptide and introduction of the promoter sequence. Furthermore, the genetic construct used for the present invention or a constituent thereof can also be obtained by chemical synthesis (Gene, 60(1), 115-127 (1987)). The obtained genetic construct used for the present invention or constituent thereof can be used as it is, or after being modified as required.

Furthermore, when two or more kinds of proteins are expressed, it is sufficient that the genetic constructs for secretory expression of the proteins are harbored by the bacterium of the present invention so that secretory expression of the objective heterologous proteins can be attained. Specifically, for example, all the genetic constructs for secretory expression of the proteins may be harbored on a single expression vector, or harbored on the chromosome. Alternatively, the genetic constructs for secretory expression of the proteins may be separately harbored on a plurality of expression vectors, or may be separately harbored on one or more expression vectors and the chromosome. The "case where two or more kinds of proteins are expressed" refers to, for example, a case where two or more kinds of heterologous proteins are produced by secretory production, or a case where a hetero-multimeric protein is produced by secretory production.

The method for introducing the genetic construct used for the present invention into the coryneform bacterium is not particularly limited, and a generally used method, for example, the protoplast method (Gene, 39, 281-286 (1985)), the electroporation method (Bio/Technology, 7, 1067-1070 (1989)), the electric pulse method (Japanese Patent Laid-open (Kokai) No. 2-207791), and so forth can be used.

### <2> Method for producing heterologous protein

By culturing the bacterium of the present invention obtained as described above to express a heterologous protein, a large amount of the heterologous protein secreted out of the cells is obtained.

The bacterium of the present invention can be cultured according to a usually used method and conditions. For example, the bacterium of the present invention can be cultured in a usual medium containing a carbon source, a nitrogen source, and inorganic ions. In order to obtain still higher proliferation, organic micronutrients such as vitamins and amino acids can also be added as required.

As the carbon source, carbohydrates such as glucose and sucrose, organic acids such as acetic acid, alcohols, and others can be used. As the nitrogen source, ammonia gas, aqueous ammonia, ammonium salts, and others can be used. As the inorganic ions, calcium ions, magnesium ions, phosphate ions, potassium ions, iron ions, and so forth are appropriately used as required. The culture is performed within appropriate ranges of pH 5.0 to 8.5 and 15 to 37°C under aerobic conditions for 1 to 7 days. Furthermore, the culture conditions for L-amino acid production by coryneform bacteria and other conditions described for the methods for secretory production of a protein using a signal peptide can be used (refer to WO01/23591 and WO2005/103278). Furthermore, when an inducible promoter is used for expression of the heterologous protein, culture may also be performed with adding a promoter-inducing agent to the medium. By culturing the bacterium of the present invention under such conditions, a large amount of the objective protein is produced in cells and efficiently secreted out of the cells. In addition, according to the method of the present invention, the produced heterologous protein is secreted out of the cells, and therefore a protein that is generally lethal if it is accumulated in a large amount in cells of microorganisms, such as transglutaminases, can also be continuously produced without lethal effect.

The protein secreted in the medium according to the method of the present invention can be separated and purified from the medium after the culture by a method well known to those skilled in the art. For example, after the cells are removed by centrifugation or the like, the protein can be separated and purified by a known appropriate method such as salting out, ethanol precipitation, ultrafiltration, gel filtration chromatography, ion exchange column chromatography, affinity chromatography, medium or high pressure liquid chromatography, reverse phase chromatography, and hydrophobic chromatography, or a combination of these. Furthermore, in a certain case, culture or culture supernatant may be used as it is. The protein secreted in the cell surface layer according to the method of the present invention can also be separated and purified in the same manner as that for the case where the protein is secreted in the medium, after solubilizing it by a method well known to those skilled in the art such as elevation of salt concentration and use of a surfactant. Furthermore, in a certain case, the protein secreted in the cell surface layer may be used as, for example, an immobilized enzyme, without solubilizing it.

Secretory production of the objective heterologous protein can be confirmed by performing SDS-PAGE for the culture supernatant and/or a fraction containing the cell surface layer as a sample, and confirming the molecular weight of the separated protein band. Furthermore, secretory production of the objective heterologous protein can also be confirmed by performing Western blotting using antibodies for the culture supernatant and/or a fraction containing the cell surface layer as a sample (Molecular Cloning, Cold spring Harbor Laboratory Press, Cold Spring Harbor (USA), 2001). Furthermore, secretory production of the objective heterologous protein can also be confirmed by detecting an N-terminal amino acid sequence of the objective protein using a protein sequencer. Furthermore, secretory production of the objective heterologous protein can also be confirmed by determining the mass of the objective protein using a mass spectrometer. Furthermore, when the objective heterologous protein is an enzyme or a protein having a certain measurable physiological activity, secretory production of the objective heterologous protein can be confirmed by measuring enzymatic activity or the physiological activity of the objective protein in the culture supernatant and/or a fraction containing the cell surface layer as a sample.

### Examples

The present invention will be further specifically explained with reference to the following non-limiting examples.

### Example 1: Construction of amplification vector of signal peptidase gene (lepB) derived from C. glutamicum ATCC 13869

The genome sequence of the *C. glutamicum* ATCC 13869 strain and the nucleotide sequence of the *lepB* gene encoding Type I signal peptidase (LepB) have already been determined (GenBank Accession No. AP017557, NCBI locus tag CGBL_0119390). The nucleotide sequence of the *lepB* gene derived from the *C. glutamicum* ATCC 13869 strain is shown as SEQ ID NO: 1, and the amino acid sequence of the LepB protein is shown as SEQ ID NO: 2.

PCR was carried out by using genomic DNA of the *C. glutamicum* ATCC 13869 strain prepared with PurElute™ Genomic DNA Kit (EdgeBio) as the template, in combination with primers of SEQ ID NOS: 3 and 4, to amplify a region containing a coding region of the *lepB* gene and a 5'-side upstream region thereof of about 0.2 kbp, to thereby obtain a DNA fragment of about 1.0 kbp. The primer of SEQ ID NO: 3 has been designed to contain a recognition sequence of restriction enzyme *Kpn*I, and the primer of SEQ ID NO: 4 has been designed to contain recognition sequences of restriction enzymes *Apa*I and *Xba*I. PCR was carried out with PrimeSTAR(R) HS DNA Polymerase (Takara Bio), and the reaction conditions were according to the protocol recommended by the manufacturer.

This PCR fragment was digested with restriction enzymes *Kpn*I and *Xba*I*,* and inserted at *Kpn*I*-Xba*I site of pPK5 vector disclosed in WO2016/171224 by ligation reaction, to obtain pPK51epB, which is an amplification vector of *lepB* gene. Similarly, this PCR fragment was digested with restriction enzymes *Kpn*I and *Apa*I, and inserted at *Kpn*I*-Apa*I site of pPK6 vector disclosed in WO2016/171224 by ligation reaction, to obtain pPK61epB, which is an amplification vector of *tatABC* secretion system genes and *lepB* gene. The ligation reaction was carried out with DNA Ligation Kit <Mighty Mix> (Takara Bio), and the reaction conditions were according to the protocol recommended by the manufacturer. The pPK5 vector is a vector obtained by modifying NaeI restriction enzyme site in pPK4 vector disclosed in Japanese Patent Laid-open (Kokai) No. 9-322774, and the pPK6 is an amplification vector of TatABC secretion system obtained by inserting *tatABC* genes into the pPK5 vector (Fig. 1, panels A and C). As a result of nucleotide sequencing of the inserted fragments, it was confirmed that the *lepB* gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

When subcloning of an expression cassette of an objective protein is carried out using pPK51epB or pPK61epB as an expression vector, the insertion order of the *lepB* gene and the expression cassette of the objective protein can be controlled by using *Kpn*I site or *Apa*I*-Xba*I site (Fig. 1, panels B and D).

### Example 2: Evaluation of effect of lepB gene-amplification on secretory expression of transglutaminase comprising a pro-structure moiety (PTG) using TorA signal sequence

### (1) Secretory expression of PTG using TorA signal peptide under existing (non-lepB gene-amplified) condition

The YDK010::phoS(W302C)/pPK6_T_PTG strain disclosed in WO2016/171224 was cultured on MMTG liquid medium (120 g of glucose, 3 g of MgSO₄·7H₂O, 30 g of (NH₄)₂SO₄, 1.5 g of KH₂PO₄, 0.03 g of FeSO₄·7H₂O, 0.03 g of MnSO₄·5H₂O, 0.45 mg of thiamine hydrochloride, 0.45 mg of biotin, 0.15 g of DL-methionine, 0.2 g (as total nitrogen) of soybean hydrolysate solution obtained with HCl, and 50 g of CaCO₃, filled up with water to 1 L, and adjusted to pH7.0) containing 25 mg/L of kanamycin at 30°C for 72 hr. Incidentally, pPK6_T_PTG is a secretory expression vector of transglutaminase comprising a pro-structure moiety (PTG) constructed from the amplification vector of *tatABC* genes pPK6, and also is a plasmid containing the promoter *of cspB* gene of the C. *glutamicum* ATCC13869 strain, DNA encoding the TorA signal peptide *of E. coli* expressively linked downstream from the promoter, and PTG gene of *Streptomyces mobaraense* linked thereto so as to be expressed as a fusion protein with the signal peptide (WO2016/171224). The YDK010::phoS(W302C) strain is a strain obtained from the YDK010 strain (WO2002/081694), which is a cell surface layer protein CspB-deficient strain of *C. glutamicum* AJ12036 (FERM BP-734), by introducing PhoS(W302C) mutation into phoS gene on the chromosome thereof (WO2016/171224). After completion of the culture, 1.0 µL of the culture supernatant obtained by centrifuging the culture broth was subjected to reduced SDS-PAGE using MULTIGEL(R) II mini 15/20 (Cosmo Bio), and then staining was carried out with SYPRO(R) Orange (Thermo Fisher Scientific). As a result, two bands were mainly detected (Fig. 2, panel A, lanes 3-5). As a result of analysis of the N-terminal amino acid sequence of each band, an amino acid sequence indicating that C-terminal side 15 residues of the TorA signal sequence remains at the N-terminus of PTG (GMLGPSLLTP; SEQ ID NO: 47) was observed for the high molecular weight-side band, whereas the N-terminal amino acid sequence of PTG (DNGAGEETKS; SEQ ID NO: 48) was observed for the low molecular weight-side band (Fig. 2, panel B). That is, it was revealed that a mis-cleaved form of C-terminal side 15 residues of the TorA signal peptide significantly remains in secretory production of PTG using the TorA signal peptide.

### (2) Construction of secretory expression vector of PTG using pPK61epB vector

PCR was carried out by using the PTG secretory expression vector pPK6_T_PTG disclosed in WO2016/171224 as the template, in combination with primers of SEQ ID NOS: 5 and 6 or in combination with primers of SEQ ID NOS: 7 and 8, to amplify a DNA fragment of about 1.9 kbp containing an expression cassette of cspB promoter-TorA signal peptide-PTG. The primers of SEQ ID NOS: 5 and 6 have been designed to contain a recognition sequence of restriction enzyme *Kpn*I, and the primers of SEQ ID NOS: 7 and 8 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer.

These DNA fragments were inserted at *Kpn*I or *Apa*I site of pPK61epB vector constructed in Example 1 by infusion reaction, to obtain pPK61epB(K)_T PTG and pPK61epB(A)_T PTG, which are co-expression plasmids of *tatABC* secretion system genes, *lepB* gene, and PTG gene fused with the TorA signal sequence (Fig. 1, panel D). The letters (K) and (A) in the plasmid names represent the restriction enzyme sites of pPK61epB vector inserted with the expression cassette of PTG (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (3) Secretory expression of PTG using TorA signal peptide under lepB gene-amplified condition

The YDK010::phoS(W302C) strain disclosed in WO2016/171224 was transformed with the PTG secretory expression plasmids pPK61epB(K)_T_PTG and pPK61epB(A)_T_PTG constructed in Example 2(2), to thereby obtain strains YDK010::phoS(W302C)/pPK61epB(K)_T_PTG and YDK010::phoS(W302C)/pPK61epB(A)_T_PTG. The obtained transformants were each cultured on MMTG liquid medium containing 25 mg/L of kanamycin at 30°C for 72 hr. After completion of the culture, 1.0 µL of the culture supernatant obtained by centrifuging the culture broth was subjected to reduced SDS-PAGE using MULTIGEL(R) II mini 15/20 (Cosmo Bio), and then staining was carried out with SYPRO(R) Orange (Thermo Fisher Scientific). As a result, two bands were mainly detected in the strain introduced with pPK6_T_PTG, in which *lepB* gene was not amplified, whereas the high molecular weight-side band was decreased so as to be scarcely detected and the low molecular weight-side band was increased in both the strains introduced with pPK61epB(K)_T_PTG and pPK61epB(A)_T_PTG, in which *lepB* gene was amplified (Fig. 2, panel A, lanes 6-11). As a result of analysis of the N-terminal amino acid sequence, the N-terminal amino acid sequence of PTG (DNGAGEETKS; SEQ ID NO: 48) was observed for the low molecular weight-side band. That is, it was revealed that although a mis-cleaved form of C-terminal side 15 residues of the TorA signal peptide significantly remains in secretory production of PTG using the TorA signal peptide under non*-lepB* gene-amplified condition, amplification of *lepB* gene can promote processing of the TorA signal peptide, to thereby significantly improve production efficiency of PTG having the correct N-terminal amino acid sequence.

### Example 3: Evaluation of effect of lepB gene-amplification on secretory expression of protein glutaminase comprising a pro-structure moiety (PPG) using TorA signal sequence

### (1) Secretory expression of PPG using TorA signal peptide under existing (non-lepB gene-amplified) condition

The YDK010::phoS(W302C)/pPK6_T_PPG strain disclosed in WO2016/171224 was cultured on MMTG liquid medium containing 25 mg/L of kanamycin at 30°C for 72 hr. Incidentally, pPK6_T_PPG is a secretory expression vector of protein glutaminase comprising a pro-structure moiety (PPG) constructed from the amplification vector of *tatABC* genes pPK6, and also is a plasmid containing the promoter of *cspB* gene of the *C. glutamicum* ATCC13869 strain, DNA encoding the TorA signal peptide of *E. coli* expressively linked downstream from the promoter, and PPG gene of *Chryseobacterium proteolyticum* linked thereto so as to be expressed as a fusion protein with the signal peptide (WO2016/171224). After completion of the culture, 2.5 µL of the culture supernatant obtained by centrifuging the culture broth was subjected to reduced SDS-PAGE using MULTIGEL(R) II mini 15/20 (Cosmo Bio), and then staining was carried out with SYPRO(R) Orange (Thermo Fisher Scientific). As a result, two bands were mainly detected (Fig. 3, panel A, lanes 3-5). As a result of analysis of the N-terminal amino acid sequence of each band, an amino acid sequence indicating that C-terminal side 15 residues of the TorA signal sequence remains at the N-terminus of PPG (GMLGPSLLTP; SEQ ID NO: 47) was observed for the high molecular weight-side band, whereas the N-terminal amino acid sequence of PPG (DSNGNQEING; SEQ ID NO: 49) was observed for the low molecular weight-side band (Fig. 3, panel B). That is, it was revealed that a mis-cleaved form of C-terminal side 15 residues of the TorA signal peptide significantly remains in secretory production of PPG using the TorA signal peptide.

### (2) Construction of secretory expression vector of PPG using pPK61epB vector

PCR was carried out by using the PPG secretory expression vector pPK6_T_PPG disclosed in WO2016/171224 as the template, in combination with primers of SEQ ID NOS: 5 and 9 or in combination with primers of SEQ ID NOS: 7 and 10, to amplify a DNA fragment of about 1.6 kbp containing an expression cassette of cspB promoter-TorA signal sequence-PPG. The primers of SEQ ID NOS: 5 and 9 have been designed to contain a recognition sequence of restriction enzyme *Kpn*I, and the primers of SEQ ID NOS: 7 and 10 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer.

These DNA fragments were inserted at *Kpn*I or *Apa*I site of pPK61epB vector constructed in Example 1 by infusion reaction, to obtain pPK6lepB(K)_T_PPG and pPK61epB(A)_T_PPG, which are co-expression plasmids of *tatABC* secretion system genes, *lepB* gene, and PPG gene fused with the TorA signal sequence (Fig. 1, panel D). The letters (K) and (A) in the plasmid names represent the restriction enzyme sites of pPK61epB vector inserted with the expression cassette of PPG (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (3) Secretory expression of PPG using TorA signal peptide under lepB gene-amplified condition

The YDK010::phoS(W302C) strain disclosed in WO2016/171224 was transformed with the PPG secretory expression plasmids pPK6lepB(K)_T_PPG and pPK6lepB(A)_T_PPG constructed in Example 3(2), to thereby obtain strains YDK010::phoS(W302C)/pPK61epB(K)_T_ PPG and YDK010::phoS(W302C)/pPK6lepB(A)_T_PPG. The obtained transformants were each cultured on MMTG liquid medium containing 25 mg/L of kanamycin at 30°C for 72 hr. After completion of the culture, 2.5 µL of the culture supernatant obtained by centrifuging the culture broth was subjected to reduced SDS-PAGE using MULTIGEL(R) II mini 15/20 (Cosmo Bio), and then staining was carried out with SYPRO(R) Orange (Thermo Fisher Scientific). As a result, two bands were mainly detected in the strain introduced with pPK6_T_PPG, in which *lepB* gene was not amplified, whereas the high molecular weight-side band was decreased so as to be scarcely detected and the low molecular weight-side band was increased in both the strains introduced with pPK6lepB(K)_T_PPG and pPK6lepB(A)_T_PPG, in which *lepB* gene was amplified (Fig. 3, panel A, lanes 6-11). As a result of analysis of the N-terminal amino acid sequence, the N-terminal amino acid sequence of PPG (DSNGNQEING; SEQ ID NO: 49) was observed for the low molecular weight-side band. That is, it was revealed that although a mis-cleaved form of C-terminal side 15 residues of the TorA signal peptide significantly remains in secretory production of PPG using the TorA signal peptide under *non-lepB* gene-amplified condition, amplification of *lepB* gene can promote processing of the TorA signal peptide, to thereby significantly improve production efficiency of PPG having the correct N-terminal amino acid sequence.

### Example 4: Evaluation of effect of lepB gene-amplification on secretory expression of beta-lactamase (Bla) using TorA signal sequence

### (1) Construction of co-expression plasmid of tatABC genes encoding Tat secretion system and gene encoding Bla added with TorA signal sequence

The amino acid sequence of class A broad-spectrum beta-lactamase TEM-116 belonging to the TEM-type, which is one of beta-lactamases (hereinafter referred to as "Bla"), has already been determined (GenBank Accession No. WP_000027050). The amino acid sequence of 263 residues corresponding to positions 24-286 of this amino acid sequence, excluding the N-terminal signal sequence, is shown as SEQ ID NO: 11. Considering the codon usage frequency of *C. glutamicum,* a nucleotide sequence encoding Bla of SEQ ID NO: 11 was designed. The designed nucleotide sequence is shown as SEQ ID NO: 12.

Then, an expression cassette of a fusion protein of the TorA signal peptide and Bla, in which a DNA encoding 39 amino acid residues consisting of a signal peptide of TorA protein derived from *E. coli* (UniProt Accession number: P33225) and the DNA of SEQ ID NO: 12 were linked downstream of the promoter of *cspB* gene of the *C. glutamicum* ATCC 13869 strain, and *Kpn*I site and *Apa*I site were further added at the 5'-side and 3'-side termini, respectively, was totally synthesized. The synthesized DNA fragment was inserted at *Kpn*I*-Apa*I site of pPK6 vector disclosed in WO2016/171224, to construct pPK6_T_Bla, which is a secretory expression plasmid of Bla using the TorA signal sequence (Fig. 1, panel C). As a result of nucleotide sequencing of the inserted fragment, it was confirmed that the gene encoding the expression cassette of Bla gene as intended was constructed. Nucleotide sequencing was carried out with BigDye(R) Terminator v3.1 Cycle Sequencing Kit (Applied Biosystems) and 3130 Genetic Analyzer (Applied Biosystems).

### (2) Secretory expression of Bla using TorA signal peptide under non-lepB gene-amplified condition

The YDK010::phoS(W302C) strain disclosed in WO2016/171224 was transformed with the Bla secretory expression plasmid pPK6_T_Bla constructed in Example 4(1), to thereby obtain a strain YDK010::phoS(W302C)/pPK6_T_Bla. The obtained transformant was cultured on MMTG liquid medium containing 25 mg/L of kanamycin at 30°C for 72 hr. After completion of the culture, 5.0 µL of the culture supernatant obtained by centrifuging the culture broth was subjected to reduced SDS-PAGE using NuPAGE(R) 4-12% Bis-Tirs Gel (Thermo Fisher Scientific), and then staining was carried out with SYPRO(R) Ruby (Thermo Fisher Scientific). As a result, two bands were mainly detected (Fig. 4, panel A, lanes 3-5). As a result of analysis of the N-terminal amino acid sequence of each band, an amino acid sequence indicating that C-terminal side 15 residues of the TorA signal sequence remains at the N-terminus of Bla (GMLGPSLLTP; SEQ ID NO: 47) was observed for the high molecular weight-side band, whereas the N-terminal amino acid sequence of Bla (HPETLVKVKD; SEQ ID NO: 50) was observed for the low molecular weight-side band (Fig. 4, panel B). That is, it was revealed that a mis-cleaved form of C-terminal side 15 residues of the TorA signal peptide remains in secretory production of Bla using the TorA signal peptide.

### (3) Construction of secretory expression vector of Bla using pPK61epB vector

PCR was carried out by using the Bla secretory expression vector pPK6_T_Bla constructed in Example 4(1) as the template, in combination with primers of SEQ ID NOS: 5 and 13 or in combination with primers of SEQ ID NOS: 7 and 14, to amplify a DNA fragment of about 1.5 kbp containing an expression cassette of cspB promoter-TorA signal sequence-Bla. The primers of SEQ ID NOS: 5 and 13 have been designed to contain a recognition sequence of restriction enzyme *Kpn*I, and the primers of SEQ ID NOS: 7 and 14 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer.

These DNA fragments were inserted at *Kpn*I or *Apa*I site of pPK61epB vector constructed in Example 1 by infusion reaction, to obtain pPK61epB(K)_T_Bla and pPK6lepB(A)_T_Bla, which are co-expression plasmids of *tatABC* secretion system genes, *lepB* gene, and Bla gene fused with the TorA signal sequence (Fig. 1, panel D). The letters (K) and (A) in the plasmid names represent the restriction enzyme sites of pPK61epB vector inserted with the expression cassette of Bla (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (4) Secretory expression of Bla using TorA signal peptide under lepB gene-amplified condition

The YDK010::phoS(W302C) strain disclosed in WO2016/171224 was transformed with the Bla secretory expression plasmids pPK6lepB(K)_T_Bla and pPK6lepB(A)_T_Bla constructed in Example 4(3), to thereby obtain strains YDK010::phoS(W302C)/pPK6lepB(K)_T_Bla and YDK010::phoS(W302C)/pPK6lepB(A)_T_Bla. The obtained transformants were each cultured on MMTG liquid medium containing 25 mg/L of kanamycin at 30°C for 72 hr. After completion of the culture, 5.0 µL of the culture supernatant obtained by centrifuging the culture broth was subjected to reduced SDS-PAGE using NuPAGE(R) 4-12% Bis-Tirs Gel (Thermo Fisher Scientific), and then staining was carried out with SYPRO(R) Ruby (Thermo Fisher Scientific). As a result, two bands were mainly detected in the strain introduced with pPK6_T_Bla, in which *lepB* gene was not amplified, whereas the high molecular weight-side band was decreased so as to be scarcely detected and the low molecular weight-side band was increased in both the strains introduced with pPK6lepB(KLT_Bla and pPK6lepB(A)_T_Bla, in which *lepB* gene was amplified (Fig. 4, panel A, lanes 6-11). As a result of analysis of the N-terminal amino acid sequence, the N-terminal amino acid sequence of Bla (HPETLVKVKD; SEQ ID NO: 50) was observed for the low molecular weight-side band. That is, it was revealed that although a mis-cleaved form of C-terminal side 15 residues of the TorA signal peptide remains in secretory production of Bla using the TorA signal peptide under *non-lepB* gene-amplified condition, amplification of *lepB* gene can promote processing of the TorA signal peptide, to thereby significantly improve production efficiency of Bla having the correct N-terminal amino acid sequence.

### Example 5: Evaluation of effect of lepB gene-amplification on secretory expression of thioredoxin (Trx) using TorA signal sequence

### (1) Construction of co-expression plasmid of tatABC genes encoding Tat secretion system and gene encoding Trx added with TorA signal sequence

The amino acid sequence of TrxA protein *of E. coli,* which is one of thioredoxins (hereinafter referred to as "Trx"), has already been determined (GenBank Accession No. WP_001323277). The amino acid sequence of 109 residues of a modified Trx, which is obtained by removing N-terminal 19 residues from this amino acid sequence and then adding one residue of Ala at the N-terminus, is shown as SEQ ID NO: 15. Considering the codon usage frequency of *C. glutamicum,* a nucleotide sequence encoding Trx of SEQ ID NO: 15 was designed. The designed nucleotide sequence is shown as SEQ ID NO: 16.

Then, an expression cassette of a fusion protein of the TorA signal peptide and Trx, in which a DNA encoding 39 amino acid residues consisting of a signal peptide of TorA protein derived from *E. coli* (UniProt Accession number: P33225) and the DNA of SEQ ID NO: 16 were linked downstream of the promoter *of cspB* gene of the *C. glutamicum* ATCC 13869 strain, and *Kpn*I site and *Apa*I site were further added at the 5'-side and 3'-side termini, respectively, was totally synthesized. The synthesized DNA fragment was inserted at *Kpn*I*-Apa*I site of pPK6 vector disclosed in WO2016/171224, to construct pPK6_T_Trx, which is a secretory expression plasmid of Trx (Fig. 1, panel C). As a result of nucleotide sequencing of the inserted fragment, it was confirmed that the gene encoding the expression cassette of Trx gene as intended was constructed. Nucleotide sequencing was carried out with BigDye(R) Terminator v3.1 Cycle Sequencing Kit (Applied Biosystems) and 3130 Genetic Analyzer (Applied Biosystems).

### (2) Secretory expression of Trx using TorA signal peptide under non-lepB gene-amplified condition

The YDK010::phoS(W302C) strain disclosed in WO2016/171224 was transformed with the Trx secretory expression plasmid pPK6_T_Trx constructed in Example 5(1), to thereby obtain a strain YDK010::phoS(W302C)/pPK6_T_Trx. The obtained transformant was cultured on MMTG liquid medium containing 25 mg/L of kanamycin at 30°C for 72 hr. After completion of the culture, 2.5 µL of the culture supernatant obtained by centrifuging the culture broth was subjected to reduced SDS-PAGE using NuPAGE(R) 12% Bis-Tirs Gel (Thermo Fisher Scientific), and then staining was carried out with SYPRO(R) Ruby (Thermo Fisher Scientific). As a result, two bands were mainly detected (Fig. 5, panel A, lanes 3-5). As a result of analysis of the N-terminal amino acid sequence of each band, an amino acid sequence indicating that C-terminal side 15 residues of the TorA signal sequence remains at the N-terminus of Trx (GMLGPSLLTP; SEQ ID NO: 47) was observed for the high molecular weight-side band, whereas the N-terminal amino acid sequence of Trx (ASDKIIHLTD; SEQ ID NO: 51) was observed for the low molecular weight-side band (Fig. 5, panel B). That is, it was revealed that a mis-cleaved form of C-terminal side 15 residues of the TorA signal peptide remains in secretory production of Trx using the TorA signal peptide.

### (3) Construction of secretory expression vector of Trx using pPK61epB vector

PCR was carried out by using the Trx secretory expression vector pPK6_T_Trx constructed in Example 5(1) as the template, in combination with primers of SEQ ID NOS: 5 and 17 or in combination with primers of SEQ ID NOS: 7 and 18, to amplify a DNA fragment of about 1.1 kbp containing an expression cassette of cspB promoter-TorA signal sequence-Trx. The primers of SEQ ID NOS: 5 and 17 have been designed to contain a recognition sequence of restriction enzyme *Kpn*I, and the primers of SEQ ID NOS: 7 and 18 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer.

These DNA fragments were inserted at *Kpn*I or *Apa*I site of pPK61epB vector constructed in Example 1 by infusion reaction, to obtain pPK61epB(K)_T_Trx and pPK61epB(A)_T _Trx, which are co-expression plasmids of *tatABC* secretion system genes, *lepB* gene, and Trx gene fused with the TorA signal sequence (Fig. 1, panel D). The letters (K) and (A) in the plasmid names represent the restriction enzyme sites of pPK61epB vector inserted with the expression cassette of Trx (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (4) Secretory expression of Trx using TorA signal peptide under lepB gene-amplified condition

The YDK010::phoS(W302C) strain disclosed in WO2016/171224 was transformed with the Trx secretory expression plasmids pPK6lepB(K)_T_Trx and pPK6lepB(A)_T_Trx constructed in Example 5(3), to thereby obtain strains YDK010::phoS(W302C)/pPK61epB(K)_T_ Trx and YDK010::phoS(W302C)/pPK6lepB(A)_T_Trx. The obtained transformants were each cultured on MMTG liquid medium containing 25 mg/L of kanamycin at 30°C for 72 hr. After completion of the culture, 2.5 µL of the culture supernatant obtained by centrifuging the culture broth was subjected to reduced SDS-PAGE using NuPAGE(R) 12% Bis-Tirs Gel (Thermo Fisher Scientific), and then staining was carried out with SYPRO(R) Ruby (Thermo Fisher Scientific). As a result, two bands were mainly detected in the strain introduced with pPK6_T_Trx, in which *lepB* gene was not amplified, whereas the high molecular weight-side band was decreased so as to be scarcely detected and the low molecular weight-side band was increased in both the strains introduced with pPK6lepB(K)_T_Bla and pPK6lepB(A)_T_Bla, in which *lepB* gene was amplified (Fig. 5, panel A, lanes 6-11). As a result of analysis of the N-terminal amino acid sequence, the N-terminal amino acid sequence of Trx (ASDKIIHLTD; SEQ ID NO: 51) was observed for the low molecular weight-side band. That is, it was revealed that although a mis-cleaved form of C-terminal side 15 residues of the TorA signal peptide remains in secretory production of Trx using the TorA signal peptide under non*-lepB* gene-amplified condition, amplification of *lepB* gene can promote processing of the TorA signal peptide, to thereby significantly improve production efficiency of Trx having the correct N-terminal amino acid sequence.

### Example 6: Evaluation of effect of lepB gene-amplification on secretory expression of Liver-type fatty acid-binding protein (LFABP) using TorA signal sequence

### (1) Construction of co-expression plasmid of tatABC genes encoding Tat secretion system and gene encoding LFABP added with TorA signal sequence

The amino acid sequence of Liver-type fatty acid-binding protein (hereinafter referred to as "LFABP") of human has already been determined (GenBank Accession No. NP_001434). This amino acid sequence of 127 residues is shown as SEQ ID NO: 19. Considering the codon usage frequency of *C. glutamicum,* a nucleotide sequence encoding LFABP of SEQ ID NO: 19 was designed. The designed nucleotide sequence is shown as SEQ ID NO: 20.

Then, an expression cassette of a fusion protein of the TorA signal peptide and LFABP, in which a DNA encoding 39 amino acid residues consisting of a signal peptide of TorA protein derived from *E. coli* (UniProt Accession number: P33225) and the DNA of SEQ ID NO: 20 were linked downstream of the promoter of *cspB* gene of the *C. glutamicum* ATCC 13869 strain, and *Kpn*I site and *Apa*I site were further added at the 5'-side and 3'-side termini, respectively, was totally synthesized. The synthesized DNA fragment was inserted at *Kpn*I*-Apa*I site of pPK6 vector disclosed in WO2016/171224, to construct pPK6_T_LFABP, which is a secretory expression plasmid of LFABP using the TorA signal sequence (Fig. 1, panel C). As a result of nucleotide sequencing of the inserted fragment, it was confirmed that the gene encoding the expression cassette of LFABP gene as intended was constructed. Nucleotide sequencing was carried out with BigDye(R) Terminator v3.1 Cycle Sequencing Kit (Applied Biosystems) and 3130 Genetic Analyzer (Applied Biosystems).

### (2) Secretory expression of LFABP using TorA signal peptide under non-lepB gene-amplified condition

The YDK010::phoS(W302C) strain disclosed in WO2016/171224 was transformed with the LFABP secretory expression plasmid pPK6_T_LFABP constructed in Example 6(1), to thereby obtain a strain YDK010::phoS(W302C)/pPK6_T_LFABP. The obtained transformant was cultured on MMTG liquid medium containing 25 mg/L of kanamycin at 30°C for 72 hr. After completion of the culture, 5.0 µL of the culture supernatant obtained by centrifuging the culture broth was subjected to reduced SDS-PAGE using NuPAGE(R) 4-12% Bis-Tirs Gel (Thermo Fisher Scientific), and then staining was carried out with SYPRO(R) Ruby (Life Technologies). As a result, two bands were mainly detected (Fig. 6, panel A, lanes 3-5). Incidentally, since the high molecular weight-side band was detected also in a culture supernatant of the YDK010::phoS(W302C)/pPK6 strain used as a negative control (Fig. 6, panel A, lane 2), a protein of a host, *C. glutamicum,* was presumed to be contained therein. As a result of analysis of the N-terminal amino acid sequence of each band, an amino acid sequence indicating that C-terminal side 15 residues of the TorA signal sequence remains at the N-terminus of LFABP (GMLGPSLLTP; SEQ ID NO: 47) and the N-terminal sequence of a mature protein of resuscitation-promoting factor Rpf1 of *C. glutamicum* (GenBank Accession No. AP017557, NCBI locus tag CGBL_0109030) (APDSDWDRLA; SEQ ID NO: 52) were detected in the high molecular weight-side band, and hence, the high molecular weight-side band was confirmed to be a mixture band consisting mainly of these two kinds of proteins (Fig. 6, panel B). Incidentally, no amino acid sequence indicating that C-terminal-side partial sequence of the Rpf1 signal sequence remains was detected, and hence, it was confirmed that the full-length signal peptide was correctly cleaved as for Rpf1. By contrast, the N-terminal amino acid sequence of LFABP (MSFSGKYQLQ; SEQ ID NO: 53) was observed for the low molecular weight-side band (Fig. 6, panel B). That is, it was revealed that a mis-cleaved form of C-terminal side 15 residues of the TorA signal peptide remains in secretory production of LFABP using the TorA signal peptide.

### (3) Construction of secretory expression vector of LFABP using pPK61epB vector

PCR was carried out by using the LFABP secretory expression vector pPK6_T_LFABP constructed in Example 6(1) as the template, in combination with primers of SEQ ID NOS: 5 and 21 or in combination with primers of SEQ ID NOS: 7 and 22, to amplify a DNA fragment of about 1.1 kbp containing an expression cassette of cspB promoter-TorA signal sequence-LFABP. The primers of SEQ ID NOS: 5 and 21 have been designed to contain a recognition sequence of restriction enzyme *Kpn*I, and the primers of SEQ ID NOS: 7 and 22 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer.

These DNA fragments were inserted at *Kpn*I or *Apa*I site of pPK61epB vector constructed in Example 1 by infusion reaction, to obtain pPK6lepB(K)_T_LFABP and pPK61epB(A)_T_FABP, which are co-expression plasmids of *tatABC* secretion system genes, *lepB* gene, and LFABP gene fused with the TorA signal sequence (Fig. 1, panel D). The letters (K) and (A) in the plasmid names represent the restriction enzyme sites of pPK61epB vector inserted with the expression cassette of LFABP (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (4) Secretory expression of LFABP using TorA signal peptide under lepB gene-amplified condition

The YDK010::phoS(W302C) strain disclosed in WO2016/171224 was transformed with the LFABP secretory expression plasmids pPK6lepB(K)_T_LFABP and pPK6lepB(A)_T_LFABP constructed in Example 6(3), to thereby obtain strains YDK010::phoS(W302C)/pPK6lepB(K)_T_LFABP and
YDK010::phoS(W302C)/pPK6lepB(A)_T_LFABP. The obtained transformants were each cultured on MMTG liquid medium containing 25 mg/L of kanamycin at 30°C for 72 hr. After completion of the culture, 5.0 µL of the culture supernatant obtained by centrifuging the culture broth was subjected to reduced SDS-PAGE using NuPAGE(R) 4-12% Bis-Tirs Gel (Thermo Fisher Scientific), and then staining was carried out with SYPRO(R) Ruby (Life Technologies). As a result, two major bands were detected in approximately equal amounts in the strain introduced with pPK6_T_LFABP, in which *lepB* gene was not amplified, whereas the low molecular weight-side band was significantly increased in both the strains introduced with pPK6lepB(K)_T_FABP and pPK6lepB(A)_T_FABP, in which *lepB* gene was amplified (Fig. 6, panel A, lanes 6-11). As a result of analysis of the N-terminal amino acid sequence, the N-terminal amino acid sequence of LFABP (MSFSGKYQLQ; SEQ ID NO: 53) was observed for the low molecular weight-side band. That is, it was revealed that although a mis-cleaved form of C-terminal side 15 residues of the TorA signal peptide remains in secretory production of LFABP using the TorA signal peptide under non*-lepB* gene-amplified condition, amplification of *lepB* gene can promote processing of the TorA signal peptide, to thereby significantly improve production efficiency of LFABP having the correct N-terminal amino acid sequence.

From these results, it was revealed that a mis-cleaved form of C-terminal side 15 residues of the TorA signal peptide remains in secretory production of a heterologous protein using the TorA signal peptide in *C. glutamicum,* and amplification of *lepB* gene can promote processing of the TorA signal peptide, to thereby significantly improve production efficiency of the heterologous protein having the correct N-terminal amino acid sequence, regardless of the kind of heterologous protein.

### Example 7: Evaluation of effect of lepB gene-amplification on secretory expression of transglutaminase comprising a pro-structure moiety (PTG) using SlpA signal sequence

### (1) Construction of secretory expression vector of PTG using pPK5 vector

PCR was carried out by using the PTG secretory expression vector pPKSPTG1 disclosed in WO2002/081694 as the template, in combination with primers of SEQ ID NOS: 23 and 24, to amplify a DNA fragment of about 1.8 kbp containing an expression cassette of cspB promoter-SlpA signal sequence-PTG. The primers of SEQ ID NOS: 23 and 24 have been designed to contain a recognition sequence of restriction enzyme *Kpn*I. PCR was carried out with PrimeSTAR(R) HS DNA Polymerase (Takara Bio), and the reaction conditions were according to the protocol recommended by the manufacturer. This DNA fragment was inserted at *Kpn*I site of pPK5 vector disclosed in WO2016/171224 by infusion reaction, to obtain pPK5_S_PTG, which is a secretory expression vector of PTG gene using the SlpA signal sequence (Fig. 1, panel A). The infusion reaction was carried out with In-Fusion(R) HD Cloning Kit (Takara Bio), and the reaction conditions were according to the protocol recommended by the manufacturer. As a result of nucleotide sequencing of the inserted fragment, it was confirmed that the gene as expected was inserted into the vector. Nucleotide sequencing was carried out with BigDye(R) Terminator v3.1 Cycle Sequencing Kit (Applied Biosystems) and 3130 Genetic Analyzer (Applied Biosystems).

### (2) Construction of secretory expression vector of PTG using pPK51epB vector

Similarly to Example 7(1), PCR was carried out by using the PTG secretory expression vector pPKSPTG1 disclosed in WO2002/081694 as the template, in combination with primers of SEQ ID NOS: 23 and 25 or in combination with primers of SEQ ID NOS: 26 and 27, to amplify a DNA fragment of about 1.8 kbp containing an expression cassette of cspB promoter-SlpA signal sequence-PTG. The primers of SEQ ID NOS: 23 and 25 have been designed to contain a recognition sequence of restriction enzyme *Kpn*I, and the primers of SEQ ID NOS: 26 and 27 have been designed to contain recognition sequences of restriction enzymes *Apa*I. PCR was carried out with PrimeSTAR(R) HS DNA Polymerase (Takara Bio), and the reaction conditions were according to the protocol recommended by the manufacturer. These DNA fragments were inserted at *Kpn*I or *Apa*I site of pPK51epB vector constructed in Example 1 by infusion reaction, to obtain pPK5lepB(K)_S_PTG and pPK5lepB(A)_S_PTG, which are co-expression vectors of *lepB* gene and PTG secretion using the SlpA signal sequence (Fig. 1, panel B). The letters (K) and (A) in the plasmid names represent the restriction enzyme sites of pPK51epB vector inserted with the expression cassette of PTG (*Kpn*I site and *Apa*I site), respectively. The infusion reaction was carried out with In-Fusion(R) HD Cloning Kit (Takara Bio), and the reaction conditions were according to the protocol recommended by the manufacturer. As a result of nucleotide sequencing of the inserted fragments, it was confirmed that the gene as expected was inserted into each vector. Nucleotide sequencing was carried out with BigDye(R) Terminator v3.1 Cycle Sequencing Kit (Applied Biosystems) and 3130 Genetic Analyzer (Applied Biosystems).

### (3) Secretory expression of SlpA signal peptide-PTG under lepB gene-amplified condition and non lepB gene-amplified condition

The YDK010::phoS(W302C) strain disclosed in WO2016/171224 was transformed with the PTG secretory expression plasmids using the SlpA signal sequence constructed in Example 7(1) and (2), pPK5_S_PTG, pPK5lepB(K)_S_PTG, and pPK5lepB(A)_S_PTG, to thereby obtain strains YDK010::phoS(W302C)/pPK5_S_PTG, YDK010::phoS(W302C)/pPK5lepB(K)_S_PTG, and YDK010::phoS(W302C)/pPK5lepB(A)_S_PTG. The obtained transformants were each cultured on MMTG liquid medium containing 25 mg/L of kanamycin at 30°C for 72 hr. After completion of the culture, 5.0 µL of the culture supernatant obtained by centrifuging the culture broth was subjected to reduced SDS-PAGE using MULTIGEL(R) II mini 15/20 (Cosmo Bio), and then staining was carried out with SYPRO(R) Orange (Thermo Fisher Scientific). As a result, in the case of using the SlpA signal sequence, in contrast to the case of using the TorA signal sequence, a single band of PTG was detected in the strain introduced with pPK5_S_PTG, in which *lepB* gene was not amplified, and that is, a mis-cleaved form in which a C-terminal partial sequence of the SlpA signal peptide was not detected at all (Fig. 7, panel A, lane 7). Furthermore, the band pattern was not changed in the strains introduced with pPK5lepB(K)_S_PTG and pPK5lepB(A)_S_PTG, in which *lepB* gene was amplified, and rather the secretory expression amount of PTG was decreased in those strains (Fig. 7, panel A, lanes 8-9).

That is, it was revealed that amplification of *lepB* gene is not necessarily effective for all signal sequences in secretory production of a heterologous protein using *C. glutamicum.*

### Industrial Applicability

According to the present invention, heterologous proteins can be efficiently produced by secretory production.

### <Explanation of Sequence Listing>

SEQ ID NOS:
1: Nucleotide sequence of *lepB* gene of *C. glutamicum* ATCC 13869
2: Amino acid sequence of LepB protein of *C. glutamicum* ATCC 13869
3 to 10: Primers
11: Amino acid sequence of beta-lactamase TEM-116
12: Nucleotide sequence encoding beta-lactamase TEM-116
13 to 14: Primers
15: Amino acid sequence of modified TrxA protein of E. coli
16: Nucleotide sequence encoding modified TrxA protein of E. coli
17 to 18: Primers
19: Amino acid sequence of LFABP of human
20: Nucleotide sequence encoding LFABP of human
21 to 27: Primers
28: Nucleotide sequence of mutant *phoS* gene of *C. glutamicum* YDK010
29: Amino acid sequence of mutant PhoS protein of *C. glutamicum* YDK010
30: Amino acid sequence of PhoS protein of *C*. *glutamicum* ATCC 13032
31: Amino acid sequence of PhoS protein of *C. glutamicum* ATCC 14067
32: Amino acid sequence of PhoS protein of *C. callunae*
33: Amino acid sequence of PhoS protein of *C*. *crenatum*
34: Amino acid sequence of PhoS protein of *C*. *efficiens*
35: Nucleotide sequence of *phoR* gene of *C. glutamicum* ATCC 13032
36: Amino acid sequence of PhoR protein of *C. glutamicum* ATCC 13032
37: Nucleotide sequence of *cspB* gene of *C*. *glutamicum* ATCC 13869
38: Amino acid sequence of CspB protein of *C*. *glutamicum* ATCC 13869
39: Nucleotide sequence of *tatA* gene of *C*. *glutamicum* ATCC 13032
40: Amino acid sequence of TatA protein of *C*. *glutamicum* ATCC 13032
41: Nucleotide sequence of *tatB* gene of *C*. *glutamicum* ATCC 13032
42: Amino acid sequence of TatB protein of *C*. *glutamicum* ATCC 13032
43: Nucleotide sequence of *tatC* gene of *C*. *glutamicum* ATCC 13032
44: Amino acid sequence of TatC protein of *C*. *glutamicum* ATCC 13032
45: Amino acid sequence of twin-arginine motif
46: Amino acid sequence of TorA signal peptide
47 to 53: N-terminal amino acid sequences
54 to 59: N-terminal amino acid sequences of proteins having signal peptides

## Claims

1. A method for producing a heterologous protein comprising:
culturing a coryneform bacterium having a genetic construct for secretory expression of the heterologous protein; and
collecting the heterologous protein produced by secretory production,
wherein the coryneform bacterium has been modified so that the activity of a LepB protein is increased,
wherein the genetic construct comprises, in the direction from 5' to 3', a promoter sequence that functions in the coryneform bacterium, a nucleic acid sequence encoding a TorA signal peptide, and a nucleic acid sequence encoding the heterologous protein, and
wherein the heterologous protein is expressed as a fusion protein with the TorA signal peptide.

2. The method according to claim 1, wherein the LepB protein is a protein defined in (a), (b), or (c) mentioned below:
(a) a protein comprising the amino acid sequence of SEQ ID NO: 2;
(b) a protein comprising the amino acid sequence of SEQ ID NO: 2, but which includes substitution, deletion, insertion, and/or addition of 1 to 10 amino acid residues, wherein said protein has a signal peptidase activity for the TorA signal peptide;
(c) a protein comprising an amino acid sequence showing an identity of 90% or higher to the amino acid sequence of SEQ ID NO: 2, wherein said protein has a signal peptidase activity for the TorA signal peptide.

3. The method according to claim 1 or 2, wherein the activity of the LepB protein is increased by increasing the expression of a *lepB* gene.

4. The method according to claim 3, wherein the expression of the *lepB* gene is increased by increasing the copy number of *lepB* gene and/or modifying an expression control sequence of the *lepB* gene.

5. The method according to any one of claims 1 to 4, wherein the TorA signal peptide is a peptide defined in (a), (b), or (c) mentioned below:
(a) a peptide comprising the amino acid sequence of SEQ ID NO: 46;
(b) a peptide comprising the amino acid sequence of SEQ ID NO: 46, but which includes substitution, deletion, insertion, and/or addition of 1 to 3 amino acid residues, wherein said protein has a function as a Tat-dependent signal peptide;
(c) a peptide comprising an amino acid sequence showing an identity of 90% or higher to the amino acid sequence of SEQ ID NO: 46, wherein said protein has a function as a Tat-dependent signal peptide.

6. The method according to any one of claims 1 to 5, wherein the TorA signal peptide consists of the amino acid sequence of SEQ ID NO: 46.

7. The method according to any one of claims 1 to 6, wherein the heterologous protein produced by secretory production is a heterologous protein from which the TorA signal peptide has been completely removed.

8. The method according to any one of claims 1 to 7, wherein the coryneform bacterium has been further modified so as to harbor a *phoS* gene encoding a mutant PhoS protein.

9. The method according to claim 8, wherein the mutation is a mutation of replacing an amino acid residue corresponding to the tryptophan residue at position 302 in SEQ ID NO: 29 with an amino acid residue other than aromatic amino acid and histidine residues in a wild-type PhoS protein.

10. The method according to claim 9, wherein the amino acid residue other than aromatic amino acid and histidine residues is a lysine residue, alanine residue, valine residue, serine residue, cysteine residue, methionine residue, aspartic acid residue, or asparagine residue.

11. The method according to claim 9 or 10, wherein the wild-type PhoS protein is a protein defined in (a), (b), or (c) mentioned below:
(a) a protein comprising any of the amino acid sequences of SEQ ID NOS: 29 to 34;
(b) a protein comprising any of the amino acid sequences of SEQ ID NOS: 29 to 34, but which includes substitution, deletion, insertion, and/or addition of 1 to 10 amino acid residues, wherein said protein has a function as a sensor kinase of a PhoRS system;
(c) a protein comprising an amino acid sequence showing an identity of 90% or higher to any of the amino acid sequences of SEQ ID NOS: 29 to 34, wherein said protein has a function as a sensor kinase of a PhoRS system.

12. The method according to any one of claims 1 to 11, wherein the coryneform bacterium has been further modified so that the expression of one or more genes selected from genes encoding a Tat secretion system is increased as compared with a non-modified strain.

13. The method according to claim 12, wherein the genes encoding a Tat secretion system consists of a *tatA* gene, *tatB* gene, *tatC* gene, and *tatE* gene.

14. The method according to any one of claims 1 to 13, wherein the coryneform bacterium is a bacterium belonging to the genus *Corynebacterium.*

15. The method according to claim 14, wherein the coryneform bacterium is *Corynebacterium glutamicum.*

16. The method according to claim 15, wherein the coryneform bacterium is a modified strain derived from *Corynebacterium glutamicum* AJ12036 (FERM BP-734) or a modified strain derived from *Corynebacterium glutamicum* ATCC 13869.

17. The method according to any one of claims 1 to 16, wherein the coryneform bacterium is a coryneform bacterium in which the number of molecules of a cell surface layer protein per cell is reduced as compared with a non-modified strain.


SEQUENCE LISTING

<110>  Ajinomoto Co., Inc.

<120>  METHOD FOR SECRETORY PRODUCTION OF PROTEIN

<130>  F118-19299

<150>  JP2018-200673
<151>  2018-10-25

<160>  59

<170>  PatentIn version 3.5

<210>  1
<211>  789
<212>  DNA
<213>  Corynebacterium glutamicum

<400>  1
gtgactgatt tttctagtgc ttcaaatgct gacgattcca cgcaggacgg tcgtcctggt      60
cgacgtgctg gaaagtctaa gaaggaatcg aagccaactc cgtggtacat cgaaattcca     120
gtggttgtgg ttctgaccct cgcgctgatt ttcgtgctcc agacgtttgt cggacgcatg     180
tacatgattc cgagtggttc gatggaacct actttgcacg gatgtgaggg ctgcacgggt     240
gaccgcatcc tggtggagaa ggtttcttac tacttcacgg atccagagcc gggcgatgtt     300
gtggtgttca agggtactga ttcctggaac gttggattca ctacgcagcg ttccgataat     360
tcggtgatcc gcggcctgca gaacctgggt tcttacgtgg gtcttgtcgc acctgatgaa     420
aatgacctgg tcaagcgcat tatcgccacc ggcggtcaga ctgtttcgtg ccaagccggt     480
gatcctggaa tcatggttga cggcaaggaa gtcgatgaca gctacacgct gcaacctgcg     540
caattcccca tcgatgagac ctccggttcc accgaatgcg gcggcaacta tttcggcccc     600
atcaccgtgc ctgacggcaa ctacttcatg atgggtgaca accgcaccaa ctccatggat     660
tcccgctacc acctggcgga tcagtaccaa ggaaccatcc ctgaggaaaa catcaagggc     720
aaagttcaag caattatcct gccatttagc cgaatcggtg gcgtcgacga ccctgccatc     780
aaaggctag                                                             789


<210>  2
<211>  262
<212>  PRT
<213>  Corynebacterium glutamicum

<400>  2
Met Thr Asp Phe Ser Ser Ala Ser Asn Ala Asp Asp Ser Thr Gln Asp
1               5                   10                  15
Gly Arg Pro Gly Arg Arg Ala Gly Lys Ser Lys Lys Glu Ser Lys Pro
            20                  25                  30
Thr Pro Trp Tyr Ile Glu Ile Pro Val Val Val Val Leu Thr Leu Ala
        35                  40                  45
Leu Ile Phe Val Leu Gln Thr Phe Val Gly Arg Met Tyr Met Ile Pro
    50                  55                  60
Ser Gly Ser Met Glu Pro Thr Leu His Gly Cys Glu Gly Cys Thr Gly
65                  70                  75                  80
Asp Arg Ile Leu Val Glu Lys Val Ser Tyr Tyr Phe Thr Asp Pro Glu
                85                  90                  95
Pro Gly Asp Val Val Val Phe Lys Gly Thr Asp Ser Trp Asn Val Gly
            100                 105                 110
Phe Thr Thr Gln Arg Ser Asp Asn Ser Val Ile Arg Gly Leu Gln Asn
        115                 120                 125
Leu Gly Ser Tyr Val Gly Leu Val Ala Pro Asp Glu Asn Asp Leu Val
    130                 135                 140
Lys Arg Ile Ile Ala Thr Gly Gly Gln Thr Val Ser Cys Gln Ala Gly
145                 150                 155                 160

Asp Pro Gly Ile Met Val Asp Gly Lys Glu Val Asp Asp Ser Tyr Thr
                165                 170                 175
Leu Gln Pro Ala Gln Phe Pro Ile Asp Glu Thr Ser Gly Ser Thr Glu
            180                 185                 190
Cys Gly Gly Asn Tyr Phe Gly Pro Ile Thr Val Pro Asp Gly Asn Tyr
        195                 200                 205
Phe Met Met Gly Asp Asn Arg Thr Asn Ser Met Asp Ser Arg Tyr His
    210                 215                 220
Leu Gly Asp Gln Tyr Gln Gly Thr Ile Pro Glu Glu Asn Ile Lys Gly
225                 230                 235                 240
Lys Val Gln Ala Ile Ile Leu Pro Phe Ser Arg Ile Gly Gly Val Asp
                245                 250                 255
Asp Pro Ala Ile Lys Gly
            260


<210>  3
<211>  30
<212>  DNA
<213>  Artificial Sequence

<220>
<223>  primer

<400>  3
gctaatcttc atggtaccag gcaaaaggta                                        30


<210>  4
<211>  35
<212>  DNA
<213>  Artificial Sequence

<220>
<223>  primer

<400>  4
gggggcgtcg atctagaggg ccctagcctt tgatg                                  35


<210>  5
<211>  34
<212>  DNA
<213>  Artificial Sequence

<220>
<223>  primer

<400>  5
cgatattatt taggggtacc caaattcctg tgaa                                   34


<210>  6
<211>  34
<212>  DNA
<213>  Artificial Sequence

<220>
<223>  primer

<400>  6
gctacctttt gcctggtacc tcacggccag ccct                                   34

<210>  7
<211>  34
<212>  DNA
<213>  Artificial Sequence

<220>
<223>  primer

<400>  7
gccatcaaag gctagggccc caaattcctg tgaa                                34


<210>  8
<211>  34
<212>  DNA
<213>  Artificial Sequence

<220>
<223>  primer

<400>  8
aggatccccg gaatgggccc tcacggccag ccct                                34


<210>  9
<211>  34
<212>  DNA
<213>  Artificial Sequence

<220>
<223>  primer

<400>  9
gctacctttt gcctggtacc ttaaaatcca cagc                                34


<210>  10
<211>  34
<212>  DNA
<213>  Artificial Sequence

<220>
<223>  primer

<400>  10
aggatccccg gaatgggccc ttaaaatcca cagc                                34


<210>  11
<211>  263
<212>  PRT
<213>  Unknown

<220>
<223>  MULTISPECIES

<400>  11


<210>  12
<211>  792
<212>  DNA
<213>  Unknown

<220>
<223>  MULTISPECIES

<400>  12
cacccagaaa cgctggtgaa agtaaaagat gctgaagatc agttgggtgc acgagtgggt      60
tacatcgaac tggatctcaa cagcggtaag atccttgaga gttttcgccc cgaagaacgt     120
tttccaatga tgagcacttt taaagttctg ctatgtggcg cggtattatc ccgtattgac     180
gccgggcaag agcaactcgg tcgccgcata cactattctc agaatgactt ggttgagtac     240
tcaccagtca cagaaaagca tcttacggat ggcatgacag taagagaatt atgcagtgct     300
gccataacca tgagtgataa cactgcggcc aacttacttc tgacaacgat cggaggaccg     360
aaggagctaa ccgctttttt gcacaacatg ggggatcatg taactcgcct tgatcgttgg     420
gaaccggagc tgaatgaagc cataccaaac gacgagcgtg acaccacgat gcctgtagca     480
atggcaacaa cgttgcgcaa actattaact ggcgaactac ttactctagc ttcccggcaa     540
caattaatag actggatgga ggcggataaa gttgcaggac cacttctgcg ctcggccctt     600
ccggctggct ggtttattgc tgataaatct ggagccggtg agcgtgggtc tcgcggtatc     660
attgcagcac tggggccaga tggtaagccc tcccgtatcg tagttatcta cacgacgggg     720
agtcaggcaa ctatggatga acgaaataga cagatcgctg agataggtgc ctcactgatt     780
aagcattggt aa                                                         792


<210>  13
<211>  34
<212>  DNA
<213>  Artificial Sequence

<220>
<223>  primer

<400>  13
gctacctttt gcctggtacc ttaccaatgc ttaa                                    34


<210>  14
<211>  34
<212>  DNA
<213>  Artificial Sequence

<220>
<223>  primer

<400>  14
aggatccccg gaatgggccc ttaccaatgc ttaa                                    34


<210>  15
<211>  109
<212>  PRT
<213>  Escherichia coli

<400>  15
Ala Ser Asp Lys Ile Ile His Leu Thr Asp Asp Ser Phe Asp Thr Asp
1               5                   10                  15
Val Leu Lys Ala Asp Gly Ala Ile Leu Val Asp Phe Trp Ala Glu Trp
            20                  25                  30
Cys Gly Pro Cys Lys Met Ile Ala Pro Ile Leu Asp Glu Ile Ala Asp
        35                  40                  45
Glu Tyr Gln Gly Lys Leu Thr Val Ala Lys Leu Asn Ile Asp Gln Asn
    50                  55                  60
Pro Gly Thr Ala Pro Lys Tyr Gly Ile Arg Gly Ile Pro Thr Leu Leu
65                  70                  75                  80
Leu Phe Lys Asn Gly Glu Val Ala Ala Thr Lys Val Gly Ala Leu Ser
                85                  90                  95
Lys Gly Gln Leu Lys Glu Phe Leu Asp Ala Asn Leu Ala
            100                 105


<210>  16
<211>  330
<212>  DNA
<213>  Escherichia coli

<400>  16
gcgtccgata agattattca ccttaccgat gactccttcg ataccgatgt cctcaaggca      60
gatggcgcta ttctcgtgga cttctgggca gaatggtgcg gcccatgcaa gatgatcgcc     120
ccaattctcg atgaaatcgc ggacgagtac cagggcaagt tgaccgtggc aaaactgaac     180
attgatcaga acccaggcac cgctcctaag tacggcatcc gcggtattcc taccctgctc     240
cttttcaaaa acggtgaggt cgcagctacc aaagtgggtg cgttgtctaa gggtcagctc     300
aaagagttcc tggatgctaa cctggcgtga                                      330


<210>  17
<211>  34
<212>  DNA
<213>  Artificial Sequence

<220>
<223>  primer

<400>  17
gctacctttt gcctggtacc tcacgccagg ttag                                    34

<210>  18
<211>  34
<212>  DNA
<213>  Artificial Sequence

<220>
<223>  primer

<400>  18
aggatccccg gaatgggccc tcacgccagg ttag                                34


<210>  19
<211>  127
<212>  PRT
<213>  Homo sapiens

<400>  19
Met Ser Phe Ser Gly Lys Tyr Gln Leu Gln Ser Gln Glu Asn Phe Glu
1               5                   10                  15
Ala Phe Met Lys Ala Ile Gly Leu Pro Glu Glu Leu Ile Gln Lys Gly
            20                  25                  30
Lys Asp Ile Lys Gly Val Ser Glu Ile Val Gln Asn Gly Lys His Phe
        35                  40                  45
Lys Phe Thr Ile Thr Ala Gly Ser Lys Val Ile Gln Asn Glu Phe Thr
    50                  55                  60
Val Gly Glu Glu Cys Glu Leu Glu Thr Met Thr Gly Glu Lys Val Lys
65                  70                  75                  80
Thr Val Val Gln Leu Glu Gly Asp Asn Lys Leu Val Thr Thr Phe Lys
                85                  90                  95
Asn Ile Lys Ser Val Thr Glu Leu Asn Gly Asp Ile Ile Thr Asn Thr
            100                 105                 110
Met Thr Leu Gly Asp Ile Val Phe Lys Arg Ile Ser Lys Arg Ile
        115                 120                 125


<210>  20
<211>  384
<212>  DNA
<213>  Homo sapiens

<400>  20
atgtccttct ccggcaagta ccagctgcag tcccaggaaa acttcgaggc attcatgaag      60
gctatcggtc tgccagaaga gctcatccag aagggcaagg atatcaaggg tgtttccgaa     120
atcgtgcaga acggcaagca cttcaagttc accatcaccg caggttccaa ggtcatccag     180
aacgagttca ccgttggcga agagtgcgaa ctcgagacca tgaccggtga aaaggttaag     240
accgtggtcc agctggaggg cgacaacaag ctcgtgacca ccttcaagaa catcaagtcc     300
gtcaccgaac tgaacggcga tatcatcacc aacaccatga ccctcggtga catcgtgttc     360
aagcgcatct ccaagcgtat ctaa                                            384


<210>  21
<211>  34
<212>  DNA
<213>  Artificial Sequence

<220>
<223>  primer

<400>  21
gctacctttt gcctggtacc ttagatacgc ttgg                                34

<210>  22
<211>  34
<212>  DNA
<213>  Artificial Sequence

<220>
<223>  primer

<400>  22
aggatccccg gaatgggccc ttagatacgc ttgg                                34


<210>  23
<211>  34
<212>  DNA
<213>  Artificial Sequence

<220>
<223>  primer

<400>  23
acgaattcga gctcggtacc caaattcctg tgaa                                34


<210>  24
<211>  34
<212>  DNA
<213>  Artificial Sequence

<220>
<223>  primer

<400>  24
ctagaggatc cccgggtacc tcacggccag ccct                                34


<210>  25
<211>  34
<212>  DNA
<213>  Artificial Sequence

<220>
<223>  primer

<400>  25
gctacctttt gcctggtacc tcacggccag ccct                                34


<210>  26
<211>  34
<212>  DNA
<213>  Artificial Sequence

<220>
<223>  primer

<400>  26
gccatcaaag gctagggccc caaattcctg tgaa                                34


<210>  27

<211>  34
<212>  DNA
<213>  Artificial Sequence

<220>
<223>  primer

<400>  27
aggtcgactc tagagggccc tcacggccag ccct                                34


<210>  28
<211>  1458
<212>  DNA
<213>  Corynebacterium glutamicum

<400>  28
atggaaaacc cttatgtcgc tgcgctcgat gacgataaaa aagaagtcgg cgcaataaaa      60
gaagcagaaa aagaacctga aataggtccc atcagagctg ccggacgagc cataccgctg     120
cgcacccgca tcattttgat cgtggtgggt atcgccgggc ttggtttgct ggtcaacgcg     180
attgctgttt ccagcctcat gcgtgaagtt tcctataccc gcatggatca agagctagag     240
acctcgatgg ggacgtgggc gcataacgtt gagctgttta atttcgatgg cgtccgccaa     300
gggccaccca gcgattatta tgtggccaag gtttttcctg atggatccag cattatttcc      360
aacgatgcac aatcggcacc caatctagct gaaaccacca tcggtactgg tccacacact     420
gtggatgctg ctagcggttc tgcctccaac actccgtggc gtgtgatggc ggaaaagaac     480
ggtgacatta tcaccgtggt gggtaaaagc atggggcgtg aaacaaacct gctgtaccga      540
ttggtgatgg tgcagatgat catcggcgcg ctgattctgg ttgctatttt gattacttca     600
ctcttcctag tcagacgctc gttgcggccg ttgagagaag ttgaagagac cgccaccagg     660
attgcgggcg gtgatttgga tcgacgtgtc ccgcagtggc caatgaccac agaagtcgga      720
cagctgtcga atgccctcaa tatcatgttg gagcagctcc aagcctcaat tctgaccgcc     780
cagcaaaaag aagctcagat gcgccgattc gttggcgacg cctcccacga gctccgcaca     840
ccactgacct ctgtgaaggg cttcaccgag ctgtattcat caggtgcaac agatgatgcc     900
aactgggtca tgtccaagat cggtggcgaa gcccaacgca tgagtgtgct tgtggaagac     960
ctcctgtcac tgacgcgtgc cgaaggccag caaatggaga agcaccgcgt tgacgtgctg    1020
gaactcgcat tggcagtacg cggatccatg cgagcagcct ggccagatcg caccgtcaac    1080
gtgtccaata aagccgagtc cattccagtt gttgaaggcg acccaacccg cctccaccaa    1140
gttctcacca acctggttgc caacggactc aaccacggcg gaccggacgc ggaagtcagc    1200
attgagatca acaccgatgg gcaaaacgtg aggattctcg tggcagacaa cggtgtcgga    1260
atgtctgaag aagatgccca gcatatcttc gagcgtttct accgcgccga ttcctcccgc    1320
tcacgcgcat ccggcggatc gggcctcggc cttgcgatca cgaaatccct ggtcgaaggc    1380
cacggcggca cagtcaccgt cgacagcgtg caaggcgaag gcacggtgtt cacgatcacc    1440
ttgccggcgg tttcttaa                                                  1458


<210>  29
<211>  485
<212>  PRT
<213>  Corynebacterium glutamicum

<400>  29
Met Glu Asn Pro Tyr Val Ala Ala Leu Asp Asp Asp Lys Lys Glu Val
1               5                   10                  15
Gly Ala Ile Lys Glu Ala Glu Lys Glu Pro Glu Ile Gly Pro Ile Arg
            20                  25                  30
Ala Ala Gly Arg Ala Ile Pro Leu Arg Thr Arg Ile Ile Leu Ile Val
        35                  40                  45
Val Gly Ile Ala Gly Leu Gly Leu Leu Val Asn Ala Ile Ala Val Ser
    50                  55                  60
Ser Leu Met Arg Glu Val Ser Tyr Thr Arg Met Asp Gln Glu Leu Glu
65                  70                  75                  80
Thr Ser Met Gly Thr Trp Ala His Asn Val Glu Leu Phe Asn Phe Asp
                85                  90                  95
Gly Val Arg Gln Gly Pro Pro Ser Asp Tyr Tyr Val Ala Lys Val Phe

                100                 105                 110
Pro Asp Gly Ser Ser Ile Ile Phe Asn Asp Ala Gln Ser Ala Pro Asn
        115                 120                 125
Leu Ala Glu Thr Thr Ile Gly Thr Gly Pro His Thr Val Asp Ala Ala
    130                 135                 140
Ser Gly Ser Ala Ser Asn Thr Pro Trp Arg Val Met Ala Glu Lys Asn
145                 150                 155                 160
Gly Asp Ile Ile Thr Val Val Gly Lys Ser Met Gly Arg Glu Thr Asn
                165                 170                 175
Leu Leu Tyr Arg Leu Val Met Val Gln Met Ile Ile Gly Ala Leu Ile
            180                 185                 190
Leu Val Ala Ile Leu Ile Thr Ser Leu Phe Leu Val Arg Arg Ser Leu
        195                 200                 205
Arg Pro Leu Arg Glu Val Glu Glu Thr Ala Thr Arg Ile Ala Gly Gly
    210                 215                 220
Asp Leu Asp Arg Arg Val Pro Gln Trp Pro Met Thr Thr Glu Val Gly
225                 230                 235                 240
Gln Leu Ser Asn Ala Leu Asn Ile Met Leu Glu Gln Leu Gln Ala Ser
                245                 250                 255
Ile Leu Thr Ala Gln Gln Lys Glu Ala Gln Met Arg Arg Phe Val Gly
            260                 265                 270
Asp Ala Ser His Glu Leu Arg Thr Pro Leu Thr Ser Val Lys Gly Phe
        275                 280                 285
Thr Glu Leu Tyr Ser Ser Gly Ala Thr Asp Asp Ala Asn Trp Val Met
    290                 295                 300
Ser Lys Ile Gly Gly Glu Ala Gln Arg Met Ser Val Leu Val Glu Asp
305                 310                 315                 320
Leu Leu Ser Leu Thr Arg Ala Glu Gly Gln Gln Met Glu Lys His Arg
                325                 330                 335
Val Asp Val Leu Glu Leu Ala Leu Ala Val Arg Gly Ser Met Arg Ala
            340                 345                 350
Ala Trp Pro Asp Arg Thr Val Asn Val Ser Asn Lys Ala Glu Ser Ile
        355                 360                 365
Pro Val Val Glu Gly Asp Pro Thr Arg Leu His Gln Val Leu Thr Asn
    370                 375                 380
Leu Val Ala Asn Gly Leu Asn His Gly Gly Pro Asp Ala Glu Val Ser
385                 390                 395                 400
Ile Glu Ile Asn Thr Asp Gly Gln Asn Val Arg Ile Leu Val Ala Asp
                405                 410                 415
Asn Gly Val Gly Met Ser Glu Glu Asp Ala Gln His Ile Phe Glu Arg
            420                 425                 430
Phe Tyr Arg Ala Asp Ser Ser Arg Ser Arg Ala Ser Gly Gly Ser Gly
        435                 440                 445
Leu Gly Leu Ala Ile Thr Lys Ser Leu Val Glu Gly His Gly Gly Thr
    450                 455                 460
Val Thr Val Asp Ser Val Gln Gly Glu Gly Thr Val Phe Thr Ile Thr
465                 470                 475                 480
Leu Pro Ala Val Ser
                485

<210>  30
<211>  485
<212>  PRT
<213>  Corynebacterium glutamicum

<400>  30
Met Glu Asn Pro Tyr Val Ala Ala Leu Asp Asp Glu Asn Gln Glu Val
1               5                   10                  15
Gly Val Lys Lys Glu Ala Glu Lys Glu Pro Glu Ile Gly Pro Ile Arg
            20                  25                  30
Ala Ala Gly Arg Ala Ile Pro Leu Arg Thr Arg Ile Ile Leu Ile Val
        35                  40                  45

Val Gly Ile Ala Gly Leu Gly Leu Leu Val Asn Ala Ile Ala Val Ser
    50                  55                  60
Ser Leu Met Arg Glu Val Ser Tyr Thr Arg Met Asp Gln Glu Leu Glu
65                  70                  75                  80
Thr Ser Met Gly Thr Trp Ala His Asn Val Glu Leu Phe Asn Phe Asp
                85                  90                  95
Gly Val Arg Gln Gly Pro Pro Ser Asp Tyr Tyr Val Ala Lys Val Phe
            100                 105                 110
Pro Asp Gly Ser Ser Ile Ile Phe Asn Asp Ala Gln Ser Ala Pro Asp
        115                 120                 125
Leu Ala Glu Thr Thr Ile Gly Thr Gly Pro His Thr Val Asp Ala Ala
    130                 135                 140
Ser Gly Ser Ala Ser Asn Thr Pro Trp Arg Val Met Ala Glu Lys Asn
145                 150                 155                 160
Gly Asp Ile Ile Thr Val Val Gly Lys Ser Met Gly Arg Glu Thr Asn
                165                 170                 175
Leu Leu Tyr Arg Leu Val Met Val Gln Met Ile Ile Gly Ala Leu Ile
            180                 185                 190
Leu Val Ala Ile Leu Ile Thr Ser Leu Phe Leu Val Arg Arg Ser Leu
        195                 200                 205
Arg Pro Leu Arg Glu Val Glu Glu Thr Ala Thr Arg Ile Ala Gly Gly
    210                 215                 220
Asp Leu Asp Arg Arg Val Pro Gln Trp Pro Met Thr Thr Glu Val Gly
225                 230                 235                 240
Gln Leu Ser Asn Ala Leu Asn Ile Met Leu Glu Gln Leu Gln Ala Ser
                245                 250                 255
Ile Leu Thr Ala Gln Gln Lys Glu Ala Gln Met Arg Arg Phe Val Gly
            260                 265                 270
Asp Ala Ser His Glu Leu Arg Thr Pro Leu Thr Ser Val Lys Gly Phe
        275                 280                 285
Thr Glu Leu Tyr Ser Ser Gly Ala Thr Asp Asp Ala Asn Trp Val Met
    290                 295                 300
Ser Lys Ile Gly Gly Glu Ala Gln Arg Met Ser Val Leu Val Glu Asp
305                 310                 315                 320
Leu Leu Ser Leu Thr Arg Ala Glu Gly Gln Gln Met Glu Lys His Arg
                325                 330                 335
Val Asp Val Leu Glu Leu Ala Leu Ala Val Arg Gly Ser Met Arg Ala
            340                 345                 350
Ala Trp Pro Asp Arg Thr Val Asn Val Ser Asn Lys Ala Glu Ser Ile
        355                 360                 365
Pro Val Val Lys Gly Asp Pro Thr Arg Leu His Gln Val Leu Thr Asn
    370                 375                 380
Leu Val Ala Asn Gly Leu Asn His Gly Gly Pro Asp Ala Glu Val Ser
385                 390                 395                 400
Ile Glu Ile Asn Thr Asp Gly Gln Asn Val Arg Ile Leu Val Ala Asp
                405                 410                 415
Asn Gly Val Gly Met Ser Glu Glu Asp Ala Gln His Ile Phe Glu Arg
            420                 425                 430
Phe Tyr Arg Ala Asp Ser Ser Arg Ser Arg Ala Ser Gly Gly Ser Gly
        435                 440                 445
Leu Gly Leu Ala Ile Thr Lys Ser Leu Val Glu Gly His Gly Gly Thr
    450                 455                 460
Val Thr Val Asp Ser Val Gln Gly Glu Gly Thr Val Phe Thr Ile Thr
465                 470                 475                 480
Leu Pro Ala Val Ser
                485

<210>  31
<211>  485
<212>  PRT
<213>  Corynebacterium glutamicum

<400> 31
Met Glu Asn Pro Tyr Val Ala Ala Leu Asp Asp Glu Asn Gln Glu Val
1               5                   10                  15
Gly Val Lys Lys Glu Ala Glu Lys Glu Pro Glu Ile Gly Pro Ile Arg
            20                  25                  30
Ala Ala Gly Arg Ala Ile Pro Leu Arg Thr Arg Ile Ile Leu Ile Val
        35                  40                  45
Val Gly Ile Ala Gly Leu Gly Leu Leu Val Asn Ala Ile Ala Val Ser
    50                  55                  60
Ser Leu Met Arg Glu Val Ser Tyr Thr Arg Met Asp Gln Glu Leu Glu
65                  70                  75                  80
Thr Ser Met Gly Thr Trp Ala His Asn Val Glu Leu Phe Asn Phe Asp
                85                  90                  95
Gly Val Arg Gln Gly Pro Pro Ser Asp Tyr Tyr Val Ala Lys Val Phe
            100                 105                 110
Pro Asp Gly Ser Ser Ile Ile Phe Asn Asp Ala Gln Ser Ala Pro Asp
        115                 120                 125
Leu Ala Glu Thr Thr Ile Gly Thr Gly Pro His Thr Val Asp Ala Ala
    130                 135                 140
Ser Gly Ser Ala Ser Asn Thr Pro Trp Arg Val Met Ala Glu Lys Asn
145                 150                 155                 160
Gly Asp Ile Ile Thr Val Val Gly Lys Ser Met Gly Arg Glu Thr Asn
                165                 170                 175
Leu Leu Tyr Arg Leu Val Val Val Gln Met Ile Ile Gly Ala Leu Ile
            180                 185                 190
Leu Val Ala Ile Leu Ile Thr Ser Leu Phe Leu Val Arg Arg Ser Leu
        195                 200                 205
Arg Pro Leu Arg Glu Val Glu Glu Thr Ala Thr Arg Ile Ala Gly Gly
    210                 215                 220
Asp Leu Asp Arg Arg Val Pro Gln Trp Pro Met Thr Thr Glu Val Gly
225                 230                 235                 240
Gln Leu Ser Asn Ala Leu Asn Ile Met Leu Glu Gln Leu Gln Ala Ser
                245                 250                 255
Ile Leu Thr Ala Gln Gln Lys Glu Ala Gln Met Arg Arg Phe Val Gly
            260                 265                 270
Asp Ala Ser His Glu Leu Arg Thr Pro Leu Thr Ser Val Lys Gly Phe
        275                 280                 285
Thr Glu Leu Tyr Ser Ser Gly Ala Thr Asp Asp Ala Asn Trp Val Met
    290                 295                 300
Ser Lys Ile Gly Gly Glu Ala Gln Arg Met Ser Val Leu Val Glu Asp
305                 310                 315                 320
Leu Leu Ser Leu Thr Arg Ala Glu Gly Gln Gln Met Glu Lys His Arg
                325                 330                 335
Val Asp Val Leu Glu Leu Ala Leu Ala Val Arg Gly Ser Met Arg Ala
            340                 345                 350
Ala Trp Pro Asp Arg Thr Val Asn Val Ser Asn Lys Ala Glu Ser Ile
        355                 360                 365
Pro Val Val Glu Gly Asp Pro Thr Arg Leu His Gln Val Leu Thr Asn
    370                 375                 380
Leu Val Ala Asn Gly Leu Asn His Gly Gly Pro Asp Ala Glu Val Ser
385                 390                 395                 400
Ile Glu Ile Asn Thr Asp Gly Gln Asn Val Arg Ile Leu Val Ala Asp
                405                 410                 415
Asn Gly Val Gly Met Ser Glu Glu Asp Ala Gln His Ile Phe Glu Arg
            420                 425                 430
Phe Tyr Arg Ala Asp Ser Ser Arg Ser Arg Ala Ser Gly Gly Ser Gly
        435                 440                 445
Leu Gly Leu Ala Ile Thr Lys Ser Leu Val Glu Gly His Gly Gly Thr
    450                 455                 460
Val Thr Val Asp Ser Val Gln Gly Glu Gly Thr Val Phe Thr Ile Thr
465                 470                 475                 480
Leu Pro Ala Val Ser
                485

<210>  32
<211>  504
<212>  PRT
<213>  Corynebacterium callunae

<400>  32
Met Glu Asn Pro Tyr Val Ala Ala Leu Asp Lys Asn Ser Asn Phe Gly
1               5                   10                  15
Ala Lys Asp Thr Asp Ser Ala Val Ser Asp Ser Thr Glu Val Ser Gln
            20                  25                  30
Asn Asn Asp Gly Ile Gly Thr Pro Ala Thr Ala Glu Pro Lys Val Gly
        35                  40                  45
Pro Ile Arg Thr Ala Gly Arg Ala Met Pro Leu Arg Thr Arg Ile Ile
    50                  55                  60
Leu Leu Val Val Gly Ile Ala Gly Leu Gly Leu Leu Val Asn Ala Val
65                  70                  75                  80
Ala Val Ser Ser Leu Met Arg Glu Val Ser Tyr Thr Arg Met Asp Gln
                85                  90                  95
Asp Leu Glu Ser Ala Met Gly Thr Trp Val Arg Asn Val Glu Leu Phe
            100                 105                 110
Asn Phe Asp Gly Val Arg Gln Gly Pro Pro Ser Asp Tyr Tyr Val Ala
        115                 120                 125
Lys Val Phe Pro Asp Gly Ser Ser Ile Ile Phe Asn Asp Ala Glu Ser
    130                 135                 140
Ala Pro Asp Leu Gly Gln Thr Thr Ile Gly Thr Gly Pro His Thr Val
145                 150                 155                 160
Glu Ala Ala Glu Gly Ser Ala Ser Ser Thr His Trp Arg Val Met Ala
                165                 170                 175
Ala Lys Asn Gly Asp Val Ile Thr Val Val Gly Lys Ser Met Gly Arg
            180                 185                 190
Glu Ser Thr Leu Leu Tyr Arg Leu Val Val Val Gln Met Val Ile Gly
        195                 200                 205
Val Leu Ile Leu Ile Ala Ile Leu Ile Gly Ser Phe Phe Leu Val Arg
    210                 215                 220
Arg Ser Leu Lys Pro Leu Arg Glu Val Glu Glu Thr Ala Ser Arg Ile
225                 230                 235                 240
Ala Gly Gly Glu Leu Asp Arg Arg Val Pro Gln Trp Pro Met Thr Thr
                245                 250                 255
Glu Val Gly Gln Leu Ala Asn Ala Leu Asn Ile Met Leu Glu Gln Leu
            260                 265                 270
Gln Thr Ser Ile Met Asn Ala Gln Gln Lys Glu Ala Gln Met Arg Arg
        275                 280                 285
Phe Val Gly Asp Ala Ser His Glu Leu Arg Thr Pro Leu Thr Ser Val
    290                 295                 300
Lys Gly Phe Thr Glu Leu Tyr Ser Ser Gly Ala Thr Gln Asp Ala Asp
305                 310                 315                 320
Trp Val Leu Ser Lys Ile Gly Gly Glu Ala Gln Arg Met Ser Val Leu
                325                 330                 335
Val Glu Asp Leu Leu Ser Leu Thr Arg Ala Glu Gly Gln Gln Met Glu
            340                 345                 350
Lys His Arg Val Asp Met Leu Glu Leu Ala Leu Ala Val Arg Gly Ser
        355                 360                 365
Leu Lys Ala Ala Trp Pro Asp Arg Thr Val Asn Val Ala Asn Arg Ser
    370                 375                 380
Glu Asn Ile Pro Val Val Glu Gly Asp Pro Thr Arg Leu His Gln Val
385                 390                 395                 400
Leu Thr Asn Leu Val Ala Asn Gly Leu Asn His Gly Gly Pro Glu Ala
                405                 410                 415
Glu Val Asn Ile Gln Val Glu Thr Ala Asp Asp Lys Val Lys Ile Leu
            420                 425                 430
Val Ile Asp Asn Gly Val Gly Met Ser Lys Glu Asp Ala Glu His Ile

                435                 440                 445
Phe Glu Arg Phe Tyr Arg Ala Asp Thr Ser Arg Ser Arg Ala Ser Gly
            450                 455                 460
Gly Ser Gly Leu Gly Leu Ala Ile Thr Lys Ser Leu Val Glu Gly His
465                 470                 475                 480
Gly Gly Thr Ile Thr Val Asp Ser Glu Leu Gly Lys Gly Thr Val Phe
                485                 490                 495
Ser Ile Ile Leu Pro Ala Ala Glu
            500

<210>  33
<211>  458
<212>  PRT
<213>  Corynebacterium crenatum

<400>  33
Ile Gly Pro Ile Arg Ala Ala Gly Arg Ala Ile Pro Leu Arg Thr Arg
1               5                   10                  15
Ile Ile Leu Ile Val Val Gly Ile Ala Gly Leu Gly Leu Leu Val Asn
            20                  25                  30
Ala Ile Ala Val Ser Ser Leu Met Arg Glu Val Ser Tyr Thr Arg Met
        35                  40                  45
Asp Gln Glu Leu Glu Thr Ser Met Gly Thr Trp Ala His Asn Val Glu
    50                  55                  60
Leu Phe Asn Phe Asp Gly Val Arg Gln Gly Pro Pro Ser Asp Tyr Tyr
65                  70                  75                  80
Val Ala Lys Val Phe Pro Asp Gly Ser Ser Ile Ile Phe Asn Asp Ala
                85                  90                  95
Gln Ser Ala Pro Asp Leu Ala Glu Thr Thr Ile Gly Thr Gly Pro His
            100                 105                 110
Thr Val Asp Ala Ala Ser Gly Ser Ala Ser Asn Thr Pro Trp Arg Val
        115                 120                 125
Met Ala Glu Lys Asn Gly Asp Ile Ile Thr Val Val Gly Lys Ser Met
    130                 135                 140
Gly Arg Glu Thr Asn Leu Leu Tyr Arg Leu Val Met Val Gln Met Ile
145                 150                 155                 160
Ile Gly Ala Leu Ile Leu Val Ala Ile Leu Ile Thr Ser Leu Phe Leu
                165                 170                 175
Val Arg Arg Ser Leu Arg Pro Leu Arg Glu Val Glu Glu Thr Ala Thr
            180                 185                 190
Arg Ile Ala Gly Gly Asp Leu Asp Arg Arg Val Pro Gln Trp Pro Met
        195                 200                 205
Thr Thr Glu Val Gly Gln Leu Ser Asn Ala Leu Asn Ile Met Leu Glu
    210                 215                 220
Gln Leu Gln Ala Ser Ile Leu Ser Ala Gln Gln Lys Glu Ala Gln Met
225                 230                 235                 240
Arg Arg Phe Val Gly Asp Ala Ser His Glu Leu Arg Thr Pro Leu Thr
                245                 250                 255
Ser Val Lys Gly Phe Thr Glu Leu Tyr Ser Ser Gly Ala Thr Asp Asp
            260                 265                 270
Ala Asn Trp Val Met Ser Lys Ile Gly Gly Glu Ala Gln Arg Met Ser
        275                 280                 285
Val Leu Val Glu Asp Leu Leu Ser Leu Thr Arg Ala Glu Gly Gln Gln
    290                 295                 300
Met Glu Lys His Arg Val Asp Val Leu Glu Leu Ala Leu Ala Val Arg
305                 310                 315                 320
Gly Ser Met Arg Ala Ala Trp Pro Asp Arg Thr Val Asn Val Ser Asn
                325                 330                 335
Lys Ala Ala Ser Ile Pro Val Val Glu Gly Asp Pro Thr Arg Leu His
            340                 345                 350
Gln Val Leu Thr Asn Leu Val Ala Asn Gly Leu Asn His Gly Gly Pro
        355                 360                 365

Asp Ala Glu Val Ser Ile Glu Ile Asn Thr Asp Gly Gln Asn Val Arg
    370                 375                 380
Ile Leu Val Ala Asp Asn Gly Val Gly Met Ser Glu Glu Asp Ala Gln
385                 390                 395                 400
His Ile Phe Glu Arg Phe Tyr Arg Ala Asp Ser Ser Arg Ser Arg Ala
                405                 410                 415
Ser Gly Gly Ser Gly Leu Gly Leu Ala Ile Thr Lys Ser Leu Val Glu
            420                 425                 430
Gly His Gly Gly Thr Val Thr Val Asp Ser Val Gln Gly Glu Gly Thr
        435                 440                 445
Val Phe Thr Ile Thr Leu Pro Ala Val Ser
    450                 455

<210>  34
<211>  471
<212>  PRT
<213>  Corynebacterium efficiens

<400>  34
Met Thr Ala Pro Glu Asn Pro His Ala Gln Val Thr Pro Val Gly Arg
1               5                   10                  15
Phe Arg Gln Ala Ala Arg Gly Val Pro Leu Arg Thr Arg Ile Ile Leu
            20                  25                  30
Leu Val Val Gly Ile Ala Gly Leu Gly Leu Leu Val Asn Ala Ile Ala
        35                  40                  45
Val Ser Ser Leu Met Arg Glu Val Ser Tyr Ser Arg Met Asp Gln Glu
    50                  55                  60
Leu Glu Ser Ala Met Asn Ser Trp Ala Gln Thr Ala Glu Leu Phe Gly
65                  70                  75                  80
Ser Ile Thr Leu Gly Pro Pro Ser Asp Tyr Tyr Val Val Arg Ile Phe
                85                  90                  95
Pro Asp Gly Ser His Met Val Phe Asn Gln Ser Asp Ser Ala Pro Asp
            100                 105                 110
Leu Gly Glu Thr Thr Ile Gly Ile Gly Pro His Thr Ala Ser Ala Ala
        115                 120                 125
Pro Gly Ser Ser Ser Ser Val Pro Trp Arg Val Ile Ala Ile Ser Asp
    130                 135                 140
Asn Gly Thr Ile Thr Val Val Gly Lys Ser Leu Ala Pro Glu Ser Met
145                 150                 155                 160
Leu Leu Tyr Arg Leu Val Ile Val Gln Leu Val Ile Gly Met Leu Ile
                165                 170                 175
Val Val Ala Ile Leu Leu Ser Ser Leu Tyr Leu Val Asn Arg Ser Leu
            180                 185                 190
Arg Pro Leu Arg Glu Val Glu Lys Thr Ala Lys Ser Ile Ala Gly Gly
        195                 200                 205
Asp Leu Asp Arg Arg Val Pro Gln Trp Pro Met Thr Thr Glu Val Gly
    210                 215                 220
Gln Leu Ala Asn Ala Leu Asn Ile Met Leu Glu Gln Leu Gln Ala Ser
225                 230                 235                 240
Ile Leu Ser Ala Gln Glu Lys Glu Ser Gln Met Arg Arg Phe Val Gly
                245                 250                 255
Asp Ala Ser His Glu Leu Arg Thr Pro Leu Thr Ser Val Lys Gly Tyr
            260                 265                 270
Ser Glu Leu Tyr His Ser Gly Ala Thr Arg Asp Ala Asp Trp Val Leu
        275                 280                 285
Ser Lys Ile Ser Gly Glu Ala Gln Arg Met Ser Val Leu Val Glu Asp
    290                 295                 300
Leu Leu Ser Leu Thr Arg Ala Glu Gly Gln Gln Met Glu Lys Arg Pro
305                 310                 315                 320
Val Asp Val Leu Glu Leu Ser Leu Ser Val Ala Ser Ser Met Arg Ala
                325                 330                 335
Ala Trp Pro Glu Arg Ser Ile Thr Val Val Asn Lys Thr Gly Ser Leu

340                 345                 350
Pro Val Val Glu Gly Asp Ala Thr Arg Leu His Gln Val Leu Thr Asn
        355                 360                 365
Leu Val Asn Asn Gly Leu Asn His Gly Gly Pro Asp Ala Ser Val Glu
    370                 375                 380
Ile Glu Ile Ser Ala Glu Gly Gly Ser Val Leu Val Arg Val Val Asp
385                 390                 395                 400
Asp Gly Val Gly Met Thr Ala Glu Asp Ala Gln His Ile Phe Glu Arg
                405                 410                 415
Phe Tyr Arg Thr Asp Thr Ser Arg Ser Arg Ala Ser Gly Gly Ser Gly
            420                 425                 430
Leu Gly Leu Ala Ile Thr Lys Ser Leu Val Glu Gly His Arg Gly Thr
        435                 440                 445
Ile Thr Val Asp Ser Glu Val Gly Glu Gly Thr Val Phe Thr Ile Thr
    450                 455                 460
Leu Pro Ser Arg Met Glu Asp
465                 470


<210>  35
<211>  708
<212>  DNA
<213>  Corynebacterium glutamicum

<400>  35
atggacaacc agtctgacgg acaaatccgc gtactcgtcg ttgatgacga gccaaacatc      60
gtcgagctgc tcaccgtaag ccttaaattc caaggcttcg cagtgatgac cgccaacgat     120
ggcaatgaag ccctgaagat tgctcgtgag ttccgtccag acgcatacat cctcgatgtc     180
atgatgccag gaatggacgg cttcgagctg ctgaccaagc tgcgcggcga aggccttgac     240
agcccagttc tgtacctcac cgcaaaggat gccgtggagc accgcatcca cggcctgacc     300
atcggcgctg acgactacgt gaccaagcct ttctccctgg aagaagtaat cacccgcctg     360
cgcgtgattc ttcgtcgcgg tggagcagtt gaagaagaca cctcaacttc cctgcagtac     420
gcagacctca ccctcaacga tgaaacccac gaggtcacca aggctggcga actgatcgat     480
ctttccccaa ctgaattcaa cctcctgcgc tacctcatgc tcaacgctga agtggtgctg     540
tccaaggcaa agatcctgga taacgtgtgg cactacgatt ttggtggcga cggcaacgtc     600
gtggaatcct acatctccta cctgcgccgc aaggtggaca cccaggatcc gcagctaatt     660
cagactgttc gtggcgttgg atatgttctg cgcaccccac gtagctaa                  708


<210>  36
<211>  235
<212>  PRT
<213>  Corynebacterium glutamicum

<400>  36
Met Asp Asn Gln Ser Asp Gly Gln Ile Arg Val Leu Val Val Asp Asp
1               5                   10                  15
Glu Pro Asn Ile Val Glu Leu Leu Thr Val Ser Leu Lys Phe Gln Gly
            20                  25                  30
Phe Ala Val Met Thr Ala Asn Asp Gly Asn Glu Ala Leu Lys Ile Ala
        35                  40                  45
Arg Glu Phe Arg Pro Asp Ala Tyr Ile Leu Asp Val Met Met Pro Gly
    50                  55                  60
Met Asp Gly Phe Glu Leu Leu Thr Lys Leu Arg Gly Glu Gly Leu Asp
65                  70                  75                  80
Ser Pro Val Leu Tyr Leu Thr Ala Lys Asp Ala Val Glu His Arg Ile
                85                  90                  95
His Gly Leu Thr Ile Gly Ala Asp Asp Tyr Val Thr Lys Pro Phe Ser
            100                 105                 110
Leu Glu Glu Val Ile Thr Arg Leu Arg Val Ile Leu Arg Arg Gly Gly
        115                 120                 125
Ala Val Glu Glu Asp Thr Ser Thr Ser Leu Gln Tyr Ala Asp Leu Thr
    130                 135                 140

Leu Asn Asp Glu Thr His Glu Val Thr Lys Ala Gly Glu Leu Ile Asp
145                 150                 155                 160
Leu Ser Pro Thr Glu Phe Asn Leu Leu Arg Tyr Leu Met Leu Asn Ala
                165                 170                 175
Glu Val Val Leu Ser Lys Ala Lys Ile Leu Asp Asn Val Trp His Tyr
            180                 185                 190
Asp Phe Gly Gly Asp Gly Asn Val Val Glu Ser Tyr Ile Ser Tyr Leu
        195                 200                 205
Arg Arg Lys Val Asp Thr Gln Asp Pro Gln Leu Ile Gln Thr Val Arg
    210                 215                 220
Gly Val Gly Tyr Val Leu Arg Thr Pro Arg Ser
225                 230                 235


<210>  37
<211>  1500
<212>  DNA
<213>  Corynebacterium glutamicum

<400>  37
atgtttaaca accgtatccg cactgcagct ctcgctggtg caatcgcaat ctccaccgca      60
gcttccggcg tagctatccc agcattcgct caggagacca acccaacctt caacatcaac     120
aacggcttca acgatgctga tggatccacc atccagccag ttgagccagt taaccacacc     180
gaggaaaccc tccgcgacct gactgactcc accggcgctt acctggaaga gttccagtac     240
ggcaacgttg aggaaatcgt tgaagcatac ctgcaggttc aggcttccgc agacggattc     300
gatccttctg agcaggctgc ttacgaggct ttcgaggctg ctcgcgttcg tgcatcccag     360
gagctcgcgg cttccgctga gaccatcact aagacccgcg agtccgttgc ttacgcactc     420
aaggctgacc gcgaagctac cgcagctttc gaggcttacc tcagcgctct tcgtcaggtt     480
tcagtcatca acgatctgat cgctgatgct aacgccaaga acaagactga ctttgcagag     540
atcgagctct acgatgttct ttacaccgac gccgacatct ctggcgatgc tccacttctt     600
gctcctgcat acaaggagct gaaggacctt caggctgagg ttgacgcaga cttcgagtgg     660
ttgggcgagt tcgcaattga taacaatgaa gacaactacg tcattcgtac tcacatccct     720
gctgtagagg cactcaaggc agcgatcgat tcactggtcg acaccgttga gccacttcgt     780
gcagacgcta tcgctaagaa catcgaggct cagaagtctg acgttctggt tccccagctc     840
ttcctcgagc gtgcaactgc acagcgcgac accctgcgtg ttgtagaggc aatcttctct     900
acctctgctc gttacgttga actctacgag aacgtcgaga acgttaacgt tgagaacaag     960
acccttcgcc agcactactc ttccctgatc cctaacctct tcatcgcagc ggttggcaac    1020
atcaacgagc tcaacaatgc agatcaggct gcacgtgagc tcttcctcga ttgggacacc    1080
gacctcacca ccaacgatga ggacgaagct tactaccagg ctaagctcga cttcgctatc    1140
gagacctacg caaagatcct gatcaacggt gaagtttggc aggagccact cgcttacgtc    1200
cagaacctgg atgcaggcgc acgtcaggaa gcagctgacc gcgaagcaga gcgcgcagct    1260
gacgcagcat accgcgctga gcagctccgc atcgctcagg aagcagctga cgctcagaag    1320
gctctcgctg aggctcttgc taatgcaggc aacaacgaca acggtggcga caactcctcc    1380
gacgacaagg gaaccggttc ttccgacatc ggaacctggg gacctttcgc agcaattgca    1440
gctatcatcg cagcaatcgc agctatcttc ccattcctct ccggtatcgt taagttctaa    1500


<210>  38
<211>  499
<212>  PRT
<213>  Corynebacterium glutamicum

<400>  38
Met Phe Asn Asn Arg Ile Arg Thr Ala Ala Leu Ala Gly Ala Ile Ala
1               5                   10                  15
Ile Ser Thr Ala Ala Ser Gly Val Ala Ile Pro Ala Phe Ala Gln Glu
            20                  25                  30
Thr Asn Pro Thr Phe Asn Ile Asn Asn Gly Phe Asn Asp Ala Asp Gly
        35                  40                  45
Ser Thr Ile Gln Pro Val Glu Pro Val Asn His Thr Glu Glu Thr Leu
    50                  55                  60
Arg Asp Leu Thr Asp Ser Thr Gly Ala Tyr Leu Glu Glu Phe Gln Tyr
65                  70                  75                  80

Gly Asn Val Glu Glu Ile Val Glu Ala Tyr Leu Gln Val Gln Ala Ser
                85                  90                  95
Ala Asp Gly Phe Asp Pro Ser Glu Gln Ala Ala Tyr Glu Ala Phe Glu
            100                 105                 110
Ala Ala Arg Val Arg Ala Ser Gln Glu Leu Ala Ala Ser Ala Glu Thr
        115                 120                 125
Ile Thr Lys Thr Arg Glu Ser Val Ala Tyr Ala Leu Lys Ala Asp Arg
    130                 135                 140
Glu Ala Thr Ala Ala Phe Glu Ala Tyr Leu Ser Ala Leu Arg Gln Val
145                 150                 155                 160
Ser Val Ile Asn Asp Leu Ile Ala Asp Ala Asn Ala Lys Asn Lys Thr
                165                 170                 175
Asp Phe Ala Glu Ile Glu Leu Tyr Asp Val Leu Tyr Thr Asp Ala Asp
            180                 185                 190
Ile Ser Gly Asp Ala Pro Leu Leu Ala Pro Ala Tyr Lys Glu Leu Lys
        195                 200                 205
Asp Leu Gln Ala Glu Val Asp Ala Asp Phe Glu Trp Leu Gly Glu Phe
    210                 215                 220
Ala Ile Asp Asn Asn Glu Asp Asn Tyr Val Ile Arg Thr His Ile Pro
225                 230                 235                 240
Ala Val Glu Ala Leu Lys Ala Ala Ile Asp Ser Leu Val Asp Thr Val
                245                 250                 255
Glu Pro Leu Arg Ala Asp Ala Ile Ala Lys Asn Ile Glu Ala Gln Lys
            260                 265                 270
Ser Asp Val Leu Val Pro Gln Leu Phe Leu Glu Arg Ala Thr Ala Gln
        275                 280                 285
Arg Asp Thr Leu Arg Val Val Glu Ala Ile Phe Ser Thr Ser Ala Arg
    290                 295                 300
Tyr Val Glu Leu Tyr Glu Asn Val Glu Asn Val Asn Val Glu Asn Lys
305                 310                 315                 320
Thr Leu Arg Gln His Tyr Ser Ser Leu Ile Pro Asn Leu Phe Ile Ala
                325                 330                 335
Ala Val Gly Asn Ile Asn Glu Leu Asn Asn Ala Asp Gln Ala Ala Arg
            340                 345                 350
Glu Leu Phe Leu Asp Trp Asp Thr Asp Leu Thr Thr Asn Asp Glu Asp
        355                 360                 365
Glu Ala Tyr Tyr Gln Ala Lys Leu Asp Phe Ala Ile Glu Thr Tyr Ala
    370                 375                 380
Lys Ile Leu Ile Asn Gly Glu Val Trp Gln Glu Pro Leu Ala Tyr Val
385                 390                 395                 400
Gln Asn Leu Asp Ala Gly Ala Arg Gln Glu Ala Ala Asp Arg Glu Ala
                405                 410                 415
Glu Arg Ala Ala Asp Ala Ala Tyr Arg Ala Glu Gln Leu Arg Ile Ala
            420                 425                 430
Gln Glu Ala Ala Asp Ala Gln Lys Ala Leu Ala Glu Ala Leu Ala Asn
        435                 440                 445
Ala Gly Asn Asn Asp Asn Gly Gly Asp Asn Ser Ser Asp Asp Lys Gly
    450                 455                 460
Thr Gly Ser Ser Asp Ile Gly Thr Trp Gly Pro Phe Ala Ala Ile Ala
465                 470                 475                 480
Ala Ile Ile Ala Ala Ile Ala Ala Ile Phe Pro Phe Leu Ser Gly Ile
                485                 490                 495
Val Lys Phe

<210>  39
<211>  318
<212>  DNA
<213>  Corynebacterium glutamicum

<400>  39
atgtccctcg gaccatggga aattggaatc attgtcctgc tgatcatcgt gctgttcggc      60

gcgaagaagc tgcctgatgc agctcgttcc atcggccgtt ccatgcgcat cttcaagtct 120
gaagtcaaag aaatgaacaa ggacggcgat accccagaac aacagcagca gcctcagcag 180
cagattgcgc ccaaccagat cgaggctcct cagccaaact ttgagcagca ctaccaggga 240
cagcaggttc agcagcctca gaaccctcag acccctgact accgtcagaa ctacgaggat 300
ccaaaccgca cctcttaa 318

<210> 40
<211> 105
<212> PRT
<213> Corynebacterium glutamicum

<400> 40
Met Ser Leu Gly Pro Trp Glu Ile Gly Ile Ile Val Leu Leu Ile Ile
1               5                   10                  15
Val Leu Phe Gly Ala Lys Lys Leu Pro Asp Ala Ala Arg Ser Ile Gly
            20                  25                  30
Arg Ser Met Arg Ile Phe Lys Ser Glu Val Lys Glu Met Asn Lys Asp
        35                  40                  45
Gly Asp Thr Pro Glu Gln Gln Gln Gln Pro Gln Gln Gln Ile Ala Pro
    50                  55                  60
Asn Gln Ile Glu Ala Pro Gln Pro Asn Phe Glu Gln His Tyr Gln Gly
65                  70                  75                  80
Gln Gln Val Gln Gln Pro Gln Asn Pro Gln Thr Pro Asp Tyr Arg Gln
                85                  90                  95
Asn Tyr Glu Asp Pro Asn Arg Thr Ser
            100                 105

<210> 41
<211> 471
<212> DNA
<213> Corynebacterium glutamicum

<400> 41
atgttttcta gcgtgggttg gggagagatc ttcctcttag tcgttgtggg ccttgttgtc 60
atcggcccgg aacggttgcc tcgtttgatc caggacgcac gcgctgcgct gctcgctgca 120
cgtaccgcta tcgacaatgc aaagcagtcg ttggacagtg attttggttc ggaatttgat 180
gaaatccgaa agccactaac ccaggttgca cagtacagcc ggatgagccc caagacggcc 240
atcactaagg cgttatttga taatgattcc tcgttcctgg atgactttga tccaaagaag 300
atcatggccg aaggaacaga aggcgaagct cagcgcaaca agcaggcagc tgacaacaat 360
gcgaatgtgg tggaacgtcc agctgatggt tccaccgcac gcccaacgca aaacgatcca 420
aaagacggcc cgaattactc aggtggcgtc tcttggaccg atattattta g 471

<210> 42
<211> 156
<212> PRT
<213> Corynebacterium glutamicum

<400> 42
Met Phe Ser Ser Val Gly Trp Gly Glu Ile Phe Leu Leu Val Val Val
1               5                   10                  15
Gly Leu Val Val Ile Gly Pro Glu Arg Leu Pro Arg Leu Ile Gln Asp
            20                  25                  30
Ala Arg Ala Ala Leu Leu Ala Ala Arg Thr Ala Ile Asp Asn Ala Lys
        35                  40                  45
Gln Ser Leu Asp Ser Asp Phe Gly Ser Glu Phe Asp Glu Ile Arg Lys
    50                  55                  60
Pro Leu Thr Gln Val Ala Gln Tyr Ser Arg Met Ser Pro Lys Thr Ala
65                  70                  75                  80
Ile Thr Lys Ala Leu Phe Asp Asn Asp Ser Ser Phe Leu Asp Asp Phe
                85                  90                  95

Asp Pro Lys Lys Ile Met Ala Glu Gly Thr Glu Gly Glu Ala Gln Arg
            100                 105                 110
Asn Lys Gln Ala Ala Asp Asn Asn Ala Asn Val Val Glu Arg Pro Ala
        115                 120                 125
Asp Gly Ser Thr Ala Arg Pro Thr Gln Asn Asp Pro Lys Asp Gly Pro
    130                 135                 140
Asn Tyr Ser Gly Gly Val Ser Trp Thr Asp Ile Ile
145                 150                 155

<210>  43
<211>  945
<212>  DNA
<213>  Corynebacterium glutamicum

<400>  43
atgtccattg ttgagcacat caaagagttt cgacgccgac ttcttatcgc tctggcgggc      60
atcctcgtgg gcaccattat cggctttatt tggtacgatt tctcattttg gcagatcccc     120
actttgggcg agctgctgag ggatccgtac tgttctctgc ctgctgaatc ccgctgggcc     180
atgagcgact cagaggaatg tcgactgctc gcaaccggcc cgtttgatcc attcatgctt     240
cgccttaaag tagcggcgtt ggtgggtatg gttcttggct cacccgtgtg gctgagccag     300
ctgtggggct ttatcacccc aggtttgatg aagaatgagc gccgttacac cgcaatcttc     360
gtcacgattg ctgttgtgct gtttgtcggc ggtgctgttc ttgcgtactt cgtcgttgca     420
tatggtttgg agttcctcct taccattggt ggagacaccc aggcagcggc cctgactggt     480
gataagtact tcggattctt gctcgcgttg ttggcgattt tcggcgtgag cttcgaagtt     540
ccactggtga tcggcatgct caacattgtg ggtatcttgc cttacgatgc cattaaagat     600
aagcgacgca tgatcatcat gattttgttc gtgttcgctg ctttcatgac acccggccag     660
gatcctttca ccatgttggt gttggcgctt tcactcaccg ttctggtaga gcttgccctg     720
cagttctgtc gtttcaacga caaacgccgg gacaagaagc gcccagaatg gcttgatggc     780
gatgacctct ctgcatcacc actggatact tctgctggtg gagaagatgc tccaagccca     840
gtcgaaaccc cagaggcggt ggagccttcg cggatgctga acccaagtgg ggaggcgtcg     900
ataagctata aacccgggcg cgccgacttc ggtgacgtgc tctag                     945

<210>  44
<211>  314
<212>  PRT
<213>  Corynebacterium glutamicum

<400>  44
Met Ser Ile Val Glu His Ile Lys Glu Phe Arg Arg Arg Leu Leu Ile
1               5                   10                  15
Ala Leu Ala Gly Ile Leu Val Gly Thr Ile Ile Gly Phe Ile Trp Tyr
            20                  25                  30
Asp Phe Ser Phe Trp Gln Ile Pro Thr Leu Gly Glu Leu Leu Arg Asp
        35                  40                  45
Pro Tyr Cys Ser Leu Pro Ala Glu Ser Arg Trp Ala Met Ser Asp Ser
    50                  55                  60
Glu Glu Cys Arg Leu Leu Ala Thr Gly Pro Phe Asp Pro Phe Met Leu
65                  70                  75                  80
Arg Leu Lys Val Ala Ala Leu Val Gly Met Val Leu Gly Ser Pro Val
                85                  90                  95
Trp Leu Ser Gln Leu Trp Gly Phe Ile Thr Pro Gly Leu Met Lys Asn
            100                 105                 110
Glu Arg Arg Tyr Thr Ala Ile Phe Val Thr Ile Ala Val Val Leu Phe
        115                 120                 125
Val Gly Gly Ala Val Leu Ala Tyr Phe Val Val Ala Tyr Gly Leu Glu
    130                 135                 140
Phe Leu Leu Thr Ile Gly Gly Asp Thr Gln Ala Ala Ala Leu Thr Gly
145                 150                 155                 160
Asp Lys Tyr Phe Gly Phe Leu Leu Ala Leu Leu Ala Ile Phe Gly Val
                165                 170                 175
Ser Phe Glu Val Pro Leu Val Ile Gly Met Leu Asn Ile Val Gly Ile

180                 185                 190
Leu Pro Tyr Asp Ala Ile Lys Asp Lys Arg Arg Met Ile Ile Met Ile
        195                 200                 205
Leu Phe Val Phe Ala Ala Phe Met Thr Pro Gly Gln Asp Pro Phe Thr
    210                 215                 220
Met Leu Val Leu Ala Leu Ser Leu Thr Val Leu Val Glu Leu Ala Leu
225                 230                 235                 240
Gln Phe Cys Arg Phe Asn Asp Lys Arg Arg Asp Lys Lys Arg Pro Glu
                245                 250                 255
Trp Leu Asp Gly Asp Asp Leu Ser Ala Ser Pro Leu Asp Thr Ser Ala
            260                 265                 270
Gly Gly Glu Asp Ala Pro Ser Pro Val Glu Thr Pro Glu Ala Val Glu
        275                 280                 285
Pro Ser Arg Met Leu Asn Pro Ser Gly Glu Ala Ser Ile Ser Tyr Lys
    290                 295                 300
Pro Gly Arg Ala Asp Phe Gly Asp Val Leu
305                 310

<210>  45
<211>  5
<212>  PRT
<213>  Artificial Sequence

<220>
<223>  Twin-Arginine Motif

<220>
<221>  misc_feature
<222>  (3)..(5)
<223>  Xaa can be any naturally occurring amino acid

<400>  45
Arg Arg Xaa Xaa Xaa
1               5

<210>  46
<211>  39
<212>  PRT
<213>  Escherichia coli

<400>  46
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala
        35

<210>  47
<211>  10
<212>  PRT
<213>  Escherichia coli

<400>  47
Gly Met Leu Gly Pro Ser Leu Leu Thr Pro
1               5                   10

<210>  48

<211>  10
<212>  PRT
<213>  Streptomyces mobaraense

<400>  48
Asp Asn Gly Ala Gly Glu Glu Thr Lys Ser
1               5                   10


<210>  49
<211>  10
<212>  PRT
<213>  Chryseobacterium proteolyticum

<400>  49
Asp Ser Asn Gly Asn Gln Glu Ile Asn Gly
1               5                   10


<210>  50
<211>  10
<212>  PRT
<213>  Unknown

<220>
<223>  MULTISPECIES

<400>  50
His Pro Glu Thr Leu Val Lys Val Lys Asp
1               5                   10


<210>  51
<211>  10
<212>  PRT
<213>  Escherichia coli

<400>  51
Ala Ser Asp Lys Ile Ile His Leu Thr Asp
1               5                   10


<210>  52
<211>  10
<212>  PRT
<213>  Corynebacterium glutamicum

<400>  52
Ala Pro Asp Ser Asp Trp Asp Arg Leu Ala
1               5                   10


<210>  53
<211>  10
<212>  PRT
<213>  Homo sapiens

<400>  53
Met Ser Phe Ser Gly Lys Tyr Gln Leu Gln
1               5                   10


<210>  54

<211>  60
<212>  PRT
<213>  Escherichia coli

<400>  54
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala Asp Asn Gly Ala Gly Glu Glu Thr Lys
        35                  40                  45
Ser Tyr Ala Glu Thr Tyr Arg Leu Thr Ala Asp Asp
    50                  55                  60


<210>  55
<211>  60
<212>  PRT
<213>  Escherichia coli

<400>  55
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala Asp Ser Asn Gly Asn Gln Glu Ile Asn
        35                  40                  45
Gly Lys Glu Lys Leu Ser Val Asn Asp Ser Lys Leu
    50                  55                  60


<210>  56
<211>  59
<212>  PRT
<213>  Escherichia coli

<400>  56
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala His Pro Glu Thr Leu Val Lys Val Lys
        35                  40                  45
Asp Ala Glu Asp Gln Leu Gly Ala Arg Val Gly
    50                  55


<210>  57
<211>  59
<212>  PRT
<213>  Escherichia coli

<400>  57
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala Ala Ser Asp Lys Ile Ile His Leu Thr
        35                  40                  45
Asp Asp Ser Phe Asp Thr Asp Val Leu Lys Ala
    50                  55

<210>  58
<211>  59
<212>  PRT
<213>  Escherichia coli

<400>  58
Met Asn Asn Asn Asp Leu Phe Gln Ala Ser Arg Arg Arg Phe Leu Ala
1               5                   10                  15
Gln Leu Gly Gly Leu Thr Val Ala Gly Met Leu Gly Pro Ser Leu Leu
            20                  25                  30
Thr Pro Arg Arg Ala Thr Ala Met Ser Phe Ser Gly Lys Tyr Gln Leu
        35                  40                  45
Gln Ser Gln Glu Asn Phe Glu Ala Phe Met Lys
    50                  55

<210>  59
<211>  59
<212>  PRT
<213>  Corynebacterium glutamicum

<400>  59
Met Arg Gly Lys Leu Phe Met Gly Arg His Ser Thr Lys Thr Ser Ser
1               5                   10                  15
Ala Phe Thr Lys Leu Ala Ala Ser Thr Ile Ala Phe Gly Ala Ala Ala
            20                  25                  30
Thr Ile Met Ala Pro Ser Ala Ser Ala Ala Pro Asp Ser Asp Trp Asp
        35                  40                  45
Arg Leu Ala Gln Cys Glu Ser Gly Gly Asn Trp
    50                  55